(19) Europäisches Patentamt
European Patent Office
Office européen des brevets

(11) EP 3 978 491 B1

(12) **EUROPÄISCHE PATENTSCHRIFT**

(45) Veröffentlichungstag und Bekanntmachung des Hinweises auf die Patenterteilung:
**31.01.2024 Patentblatt 2024/05**

(21) Anmeldenummer: **21204008.3**

(22) Anmeldetag: **04.12.2017**

(51) Internationale Patentklassifikation (IPC):
***C07D 403/04*** *(2006.01)* ***C09K 11/06*** *(2006.01)*
***C07D 519/00*** *(2006.01)* ***C07D 495/12*** *(2006.01)*
***C07D 495/02*** *(2006.01)* ***C07D 493/12*** *(2006.01)*
***C07D 493/04*** *(2006.01)* ***H05B 33/14*** *(2006.01)*
***H10K 50/00*** *(2023.01)*

(52) Gemeinsame Patentklassifikation (CPC):
**C07D 519/00; C07D 403/04; C07D 493/04; C07D 493/12; C07D 495/02; C07D 495/12;**
Y02E 10/549

(54) **STICKSTOFFHALTIGE HETEROCYCLEN ZUR VERWENDUNG IN OLEDS**

NITROGEN-CONTAINING HETEROCYCLES FOR USE IN OLEDS

HÉTÉROCYCLES AZOTÉS UTILISÉ DANS LES OLEDS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2016 EP 16202147**

(43) Veröffentlichungstag der Anmeldung:
**06.04.2022 Patentblatt 2022/14**

(62) Dokumentnummer(n) der früheren Anmeldung(en) nach Art. 76 EPÜ:
**17807885.3 / 3 548 477**

(73) Patentinhaber: **Merck Patent GmbH**
**64293 Darmstadt (DE)**

(72) Erfinder:
- **Parham, Amir**
**64293 DARMSTADT (DE)**
- **Kroeber, Jonas**
**64293 DARMSTADT (DE)**
- **Joosten, Dominik**
**64293 DARMSTADT (DE)**
- **Ludemann, Aurélie**
**64293 DARMSTADT (DE)**
- **Grossmann, Tobias**
**SHANGHAI, 201206 (CN)**
- **Stoessel, Philipp**
**64293 DARMSTADT (DE)**
- **Eickhoff, Christian**
**64293 DARMSTADT (DE)**

(74) Vertreter: **Merck Patent Association**
**Merck Patent GmbH**
**64271 Darmstadt (DE)**

(56) Entgegenhaltungen:
**WO-A1-2016/195441 WO-A1-2017/109722**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 978 491 B1

## Beschreibung

Die vorliegende Erfindung beschreibt stickstoffhaltige Heterocyclen, welche mit Carbazol-Gruppen substituiert sind, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer. Die Eigenschaften organischer elektrolumineszierender Vorrichtungen werden nicht nur durch die eingesetzten Emitter bestimmt. Hier sind insbesondere auch die anderen verwendeten Materialien, wie Host-/Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen sowie als Elektronentransportmaterialien häufig heteroaromatische Verbindungen eingesetzt, wie zum Beispiel Chinazolinderivate. Weiterhin werden als Matrixmaterialien auch Carbazolderivate verwendet, wobei auch Verbindungen bekannt sind, die sowohl Carbazol-Strukturen als auch Strukturen aufweisen, die von Chinazolinen abgeleitet sind.

Generell besteht bei diesen Materialien, beispielsweise für die Verwendung als Matrixmaterialien, Lochtransportmaterialien oder Elektronentransportmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung, eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochleitermaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs eignen.

Weiterhin sollten die Verbindungen, insbesondere bei ihrem Einsatz als Matrixmaterialien, als Lochleitmaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Diese Verbindungen und elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Aus WO 2017/109727 und WO 2016/195441 sind heteroaromatische Verbindungen bekannt, bei welchen die Gruppen, welche den Gruppen $X^1$ der erfindungsgemäßen Verbindungen entsprechen, beides Mal ein N-Atom darstellen.

Gegenstand der vorliegenden Erfindung ist daher eine Verbindung, umfassend mindestens eine Struktur gemäß einer der folgenden Formeln (IIIa), (IIIb), (IIIc) oder (IIId)

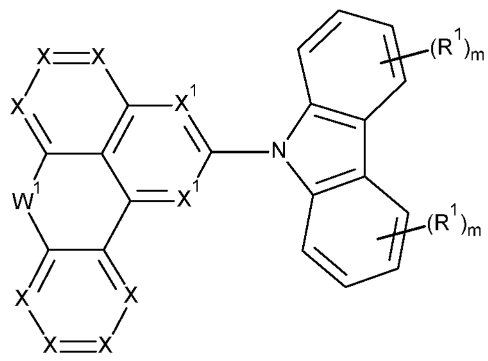

Formel (IIIa)

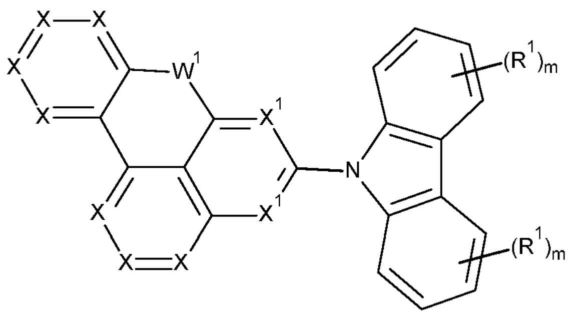

Formel (IIIb)

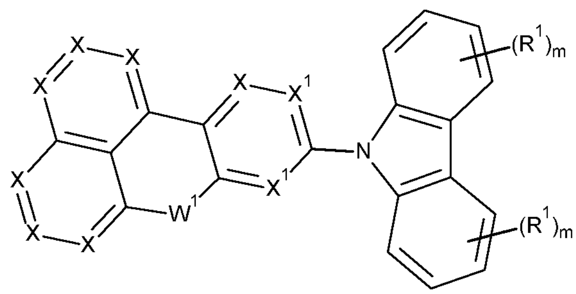

Formel (IIIc)

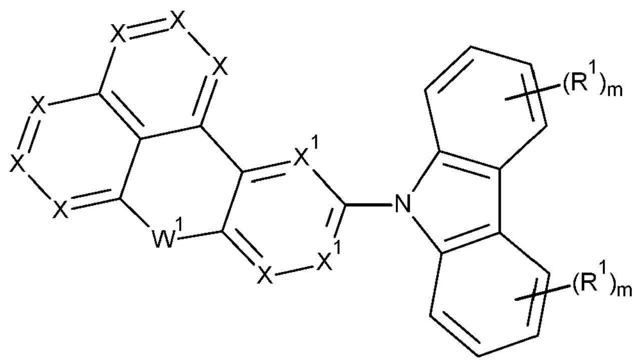

Formel (IIId)

wobei für die verwendeten Symbole gilt:

X ist bei jedem Auftreten gleich oder verschieden N oder $CR^1$;

$X^1$ ist N oder $CR^1$, wobei genau eine Gruppe $X^1$ für N steht;

$W^1$ ist O, S, SO, $SO_2$, $Si(R^1)_2$ oder C=O, vorzugsweise O, S, SO, $SO_2$ oder C=O, besonders bevorzugt O, S oder C=O, ganz besonders bevorzugt O oder S;

$R^1$ ist bei jedem Auftreten gleich oder verschieden H, D, F, Cl, Br, I, CN, $NO_2$, $N(Ar^1)_2$, $N(R^2)_2$, $OAr^1$, $OR^2$, $SAr^1$, $SR^1$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenylgruppe mit 2 bis 40 C-Atomen, die jeweils mit einem oder mehreren Resten $R^2$ substituiert sein kann und wobei eine oder mehrere nicht benachbarte $CH_2$-Gruppen durch $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere, vorzugsweise benachbarte Substituenten $R^1$ auch miteinander ein mono- oder polycyclisches, aliphatisches, heteroaliphatisches, aromatisches oder heteroaromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $OR^3$, $SR^3$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^{13})_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann und wobei eine oder mehrere nicht benachbarte $CH_2$-Gruppen durch $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehrere, vorzugsweise benachbarte Substituenten $R^3$ miteinander ein mono- oder polycyclisches, aliphatisches Ringsystem bilden und

m ist gleich oder verschieden 0, 1, 2, 3 oder 4.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende

Schema verdeutlicht:

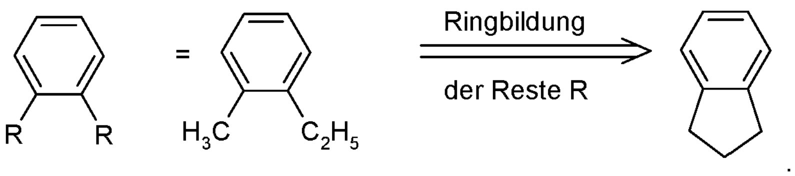


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

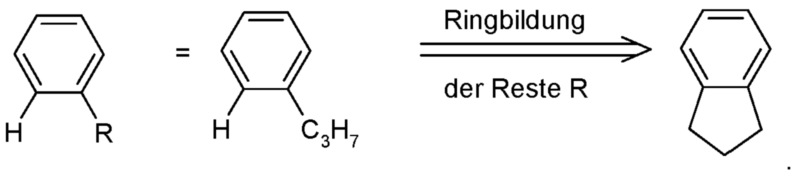


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ferner sind Verbindungen mit Strukturen bevorzugt, in denen höchstens zwei Gruppen X pro Ring für N stehen und bevorzugt mindestens eine, besonders bevorzugt mindestens zwei der Gruppen X pro Ring ausgewählt sind aus C-H und C-D.

Vorzugsweise können die erfindungsgemäßen Verbindungen mindestens eine Struktur der Formel (IVa), (IVb), (IVc) oder (IVd) umfassen,

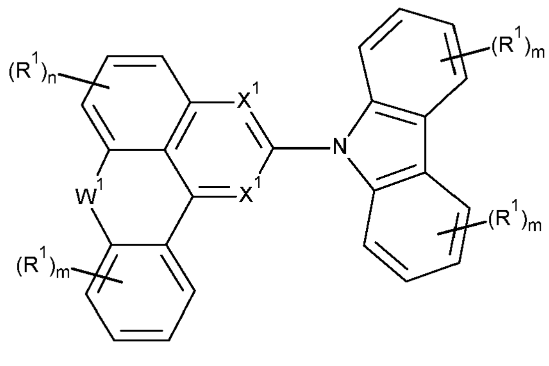

Formel (IVa)

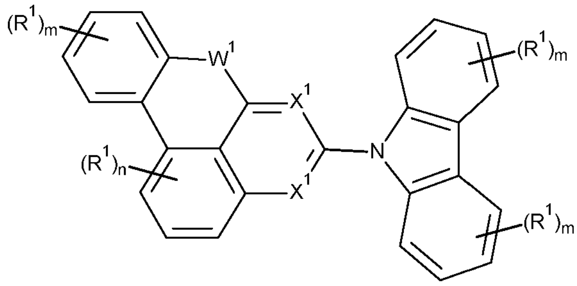

Formel (IVb)

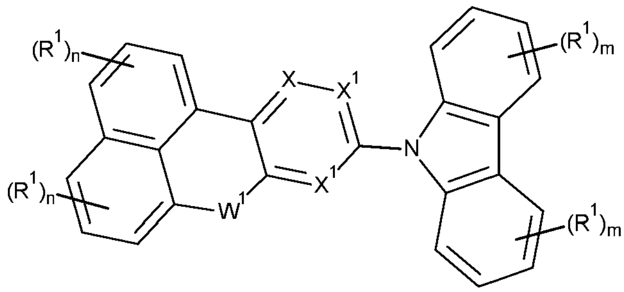

Formel (IVc)

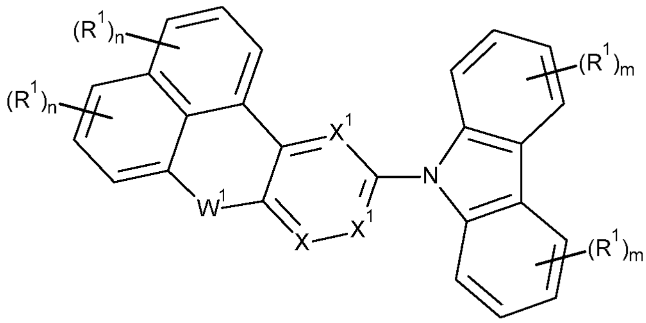

Formel (IVd)

wobei die verwendeten Symbole $R^1$, $W^1$, m, X und $X^1$ die zuvor dargelegte Bedeutung aufweisen und n gleich oder verschieden 0, 1, 2, oder 3, vorzugsweise 0, 1 oder 2 ist. X in Formel (IVc) und (IVd) steht bevorzugt für $CR^1$.

Ferner kann in den Strukturen der zuvor und nachfolgend dargelegten Formeln vorgesehen sein, dass zwei benachbarte Reste $R^1$ einen Ring der Formel (DB-1), (DB-2) oder (DB-3) bilden,

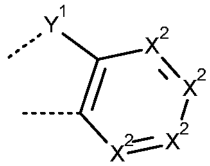

Formel (DB-1)

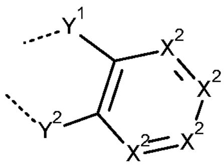

Formel (DB-2)

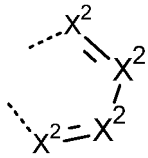

Formel (DB-3)

wobei $X^2$ gleich oder verschieden für N oder $CR^2$, vorzugsweise $CR^2$ steht, $Y^1$ und $Y^2$ bei jedem Auftreten unabhängig für O, S, $C(R^2)_2$ oder $NR^2$ stehen und die gestrichelten Linien die Bindung an die Aryl- oder Heteroarylgruppe darstellen,

wobei $R^2$ die zuvor genannte Bedeutung aufweist. Aus Gründen der Klarheit ist festzuhalten, dass die Gruppe der Formel (DB-3) zusammen mit einem aromatischen oder heteroaromatischen Rest der Carbazoleinheit ein kondensiertes Ringsystem bildet.

In einer weiterhin bevorzugten Ausführungsform können die erfindungsgemäßen Verbindungen mindestens eine Struktur der Formel (VIa), (VIb), (VIc) oder (VId) aufweisen,

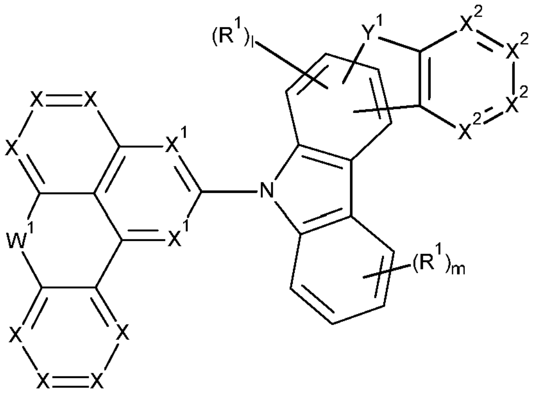

Formel (VIa)

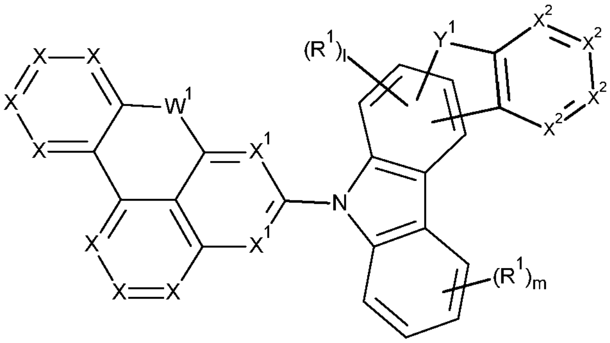

Formel (VIb)

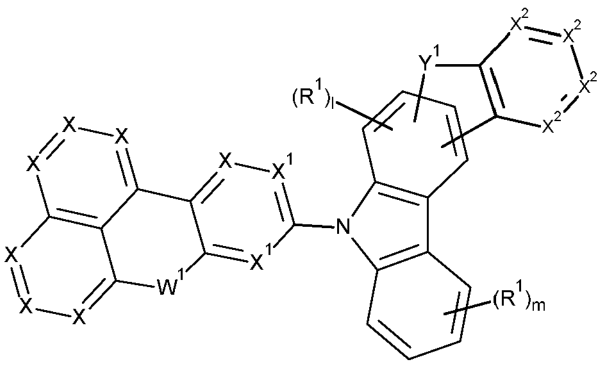

Formel (VIc)

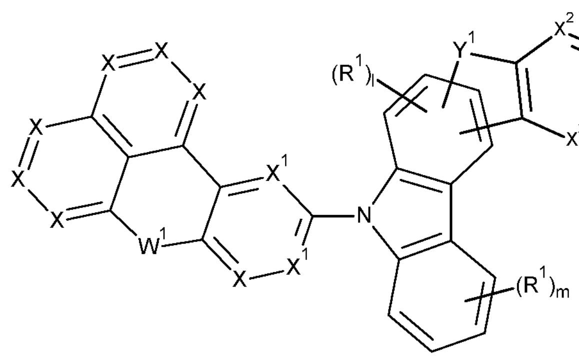

Formel (VId)

wobei die verwendeten Symbole $W^1$, $R^1$, X und $X^1$ die zuvorgenannte Bedeutung aufweisen, die Symbole $Y^1$ und $X^2$ die zuvor, insbesondere für Formel (DB-1) genannte Bedeutung aufweisen, l für 0, 1 oder 2, vorzugsweise 0 oder 1 steht und m für 0, 1, 2, 3 oder 4, vorzugsweise 0, 1 oder 2 steht. Dabei steht X bevorzugt gleich oder verschieden für $CR^1$ oder N, besonders bevorzugt für $CR^1$.

Die oben abgebildeten und nachfolgend genauer erläuterten Formeln schließen die Kondensation der Gruppen (DB-1) bis (DB-2) in jeder möglichen Position und Orientierung mit ein. Dies ist nachfolgend beispielhaft für die Verbindung (VIa) gezeigt, wobei Reste $R^1$ der Übersichtlichkeit halber nicht mit eingezeichnet sind:

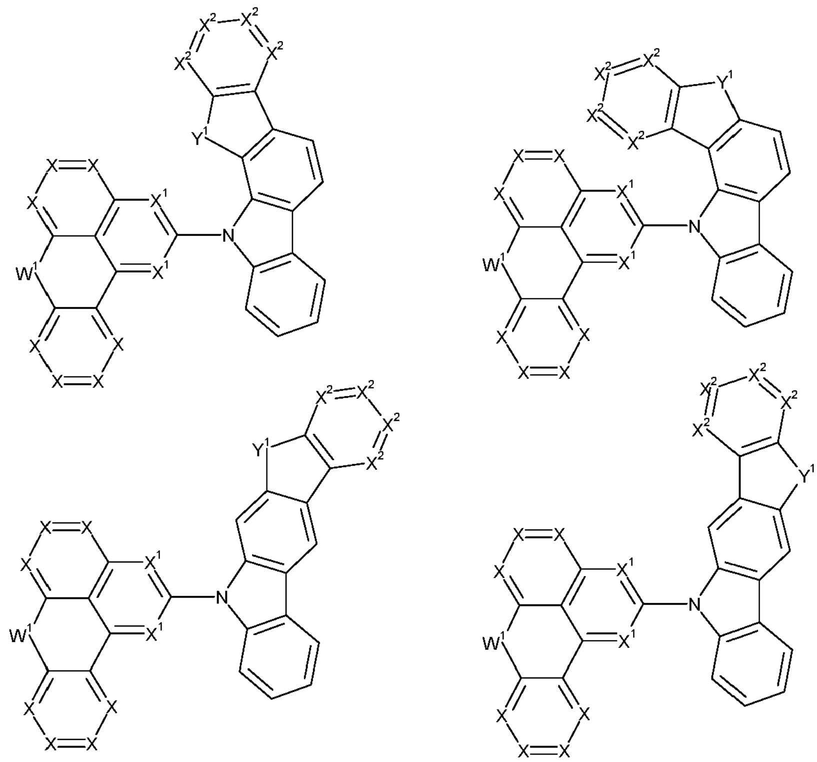

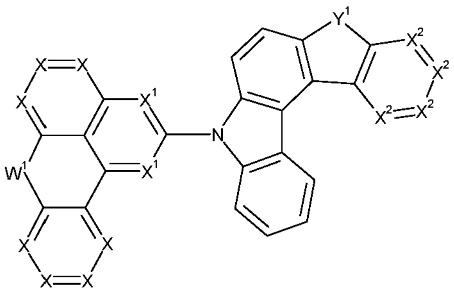

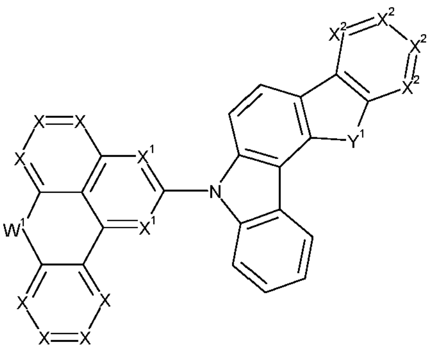

.

Ferner kann vorgesehen sein, dass erfindungsgemäße Verbindungen mindestens eine Struktur der Formel (VIIa), (VIIb), (VIIc) oder (VIId) umfassen,

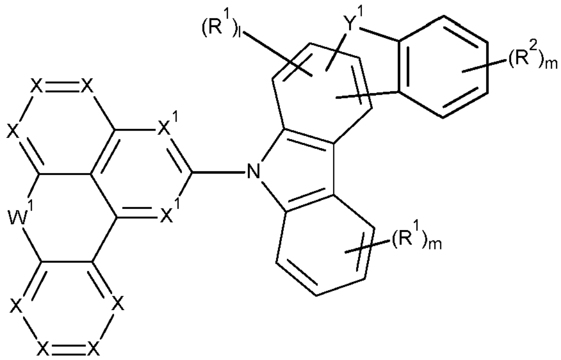

Formel (VIIa)

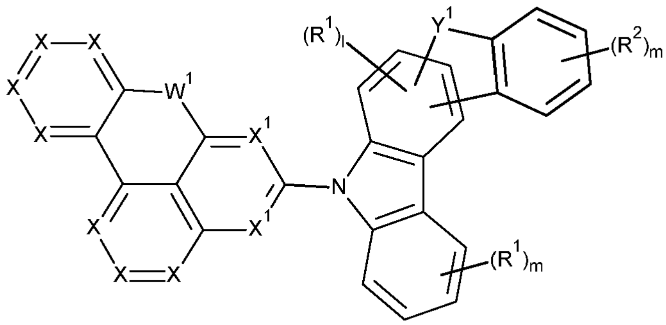

Formel (VIIb)

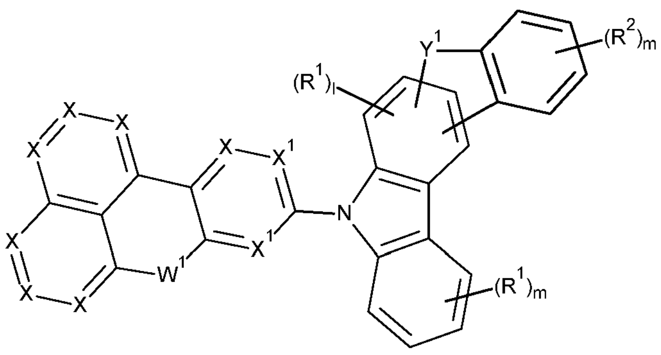

Formel (VIIc)

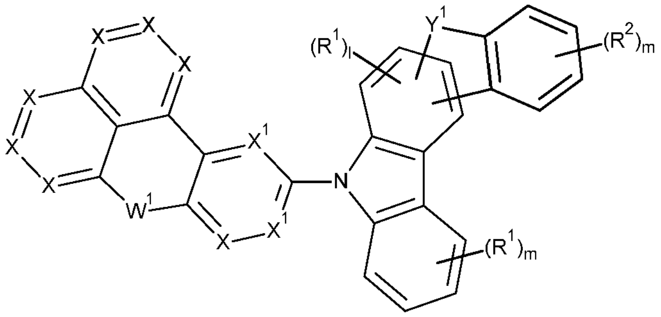

Formel (VIId)

wobei die verwendeten Symbole $W^1$, $R^1$, $R^2$, X und $X^1$ die zuvordargelegte Bedeutung aufweisen, das Symbol $Y^1$ die zuvor, insbesondere für Formel (DB-1) genannte Bedeutung aufweist, l für 0, 1 oder 2, vorzugsweise 0 oder 1 steht und m gleich oder verschieden für 0, 1, 2, 3 oder 4, vorzugsweise 0, 1 oder 2 steht. Dabei steht X bevorzugt gleich oder verschieden für $CR^1$ oder N, besonders bevorzugt für $CR^1$.

In einer weiterhin bevorzugten Ausführungsform können die erfindungsgemäßen Verbindungen mindestens eine Struktur der Formel (IXa), (IXb), (IXc) oder (IXd) umfassen,

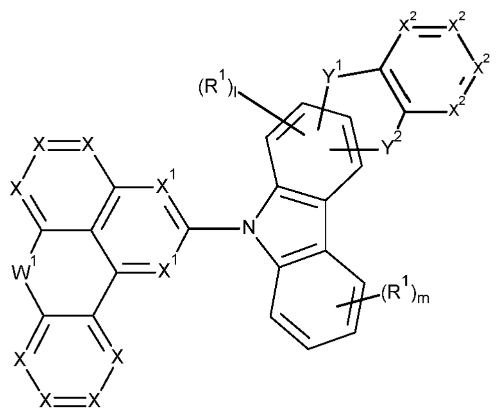

Formel (IXa)

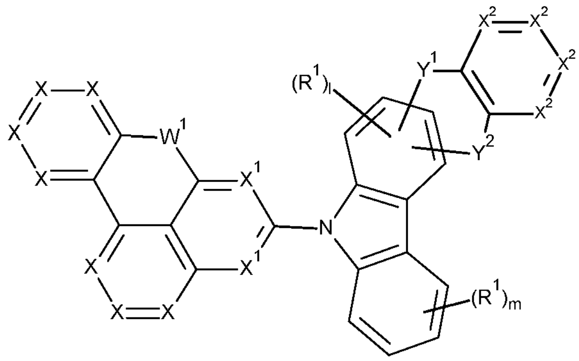

Formel (IXb)

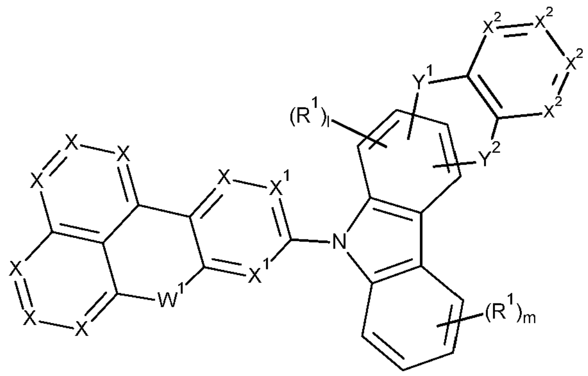

Formel (IXc)

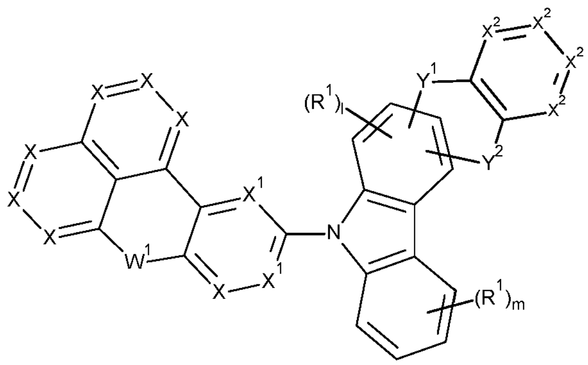

Formel (IXd)

wobei die verwendeten Symbole $W^1$, $R^1$, X und $X^1$ die zuvor dargelegte Bedeutung aufweisen, die Symbole $Y^1$, $Y^2$ und $X^2$ die zuvor, insbesondere für Formel (DB-2) genannte Bedeutung aufweisen, l für 0, 1 oder 2, vorzugsweise 0 oder 1 steht und m für 0, 1, 2, 3 oder 4, vorzugsweise 0, 1 oder 2 steht. Dabei steht X bevorzugt gleich oder verschieden für $CR^1$ oder N, besonders bevorzugt für $CR^1$.

Weiterhin können die erfindungsgemäßen Verbindungen mindestens eine Struktur der Formel (Xa), (Xb), (Xc) oder (Xd) umfassen,

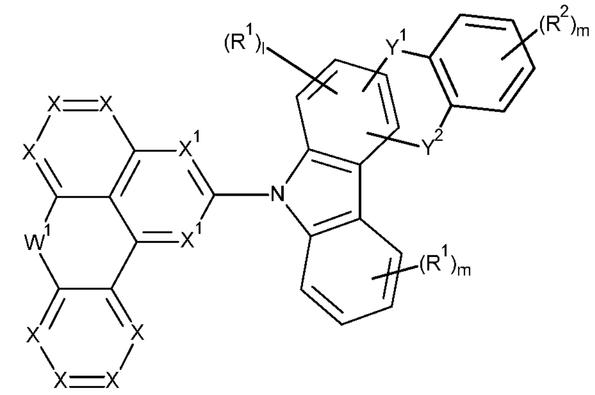

Formel (Xa)

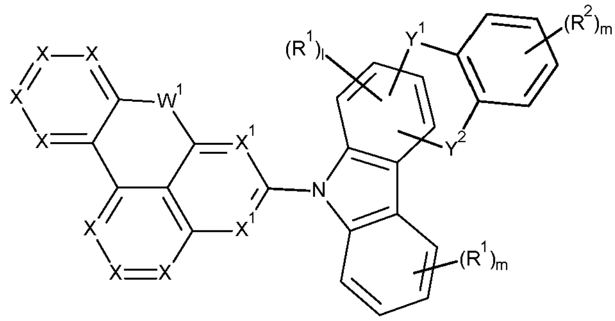

Formel (Xb)

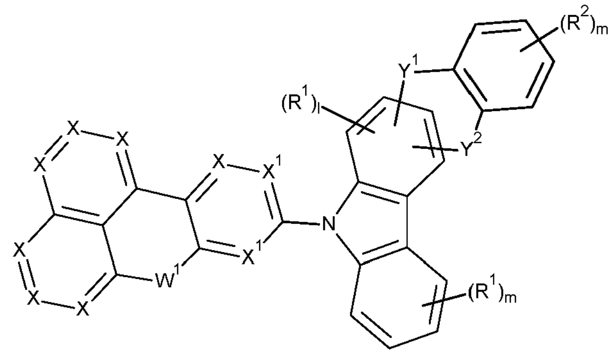

Formel (Xc)

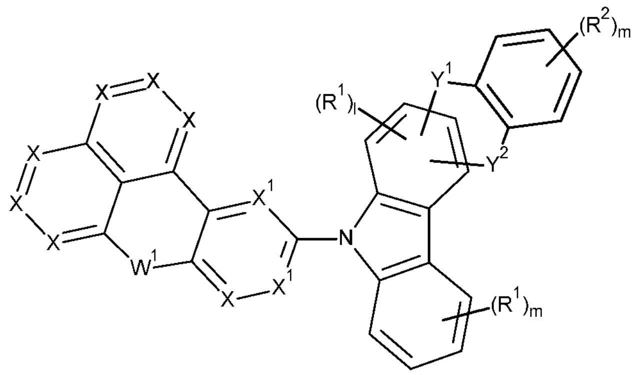

Formel (Xd)

wobei die verwendeten Symbole $W^1$, $R^1$, $R^2$, X und $X^1$ die zuvor dargelegte Bedeutung aufweisen, die Symbole $Y^1$ und $Y^2$ die zuvor, insbesondere für Formel (DB-2) genannte Bedeutung aufweisen, l für 0, 1 oder 2, vorzugsweise 0 oder 1 steht und m gleich oder verschieden für 0, 1, 2, 3 oder 4, vorzugsweise 0, 1 oder 2 steht. Dabei steht X bevorzugt gleich oder verschieden für $CR^1$ oder N, besonders bevorzugt für $CR^1$.

In einer weiterhin bevorzugten Ausführungsform können die erfindungsgemäßen Verbindungen mindestens eine Struktur der Formel (XIIa), (XIIb), (XIIc) oder (XIId) umfassen,

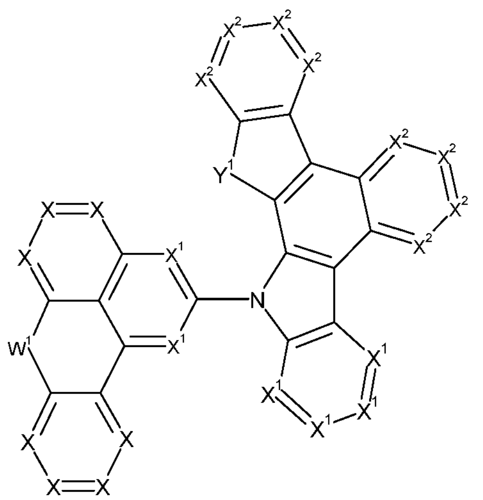

Formel (XIIa)

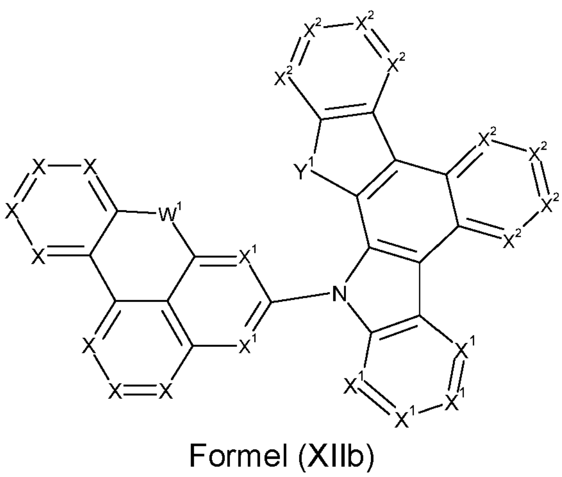

Formel (XIIb)

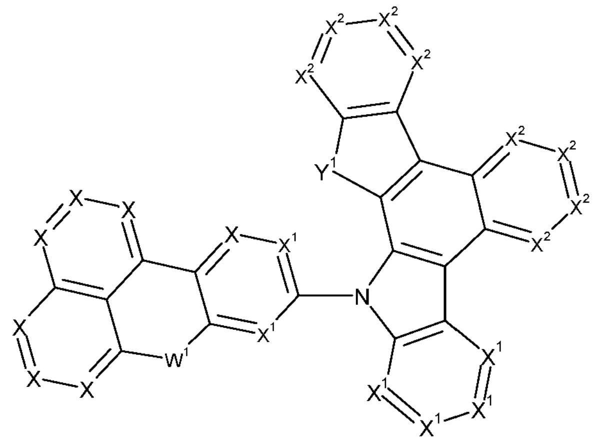

Formel (XIIc)

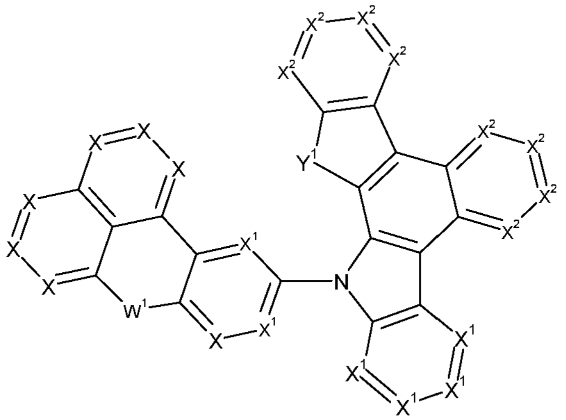

Formel (XIId)

wobei die verwendeten Symbole $W^1$ und X die zuvor dargelegte Bedeutung aufweisen, die Symbole $X^2$ und $Y^1$ die zuvor, insbesondere für Formel (DB-2) und (DB-3) genannte Bedeutung aufweisen und $X^1$ N oder $CR^1$ ist, wobei genau eine Gruppe $X^1$, welche benachbart ist zu dem Kohlenstoffatom, an das die Carbazolderivat-Gruppe gebunden ist, für N steht. Dabei steht X bevorzugt gleich oder verschieden für $CR^1$ oder N, besonders bevorzugt für $CR^1$.

Weiterhin können die erfindungsgemäßen Verbindungen mindestens eine Struktur der Formel (XIIIa), (XIIIb), (XIIIc) oder (XIIId) umfassen,

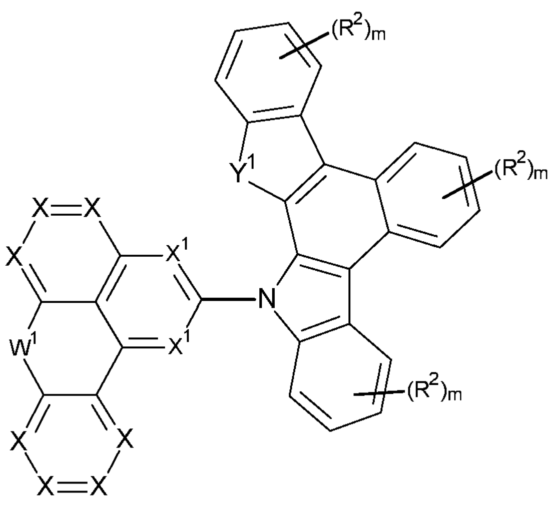

Formel (XIIIa)

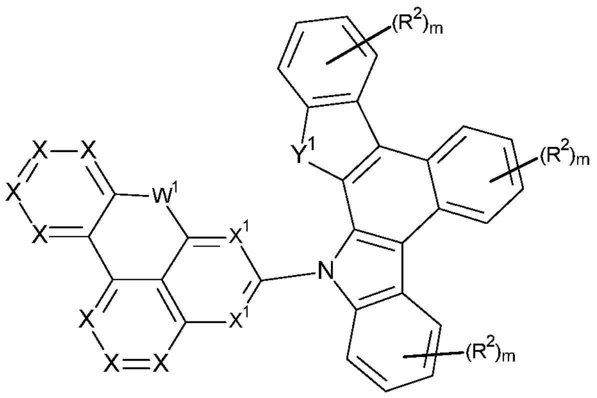

Formel (XIIIb)

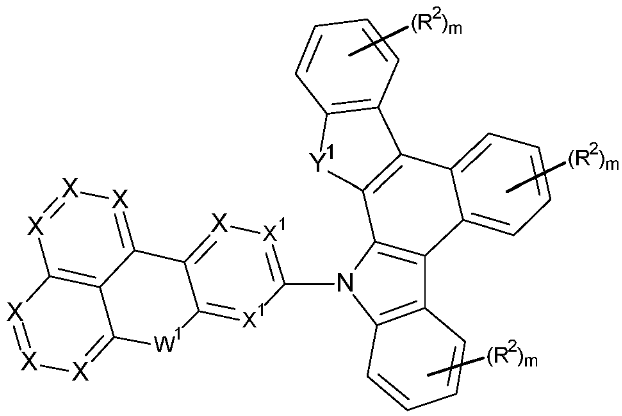

Formel (XIIIc)

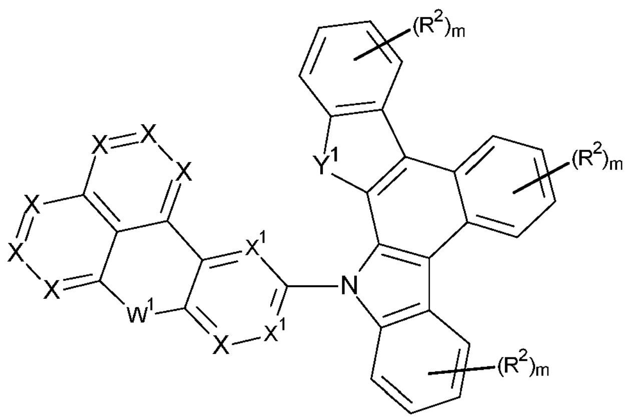

Formel (XIIId)

wobei die verwendeten Symbole $W^1$, $R^2$, X und $X^1$ die zuvor dargelegte Bedeutung aufweisen, das Symbol $Y^1$ die zuvor, insbesondere für Formel (DB-2) genannte Bedeutung aufweist, l für 0, 1 oder 2, vorzugsweise 0 oder 1 steht und m gleich oder verschieden für 0, 1, 2, 3 oder 4, vorzugsweise 0, 1 oder 2 steht. Dabei steht X bevorzugt gleich oder verschieden für $CR^1$ oder N, besonders bevorzugt für $CR^1$.

In einer weiteren Ausführungsform kann vorgesehen sein, dass in den Formeln (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) die Symbole $W^1$ und $Y^1$ gleich sind. Ferner können die Symbole $W^1$ und $Y^1$ verschieden sein.

Weiterhin sind Verbindungen bevorzugt, die dadurch gekennzeichnet sind, dass in den Formeln (VIa), (VIb),

(VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) das Symbol $W^1$ für O und das Symbol $Y^1$ für O, S, $C(R^2)_2$ oder $NR^2$ steht.

Ferner sind Verbindungen bevorzugt, die dadurch gekennzeichnet sind, dass in den Formeln (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) das Symbol $W^1$ für S und das Symbol $Y^1$ für O, S, $C(R^2)_2$ oder $NR^2$ steht.

In einer weiteren Ausführungsform kann vorgesehen sein, dass in den Formeln (IXa), (IXb), (IXc), (IXd), (Xa), (Xb), (Xc) und/oder (Xd) die Symbole $W^1$ und $Y^2$ gleich sind. Ferner können die Symbole $W^1$ und $Y^2$ verschieden sein.

Weiterhin sind Verbindungen bevorzugt, die dadurch gekennzeichnet sind, dass in den Formeln (IXa), (IXb), (IXc), (IXd), (Xa), (Xb), (Xc) und/oder (Xd) das Symbol $W^1$ für O und das Symbol $Y^2$ für O, S, $C(R^2)_2$ oder $NR^2$ steht.

Ferner sind Verbindungen bevorzugt, die dadurch gekennzeichnet sind, dass in den Formeln (IXa), (IXb), (IXc), (IXd), (Xa), (Xb), (Xc) und/oder (Xd) das Symbol $W^1$ für S und das Symbol $Y^2$ für O, S, $C(R^2)_2$ oder $NR^2$ steht.

In einer weiteren Ausführungsform kann vorgesehen sein, dass in den Formeln (IXa), (IXb), (IXc), (IXd), (Xa), (Xb), (Xc) und/oder (Xd), die Symbole $Y^1$ und $Y^2$ gleich sind. Ferner können die Symbole $Y^1$ und $Y^2$ verschieden sein.

Darüber hinaus sind Verbindungen mit Strukturen gemäß Formel (IIa), (IIb), (IIc), (IId), (IIIa), (IIIb), (IIIc), (IIId), (VIa), (VIb), (VIc), (VId), (VIIa), (VIIb), (VIIc), (VIId), (IXa), (IXb), (IXc), (IXd), (Xa), (Xb), (Xc) und/oder (Xd) bevorzugt, in denen nicht mehr als vier, vorzugsweise nicht mehr als zwei Gruppen X für N stehen, und besonders bevorzugt alle Gruppen X für $CR^1$ stehen, wobei vorzugsweise höchstens vier, besonders bevorzugt höchstens drei und speziell bevorzugt höchstens zwei der Gruppen $CR^1$ für die X steht, ungleich der Gruppe CH ist.

Weiterhin kann vorgesehen sein, dass die Substitutenten $R^1$ der Carbazolgruppe mit den Ringatomen der Carbazol-Gruppe kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substitutenten $R^1$ der Carbazol-Gruppe mit den Ringatomen der Carbazol-Gruppe kein Ringsystem bilden. Dies schließt die Bildung eines Ringsystems mit möglichen Substituenten $R^2$, $R^3$ ein, die an die Reste $R^1$ gebunden sein können. Wenn die Substituenten $R^1$ der Carbazol-Gruppe ein kondensiertes Ringsystem bilden, dann handelt es sich sich bevorzugt um eine Gruppe der vorne beschriebenen Formel (DB-1), (DB-2) und/oder (DB-3).

Falls die Carbazoleinheit in den Formeln (IIIa), (IIIb), (IIIc) oder (IIId) ein einfaches Carbazol darstellt, wenn also insbesondere keine Gruppe der vorne beschriebenen Formeln (DB-1), (DB-2) und/oder (DB-3) ankondensiert ist, dann ist es bevorzugt, wenn das Carbazol genau einen Substituent $R^1$ aufweist. Dieser Substituent $R^1$ steht vorzugsweise in para-Position zum Stickstoffatom des Carbazolrestes.

Ferner kann in den Strukturen gemäß den Formeln (IIIa), (IIIb), (IIIc), (IIId), (VIa), (VIb), (VIc), (VId), (VIIa), (VIIb), (VIIc), (VIId), (IXa), (IXb), (IXc), (IXd), (Xa), (Xb), (Xc), (Xd), (XIIIa), (XIIIb), (XIIIc) und/oder (XIIId), vorgesehen sein, dass die Summe der Indices l, m und n höchstens 6, vorzugsweise höchstens 4 und besonders bevorzugt höchstens 2 beträgt.

Weiterhin kann vorgesehen sein, dass die Substitutenten $R^1$ des heteroaromatischen Ringssystems gemäß den Formeln (IIIa), (IIIb), (IIIc), (IIId), (IVa), (IVb), (IVc), (IVd), (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) mit den Ringatomen des heteroaromatischen Ringssystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substituenten $R^1$ des heteroaromatischen Ringssystems gemäß den Formeln (IIIa), (IIIb), (IIIc), (IIId), (IVa), (IVb), (IVc), (IVd), (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId)mit den Ringatomen des heteroaromatischen Ringssystems kein Ringsystem bilden. Dies schließt die Bildung eines Ringsystems mit möglichen Substituenten $R^2$, $R^3$ ein, die an die Reste $R^1$ gebunden sein können.

Gemäß einer bevorzugten Ausgestaltung sind erfindungsgemäße Verbindungen durch Strukturen der Formel (IIIa), (IIIb), (IIIc), (IIId), (IVa), (IVb), (IVc), (IVd), (Va), (VIa), (VIb), (VIc), (VId), (VIIa), (VIIb), (VIIc), (VIId), (IXa), (IXb), (IXc), (IXd), (Xa), (Xb), (Xc), (Xd), (XIa), (XIIa), (XIIb), (XIIc), (XIId), (XIIIa), (XIIIb), (XIIIc) und/oder (XIIId) darstellbar.

Vorzugsweise weisen Verbindungen, umfassend Strukturen gemäß Formel (IIIa), (IIIb), (IIIc), (IIId), (IVa), (IVb), (IVc), (IVd), (Va), (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Vorzugsweise ist die aromatische oder heteroaromatische Gruppe des durch das Symbol $Ar^1$ dargestellten aromatischen oder heteroaromatischen Ringsystems direkt, d.h. über ein Atom der aromatischen oder heteroaromatischen Gruppe, an das jeweilige Atom der weiteren Gruppe gebunden, wobei das Symbol $Ar^1$ besonders bevorzugt einen

Aryl- oder Heteroarylrest darstellt.

In einer weiteren bevorzugten Ausführungsform der Erfindung steht $Ar^1$ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste $R^2$ substituiert sein kann, bevorzugt aber unsubstituiert ist, wobei $R^2$ die zuvor dargestellte Bedeutung aufweisen kann.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt bilden die Reste $R^2$ mit den Ringatomen der Arylgruppe oder Heteroarylgruppe $Ar^1$, an die die Reste $R^2$ gebunden sein können, kein kondensiertes Ringsystem. Dies schließt die Bildung eines kondensierten Ringsystems mit möglichen Substituenten $R^3$ ein, die an die Reste $R^2$ gebunden sein können.

Wenn X oder $X^1$ für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nicht-benachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die vorzugsweise an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann, wobei die Gruppe $Ar^1$ die zuvor genannte Bedeutung aufweist.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (IIIa), (IIIb), (IIIc), (IIId), (IVa), (IVb), (IVc), (IVd), (VIa), (VIb), (VIc), (VId), (VIIa), (VIIb), (VIIc), (VIId), (IXa), (IXb), (IXc), (IXd), (Xa), (Xb), (Xc), (Xd), (XIIa), (XIIb), (XIIc), (XIId), (XIIIa), (XIIIb), (XIIIc) und/oder (XIIId) mindestens ein Rest $R^1$ oder $Ar^1$ für eine Gruppe steht, die ausgewählt ist aus den Formeln ($R^1$-1) bis ($R^1$- 80)

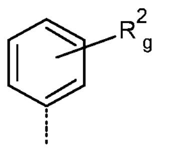

Formel ($R^1$-1)

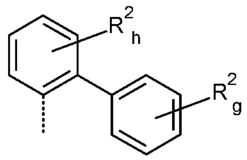

Formel ($R^1$-2)

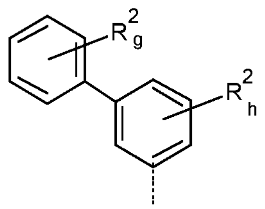

Formel ($R^1$-3)

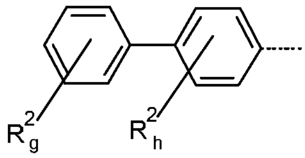

Formel ($R^1$-4)

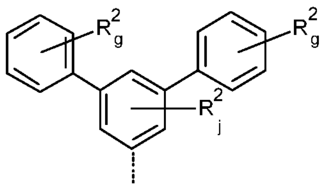

Formel ($R^1$-5)

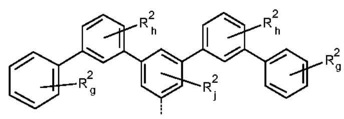

Formel ($R^1$-6)

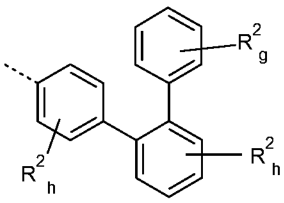

Formel ($R^1$-7)

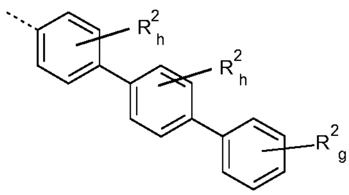

Formel ($R^1$-8)

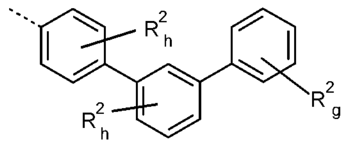

Formel ($R^1$-9)

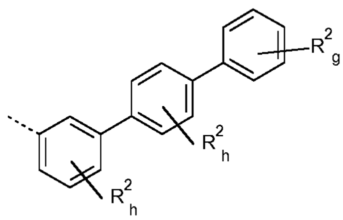

Formel ($R^1$-10)

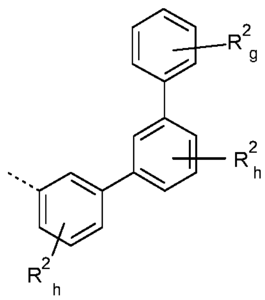

Formel ($R^1$-11)

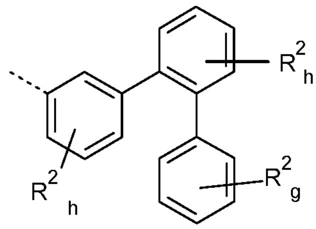

Formel ($R^1$-12)

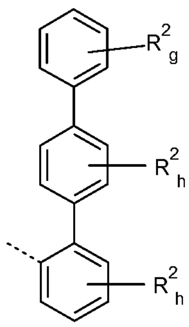

Formel ($R^1$-13)

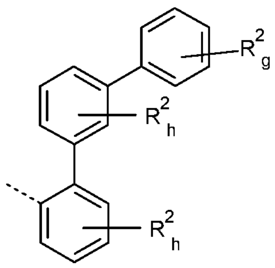

Formel ($R^1$-14)

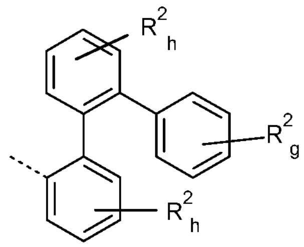

Formel ($R^1$-15)

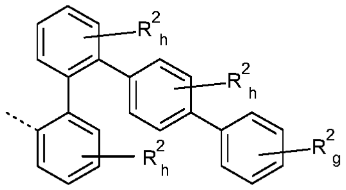

Formel ($R^1$-16)

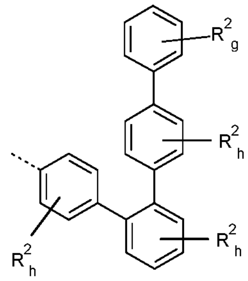

Formel ($R^1$-17)

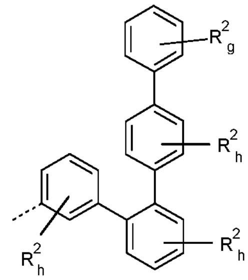

Formel ($R^1$-18)

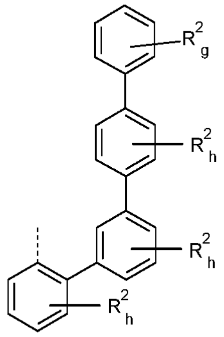

Formel ($R^1$-19)

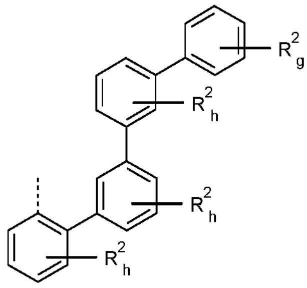

Formel ($R^1$-20)

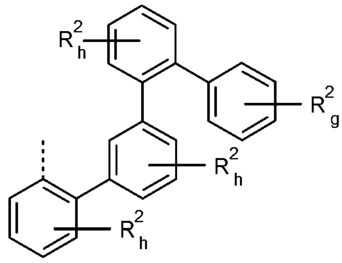

Formel ($R^1$-21)

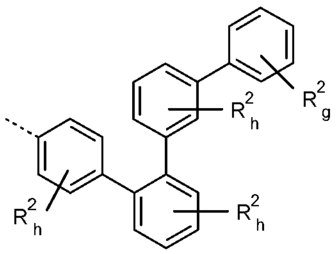

Formel ($R^1$-22)

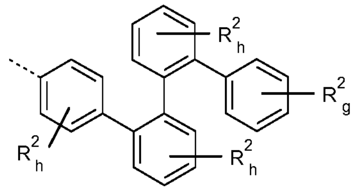

Formel ($R^1$-23)

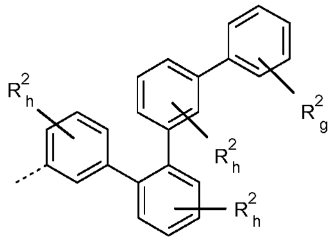

Formel ($R^1$-24)

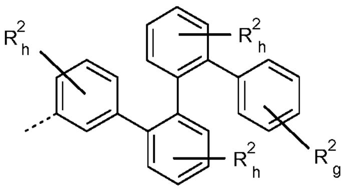

Formel ($R^1$-25)

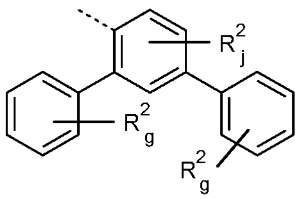

Formel ($R^1$-26)

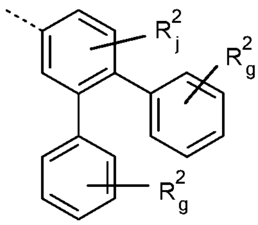

Formel ($R^1$-27)

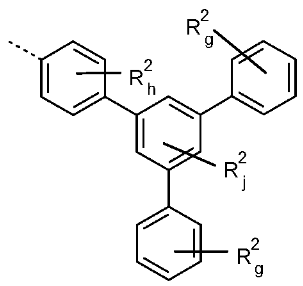

Formel ($R^1$-28)

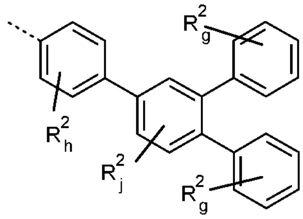

Formel ($R^1$-29)

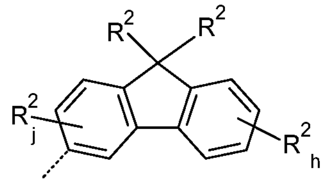

Formel ($R^1$-30)

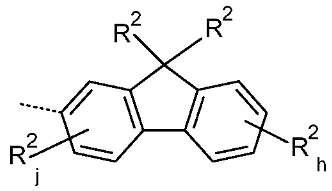

Formel ($R^1$-31)

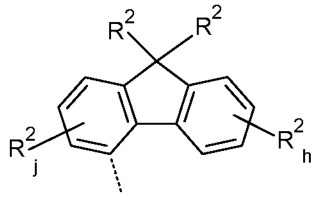

Formel ($R^1$-32)

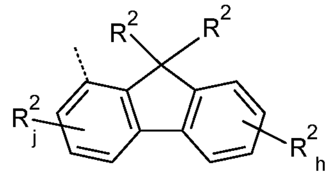

Formel ($R^1$-33)

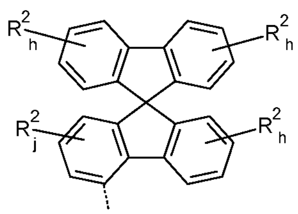

Formel ($R^1$-34)

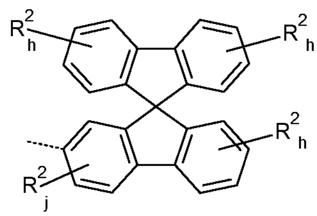

Formel ($R^1$-35)

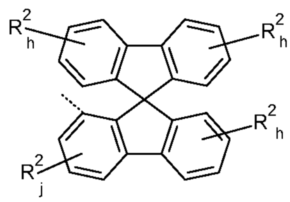

Formel ($R^1$-36)

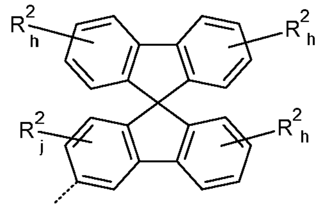

Formel ($R^1$-37)

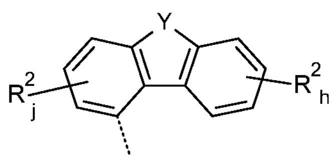

Formel ($R^1$-38)

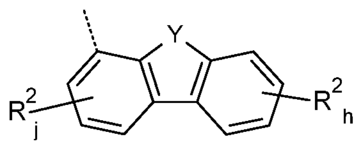

Formel ($R^1$-39)

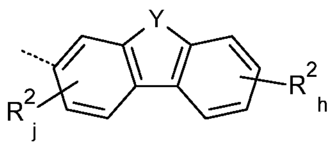

Formel ($R^1$-40)

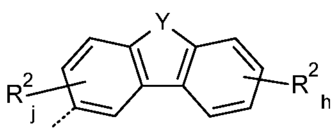

Formel ($R^1$-41)

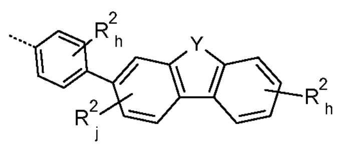

Formel ($R^1$-42)

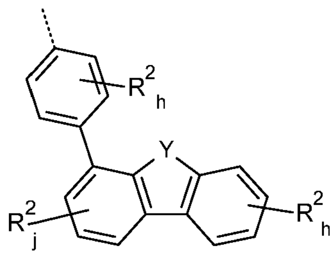

Formel ($R^1$-43)

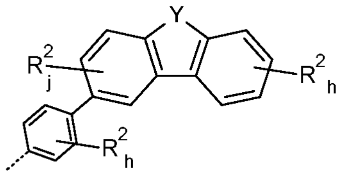

Formel ($R^1$-44)

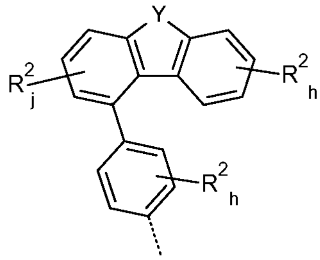

Formel ($R^1$-45)

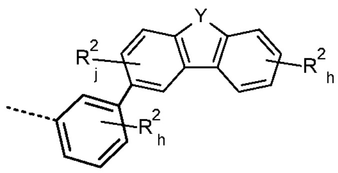

Formel ($R^1$-46)

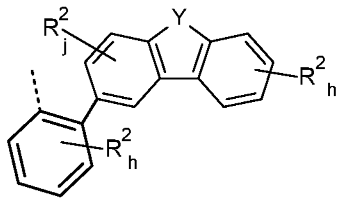

Formel ($R^1$-47)

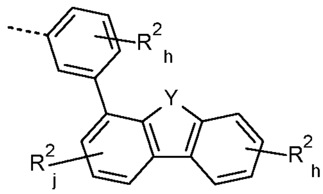

Formel ($R^1$-48)

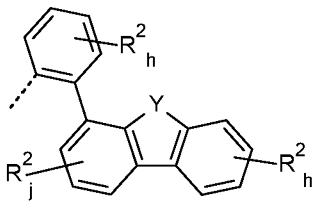

Formel ($R^1$-49)

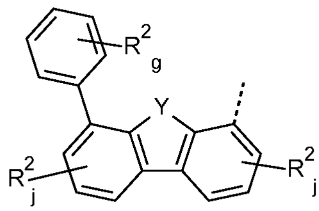

Formel ($R^1$-50)

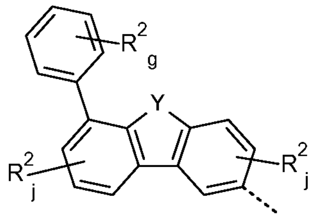

Formel ($R^1$-51)

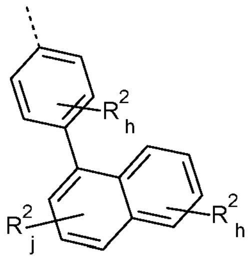

Formel ($R^1$-52)

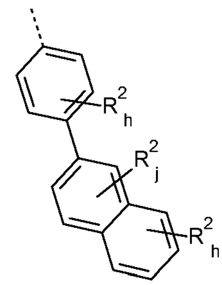

Formel ($R^1$-53)

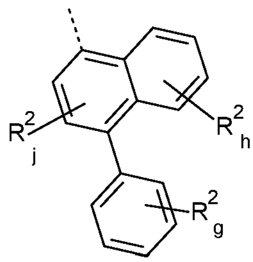

Formel ($R^1$-54)

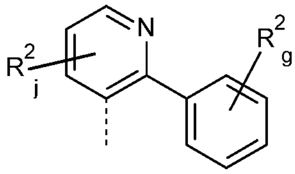

Formel ($R^1$-55)

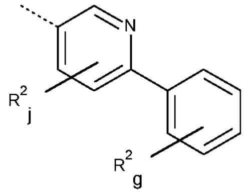

Formel ($R^1$-56)

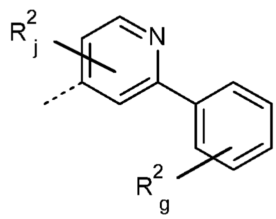

Formel ($R^1$-57)

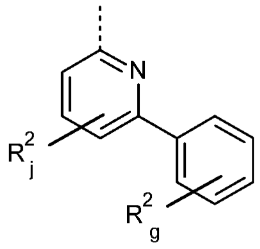
Formel ($R^1$-58)

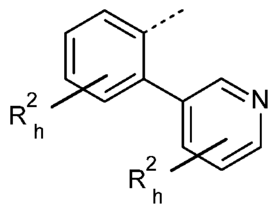
Formel ($R^1$-59)

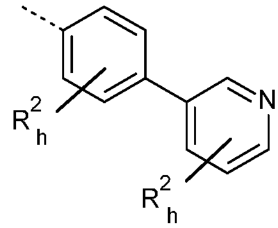
Formel ($R^1$-60)

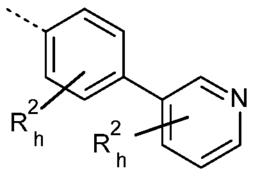
Formel ($R^1$-61)

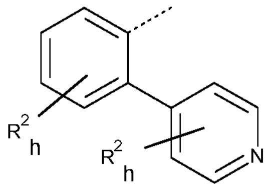
Formel ($R^1$-62)

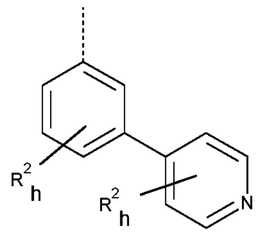
Formel ($R^1$-63)

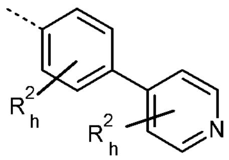
Formel ($R^1$-64)

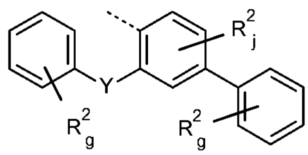
Formel ($R^1$-65)

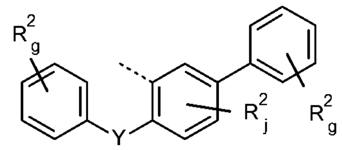
Formel ($R^1$-66)

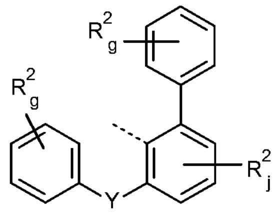
Formel ($R^1$-67)

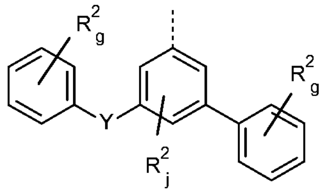
Formel ($R^1$-68)

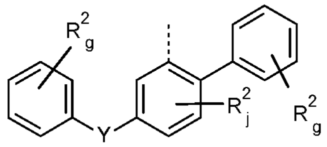
Formel ($R^1$-69)

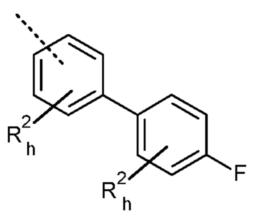
Formel ($R^1$-70)

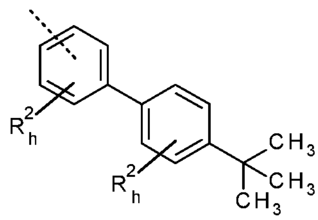
Formel ($R^1$-71)

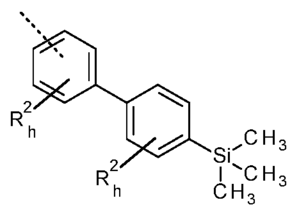
Formel ($R^1$-72)

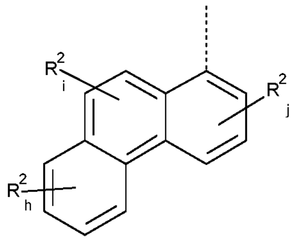

Formel ($R^1$-73)

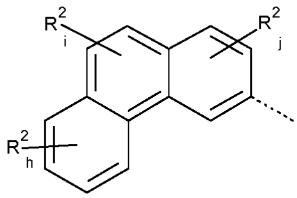

Formel ($R^1$-74)

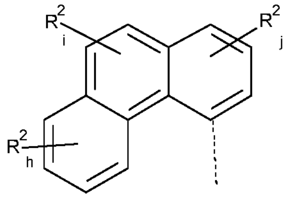

Formel ($R^1$-75)

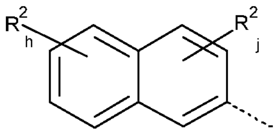

Formel ($R^1$-76)

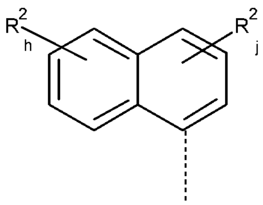

Formel ($R^1$-77)

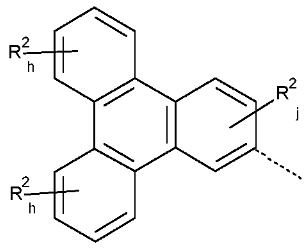

Formel ($R^1$-78)

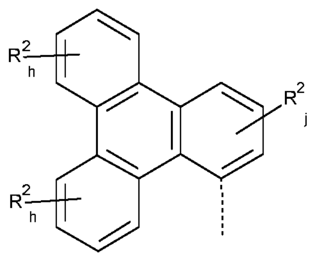

Formel ($R^1$-79)

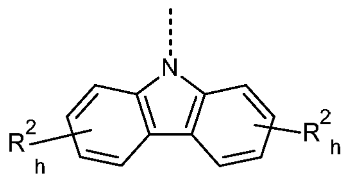

Formel ($R^1$-80)

wobei für die verwendeten Symbole gilt:

- Y ist O, S oder $NR^2$, vorzugsweise O oder S;
- i ist bei jedem Auftreten unabhängig 0, 1 oder 2;
- j ist bei jedem Auftreten unabhängig 0, 1, 2 oder 3;
- h ist bei jedem Auftreten unabhängig 0, 1, 2, 3 oder 4;
- g ist bei jedem Auftreten unabhängig 0, 1, 2, 3, 4 oder 5;
- $R^2$ kann die zuvor genannte genannte Bedeutung aufweisen; und die gestrichelte Bindung markiert die Anbindungsposition.

Hierbei sind die Gruppen der Formeln $R^1$-1 bis $R^1$-56 bevorzugt, wobei die Gruppen $R^1$-1, $R^1$-3, $R^1$-5, $R^1$-6, $R^1$-15, $R^1$-29, $R^1$-30, $R^1$-31, $R^1$-32, $R^1$-33, $R^1$-38, $R^1$-39, $R^1$-40, $R^1$-41, $R^1$-42, $R^1$-43, $R^1$-44 und/oder $R^1$-45 besonders bevorzugt sind.

Vorzugsweise kann vorgesehen sein, dass die Summe der Indices i, j, h und g in den Strukturen der Formel ($R^1$-1) bis ($R^1$-80) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-80) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der

geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

In einer weiteren bevorzugten Ausführungsform der Erfindung ist $R^2$ bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste $R^2$ mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^3$bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß einer der Formeln (IIIa), (IIIb), (IIIc) oder (IIId) bei dem in einer Kupplungsreaktion eine Verbindung, umfassend mindestens eine stickstoffhaltige heterocyclische Gruppemit einer Verbindung, umfassend mindestens eine Carbazol-Gruppe, verbunden wird.

Geeignete Verbindungen mit einer Carbazol-Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

## Schema 1

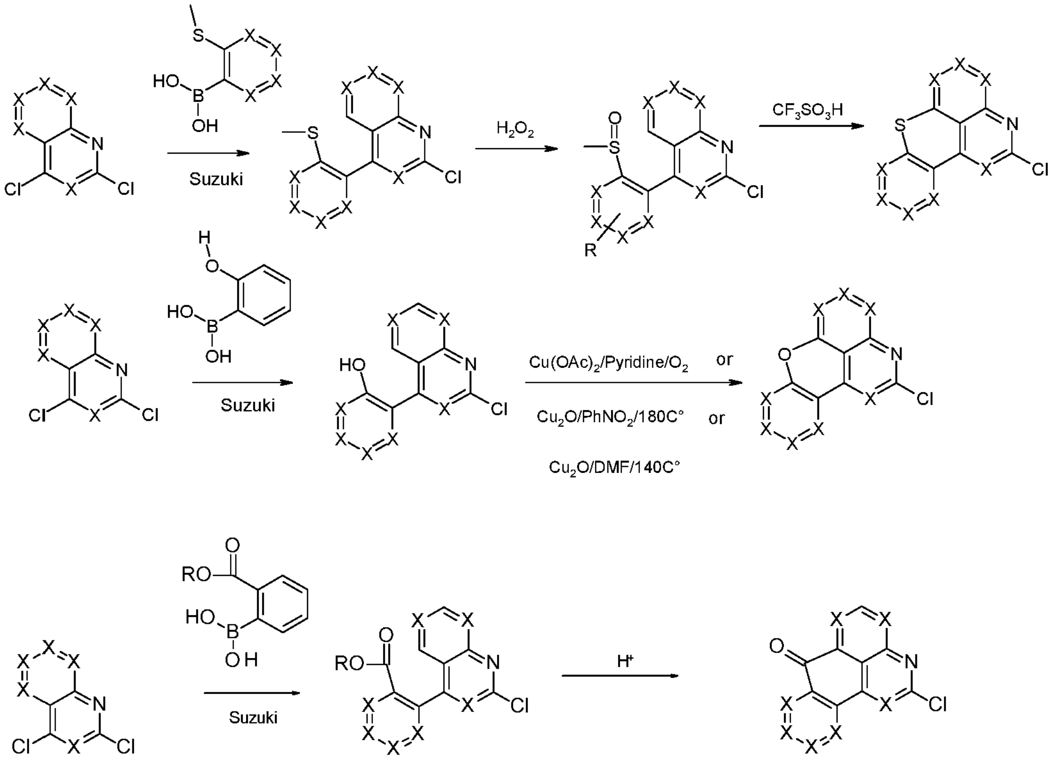


## Schema 2

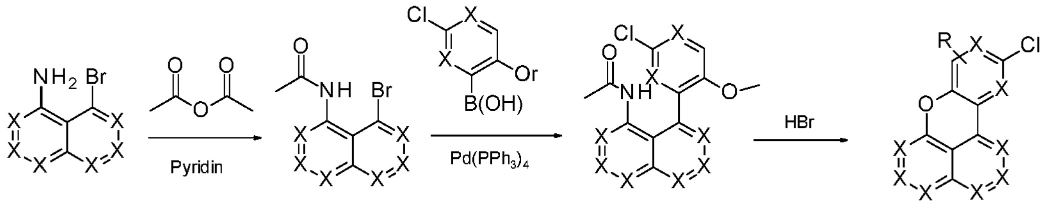


## Schema 3

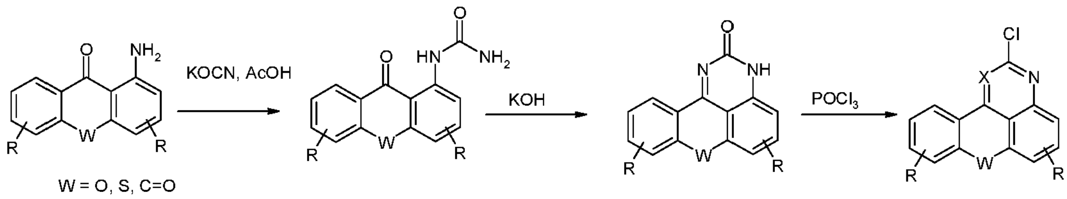


## Schema 4

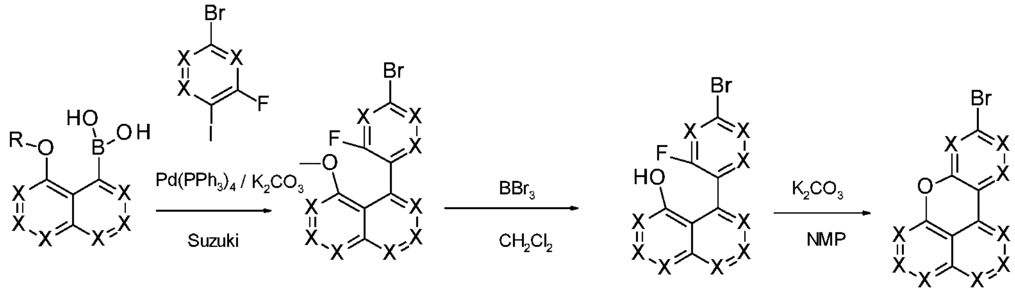


Schema 5

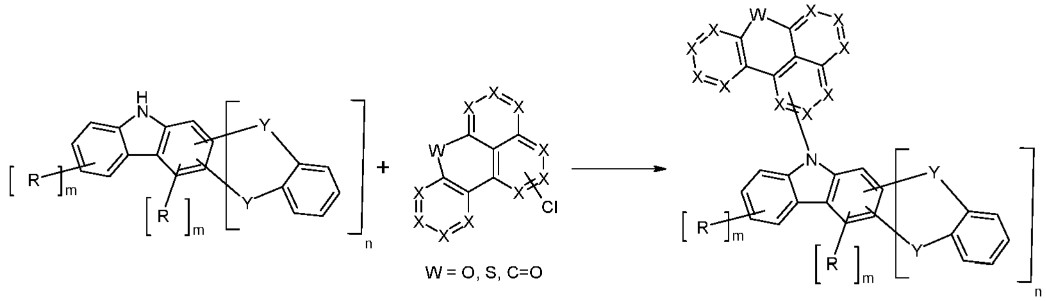


Die Bedeutung der in den Schemata 1 bis 5 verwendeten Symbole entspricht im Wesentlichen denen, die für eine der Formeln (IIIa), (IIIb), (IIIc) oder (IIId) definiert wurden, wobei aus Gründen der Übersichtlichkeit auf eine Nummerierung verzichtet wurde.

Die Chlor- bzw. Bromsubstituenten in den Strukturen gemäß den oben angegebenen Schemata können in einer Kupplungsreaktion mit der entsprechenden Carbazol-Gruppe, welche am Stickstoffatom unsubstituiert ist, zur erfindungsgemäßen Verbindung umgesetzt werden.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln. Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formeln (IIIa), (IIIb), (IIIc) oder (IIId) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formeln (IIIa), (IIIb), (IIIc) oder (IIId) oder erfindungsgemäße Verbindungen, wobei ein oder mehrere Bindungen der erfindungsgemäßen Verbindungen oder der Strukturen von einer der Formeln (IIIa), (IIIb), (IIIc) oder (IIId) zum Polymer, Oligomer oder Dendrimer vorhanden sind. Je nach Verknüpfung der Strukturen von einer der Formeln (IIIa), (IIIb), (IIIc) oder (IIId)bzw. der Verbindungen bilden diese daher eine Seitenkette

des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß einer der Formeln (IIIa), (IIIb), (IIIc) oder (IIId) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen von einer der Formeln (IIIa), (IIIb), (IIIc) oder (IIId) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, beispielsweise ein fluoreszierender Dotand, ein phosphoreszierender Dotand oder eine Verbindung, die TADF (thermally activated delayed fluorescence) zeigt, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung umfassend Strukuren gemäß einer der Formeln (IIIa), (IIIb), (IIIc) oder (IIId) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden Erfindung stellt eine Zusammensetzung dar, enthaltend wenigstens eine Verbindung, umfassend mindestens eine Struktur gemäß einer der Formeln (IIIa), (IIIb), (IIIc) oder (IIId) bzw. die

zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen. Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau Heh bzw. LUMO-Energieniveau Leh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((Heh*27.212)-0.9899)/1.1206$$

$$LUMO(eV) = ((Leh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß einer der Formeln (IIIa), (IIIb), (IIIc) oder (IIId) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US

2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und den noch nicht offen gelegten Anmeldungen EP 16179378.1 und EP 16186313.9 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind nachfolgend aufgeführt.

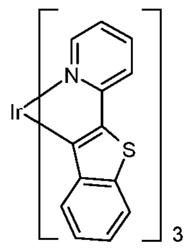
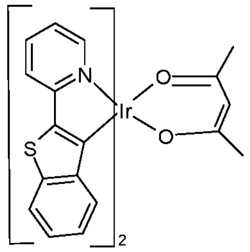
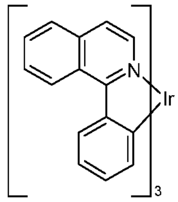
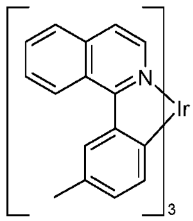
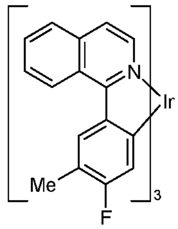
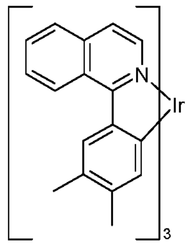
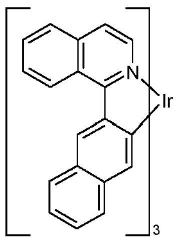
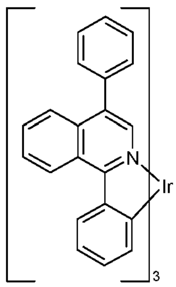
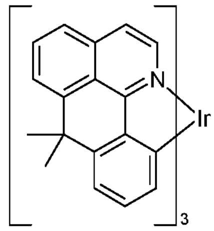
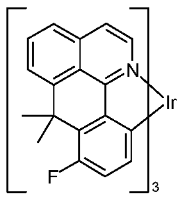
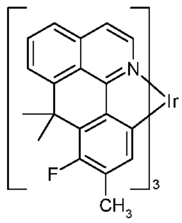
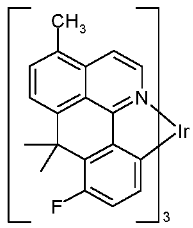

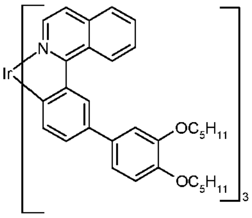

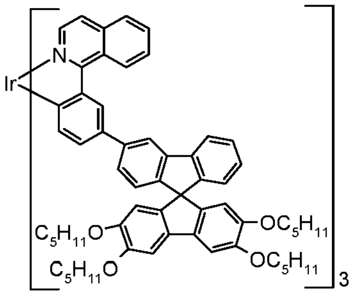

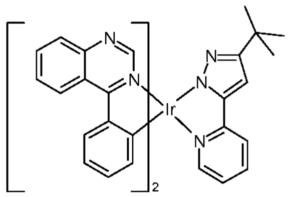

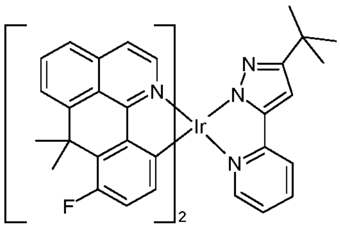

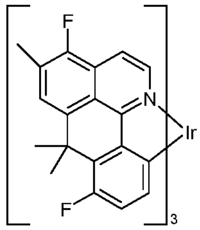

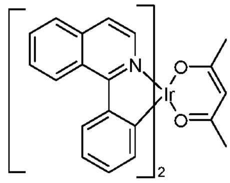

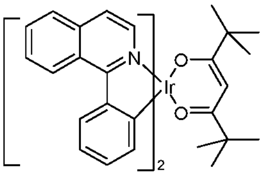

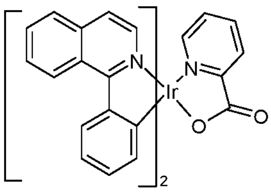

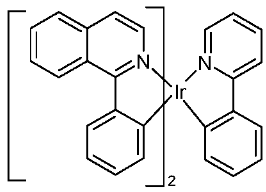

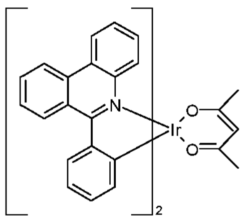

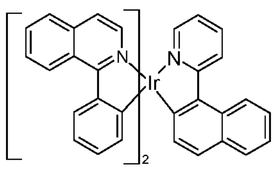

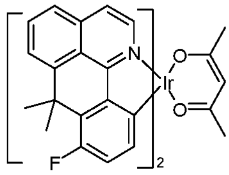

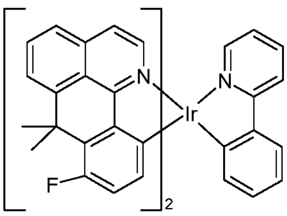

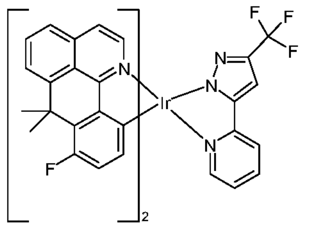

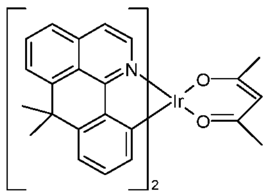

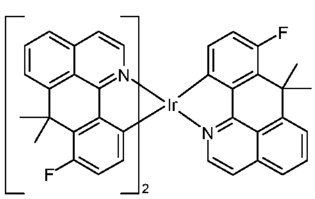

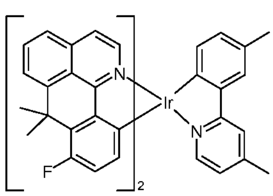

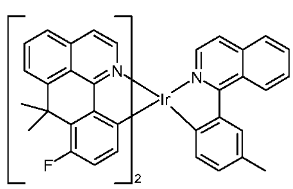

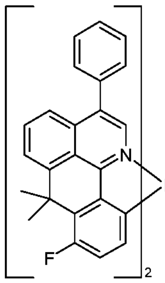
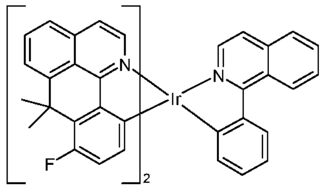
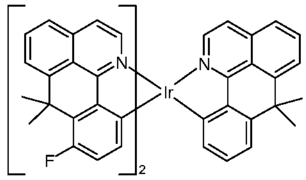
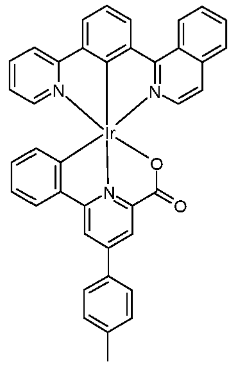
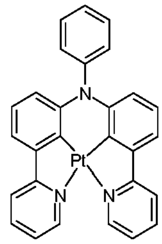
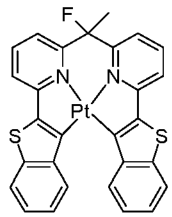
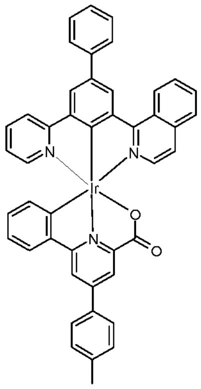
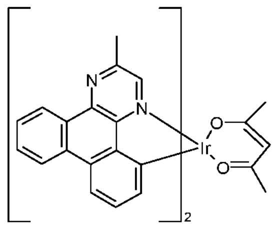
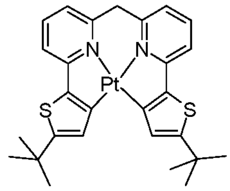
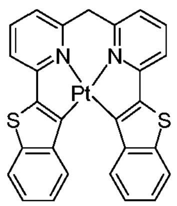
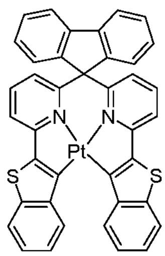
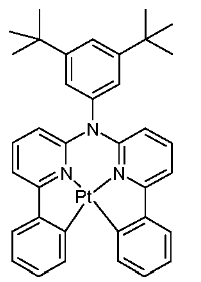
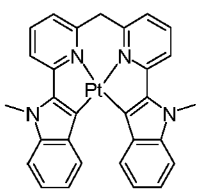
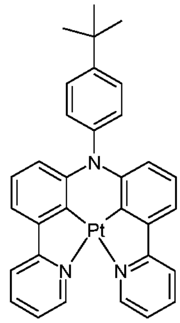
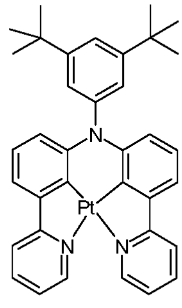

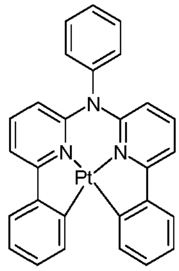

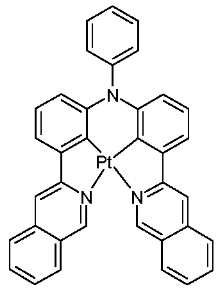

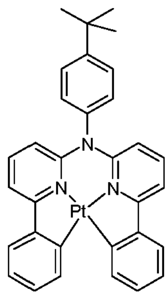

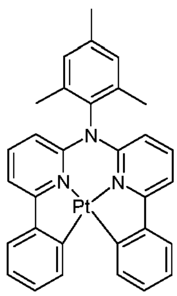

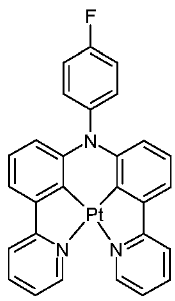

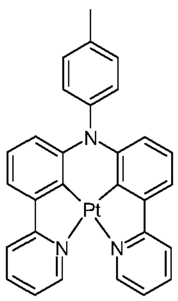

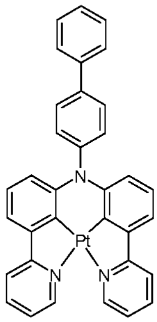

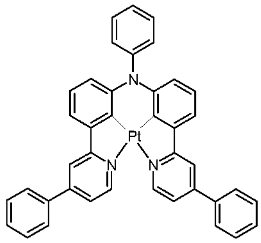

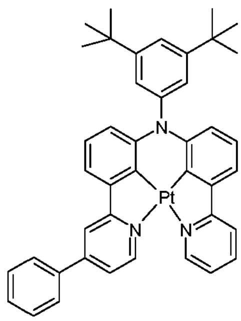

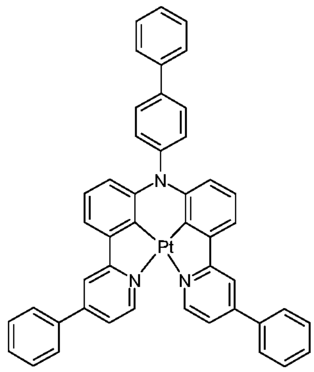

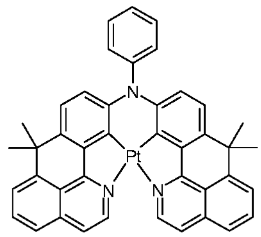

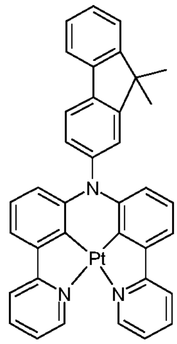
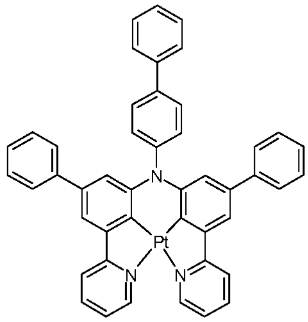
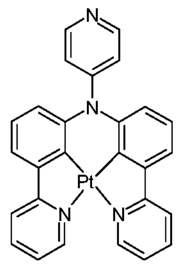

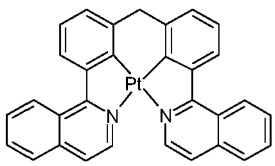
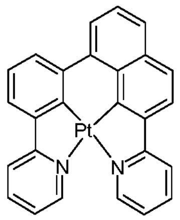
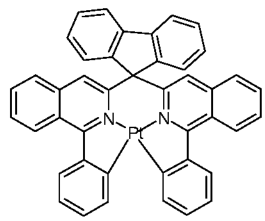

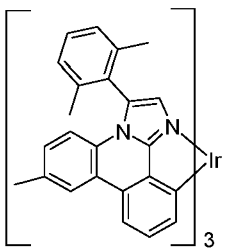
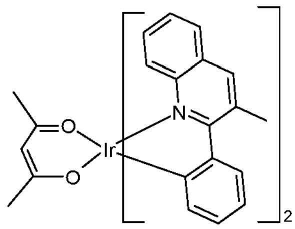
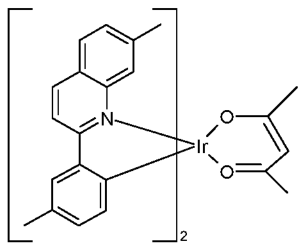

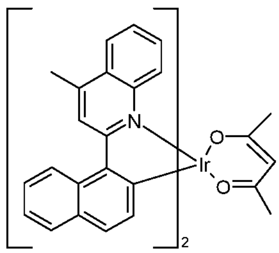
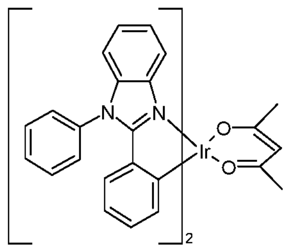
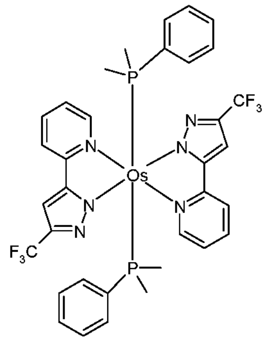

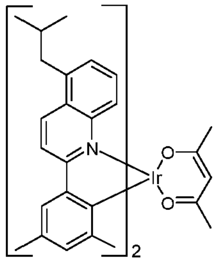
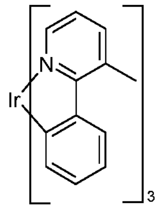
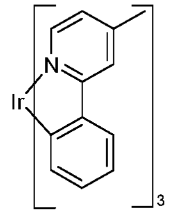

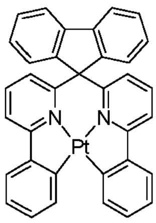
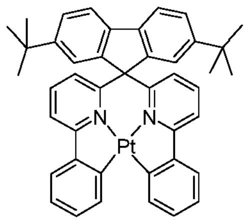
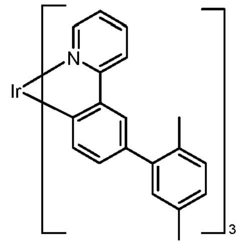
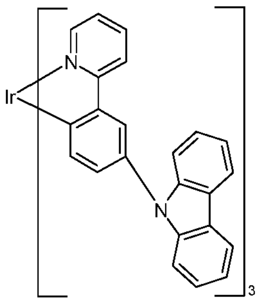
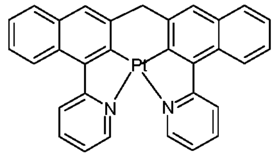
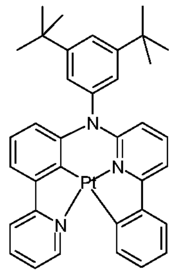
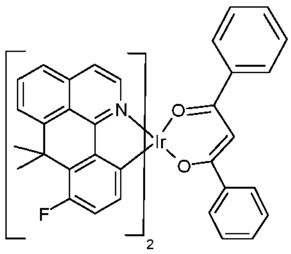
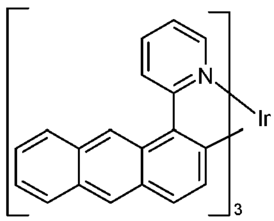
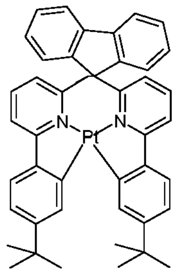
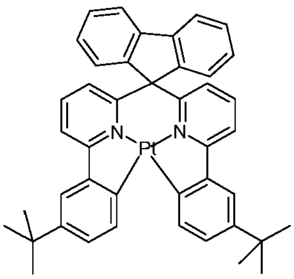
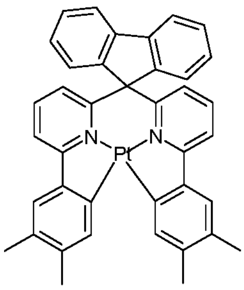
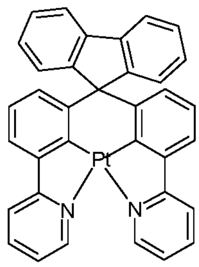
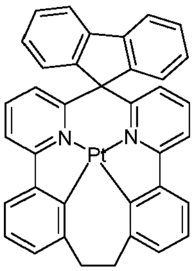
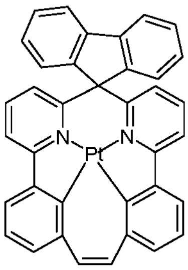
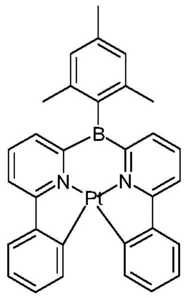

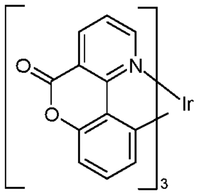

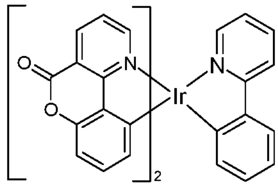

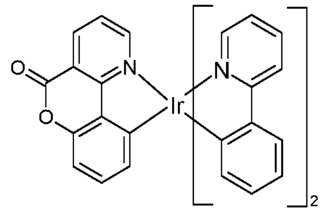

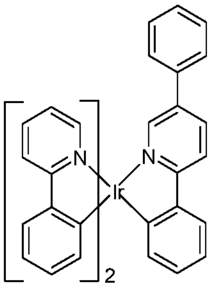

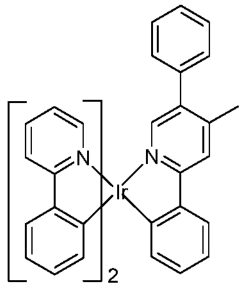

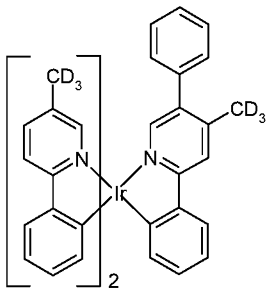

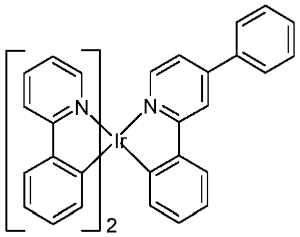

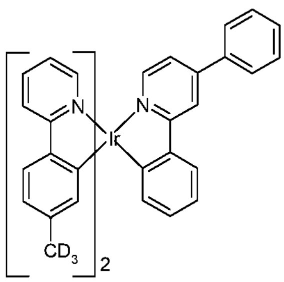

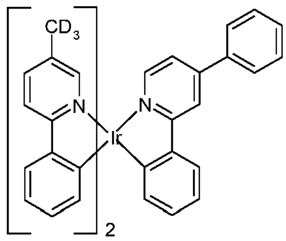

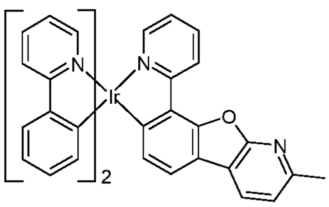

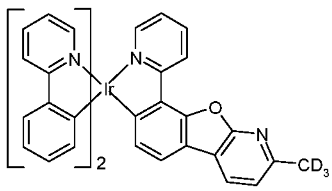

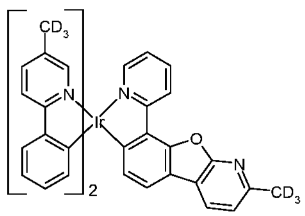

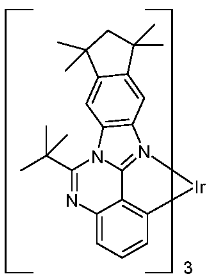

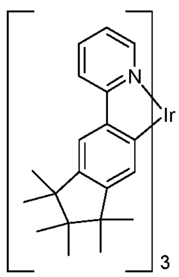

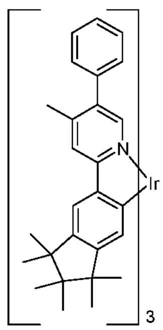

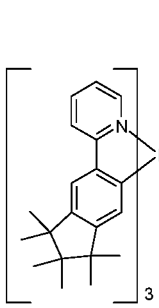
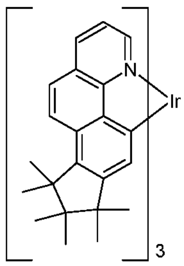
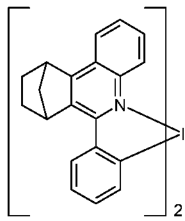

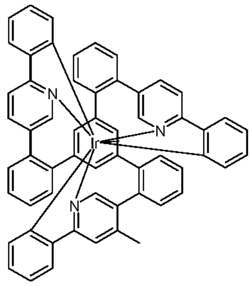
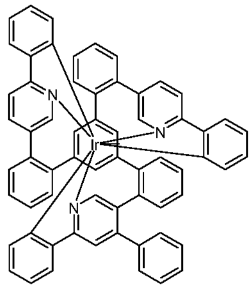
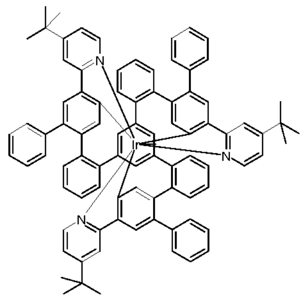

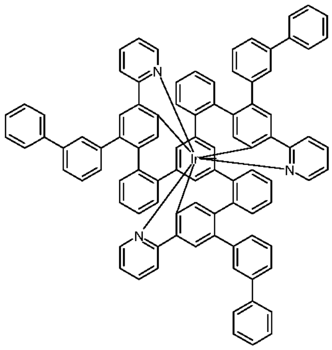
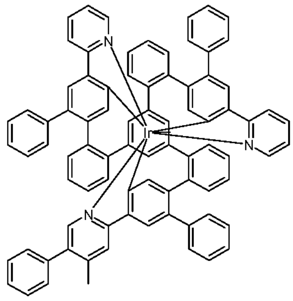
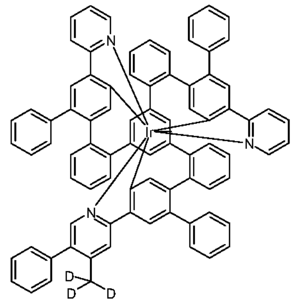

Die oben beschriebenen Verbindung, umfassend Strukturen der Formeln (IIIa), (IIIb), (IIIc) oder (IIId) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formeln (IIIa), (IIIb), (IIIc) oder (IIId) enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formeln (IIIa), (IIIb), (IIIc) oder (IIId). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen bzw. Systeme, welche mehr als drei emittierende Schichten aufweisen. Weiterhin bevorzugt sind auch Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die erfindungsgemäße Verbindung umfassend Strukturen gemäß einer der Formeln (IIIa), (IIIb), (IIIc) oder (IIId) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß einer der Formeln (IIIa), (IIIb), (IIIc) oder (IIId) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-Spirocarbazol-Derivate, z. B. gemäß WO 2014/094963 oder WO 2015/192939, oder Dibenzofuran-Derivate, z. B. gemäß WO 2015/169412, WO 2016/015810, WO 2016/023608 oder den noch nicht offen gelegten Anmeldungen EP 16158460.2 und EP 16159829.7. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

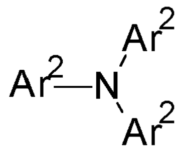

Formel (TA-1)

wobei $Ar^2$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit

einem oder mehreren Resten $R^3$ substituiert sein kann, wobei das Symbol $R^2$ die zuvor genannte Bedeutung aufweist. Vorzugsweise stellt $Ar^2$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^2$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^2$ gleich oder verschieden bei jedem Auftreten ausgewählt aus den oben genannten Gruppen $R^1$-1 bis $R^1$-80, besonders bevorzugt $R^1$-1 bis $R^1$-51.

In einer bevorzugten Ausführungsform der Verbindungen der Formel (TA-1) ist mindestens eine Gruppe $Ar^2$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^2$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^2$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^2$ ausgewählt aus einer Fluoren- oder Spirobifluorengruppe, insbesondere einer 4-Fluoren- bzw. 4-Spirobifluorengruppe, und eine Gruppe $Ar^2$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^2$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

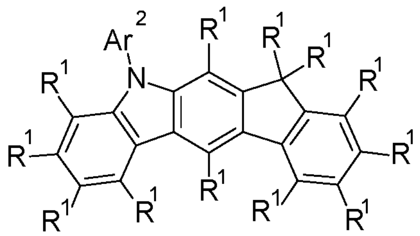

Formel (TA-2)

wobei $Ar^2$ und $R^1$ die oben insbesondere für Formeln (I) und/oder (TA-1) aufgeführten Bedeutungen aufweisen. Dabei sind bevorzugte Ausführungsformen der Gruppe $Ar^2$ die oben aufgeführten Strukturen $R^1$-1 bis $R^1$-80, besonders bevorzugt $R^1$-1 bis $R^1$-51.

Eine bevorzugte Ausführungsform der Verbindungen der Formel (TA-2) sind die Verbindungen der folgenden Formel (TA-2a),

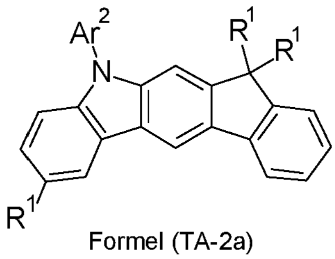

Formel (TA-2a)

wobei $Ar^2$ und $R^1$ die oben aufgeführten Bedeutungen aufweisen. Dabei stehen die beiden Gruppen $R^1$, die an das

Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

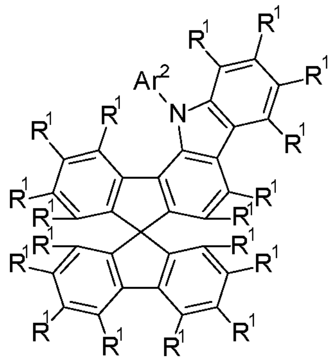

Formel (TA-3)

wobei $Ar^2$ und $R^1$ die oben aufgeführten Bedeutungen aufweisen. Dabei sind bevorzugte Ausführungsformen der Gruppe $Ar^2$ die oben aufgeführten Strukturen $R^1$-1 bis $R^1$-80, besonders bevorzugt $R^1$-1 bis $R^1$-51.

Eine bevorzugte Ausführungsform der Verbindungen der Formel (TA-3) sind die Verbindungen der folgenden Formel (TA-3a),

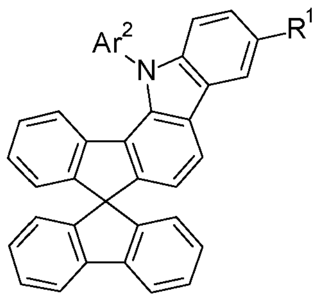

Formel (TA-3a)

wobei $Ar^2$ und $R^1$ die oben aufgeführten Bedeutungen aufweisen. Dabei sind bevorzugte Ausführungsformen der Gruppe $Ar^2$ die oben aufgeführten Strukturen $R^1$-1 bis $R^1$-80, besonders bevorzugt $R^1$-1 bis $R^1$-51.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

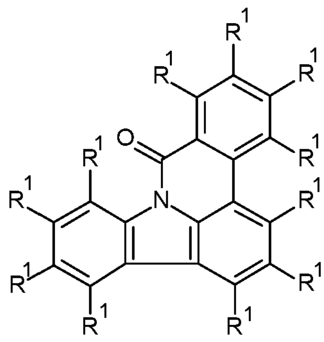

Formel (LAC-1)

wobei $R^1$ die oben aufgeführte Bedeutung aufweist.

Eine bevorzugte Ausführungsform der Verbindungen der Formel (LAC-1) sind die Verbindungen der folgenden Formel (LAC-1a),

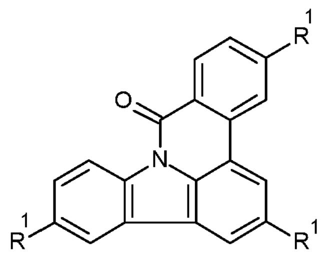

Formel (LAC-1a)

wobei $R^1$ die oben genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß eine der Formeln (IIIa), (IIIb), (IIIc) oder (IIId) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß einer der Formeln (IIIa), (IIIb), (IIIc) oder (IIId) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten

zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß einer der Formeln (IIIa), (IIIb), (IIIc) oder (IIId) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder $WO_3$, oder (per)fluorierte elektronenarme Aromaten eignen. Weitere geeignete p-Dotanden sind HAT-CN (Hexacyanohexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von übli-

cherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung umfassend Strukturen gemäß einer der Formeln (IIIa), (IIIb), (IIIc) oder (IIId) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend erfindungsgemäße Verbindungen umfassend Strukturen gemäß einer der Formeln (IIIa), (IIIb), (IIIc) oder (IIId) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen, insbesondere als elektronenleitende Materialien und/oder Lochleitermaterialien oder als Matrixmaterialien, weisen eine sehr gute Lebensdauer auf.

2. Elektronische Vorrichtungen, insbesondere organische Elektrolumineszenzvorrichtungen enthaltend Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen insbesondere als Elektronentransportmaterialien, Lochleitermaterialien und/oder als Hostmaterialien weisen eine hervorragende Effizienz auf. Insbesondere ist die Effizienz deutlich höher gegenüber analogen Verbindungen, die keine Struktureinheit gemäß einer der Formeln (IIIa), (IIIb), (IIIc) oder (IIId) enthalten. Hierbei bewirken erfindungsgemäßen Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d. h. einen geringen Abfall der Leistungseffizienz der Vorrichtung bei hohen Leuchtdichten.

3. Elektronische Vorrichtungen, insbesondere organische Elektrolumineszenzvorrichtungen enthaltend Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen als Elektronentransportmaterialien, Lochleitermaterialien und/oder als Hostmaterialien weisen eine hervorragende Farbreinheit auf.

4. Die erfindungsgemäßen Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen zeigen eine sehr hohe thermische und photochemische Stabilität und führen zu Verbindungen mit einer sehr hohen Lebensdauer.

5. Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen zeichnen sich durch eine ausgezeichnete thermische Stabilität aus, wobei Verbindungen mit einer Molmasse von weniger als ca. 1200 g/mol gut sublimierbar sind.

6. Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen weisen eine ausgezeichnete Glasfilmbildung auf.

7. Verbindungen, Oligomere, Polymere oder Dendrimere mit Strukturen gemäß einer der Formeln (IIIa), (IIIb), (IIIc) oder (IIId) bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als als Hostmaterial, Lochleitmaterial, Elektroneninjektionsmaterial und/oder Elektronentransportmaterial, vorzugsweise als Hostmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß einer der Formeln (IIIa), (IIIb), (IIIc) oder (IIId)bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat

verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

**Beispiele**

Die nachfolgenden Synthesen werden, sofern nicht anders angegeben, unter einer Schutzgasatmosphäre in getrockneten Lösungsmitteln durchgeführt. Die Edukte können von ALDRICH bezogen werden. Die Nummern bei den literaturbekannten Edukten, die teilweise in eckigen Klammern angegeben sind, sind die entsprechenden CAS-Nummern.

**Synthesebeispiele**

**a) (9-Oxo-9H-xanthen-1-yl)-urea**

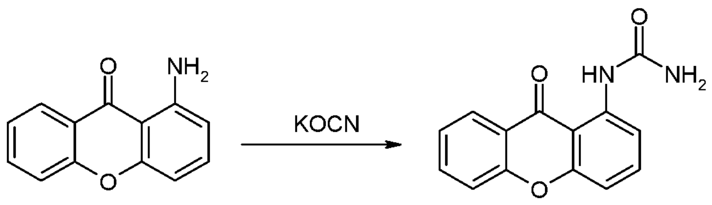


[54829-44-6 ]

Zu einer entgasten Lösung von 51,1 g (244 mmol) 1-Amino-xanthen-9-on in 1300 ml AcOH gibt man portionsweise 27 g (319 mmol) KOCN über einen Zeitraum von 5h bei Raumtemperatur. Das Lösungsmittel wird im Vakuum entfernt und anschließend mit $CH_2CL_2$, dann mit Wasser versetzt. Nach Phasentrennung wird das Produkt chromatographisch gereinigt (90:3, $CH_2Cl_2$/MeOH). Ausbeute: 38 g (150 mmol), 62 % d. Th.; Reinheit: 93 % n. HPLC

Analog können folgende Verbindungen hergestellt werden:

| | Edukt 1 | Produkt | Ausbeute |
|---|---|---|---|
| 1a | 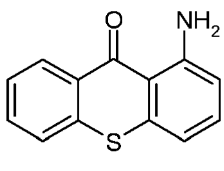 [40021-31-6 ] | 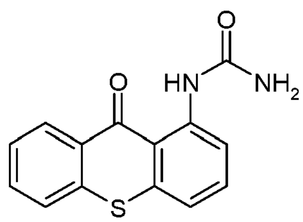 | 59% |

(fortgesetzt)

| | Edukt 1 | Produkt | Ausbeute |
|---|---|---|---|
| 2a | 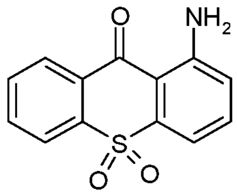 96583-32-3 ] | 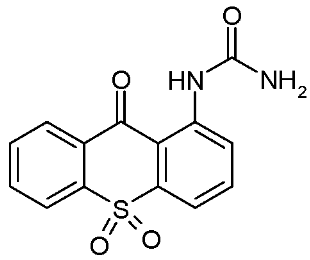 | 61% |
| 3a | 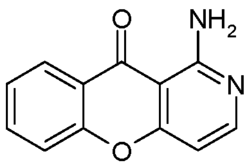 [124557-83-1 ] | 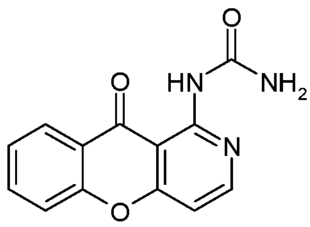 | 60% |
| 4a | 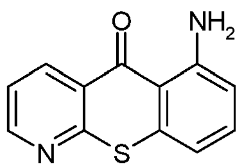 [151050-99-6 ] | 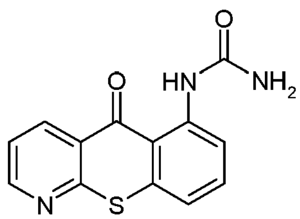 | 63% |

**b) 3H-7-Oxa-1,3-diaza-benzo[de]anthracen-2-on**

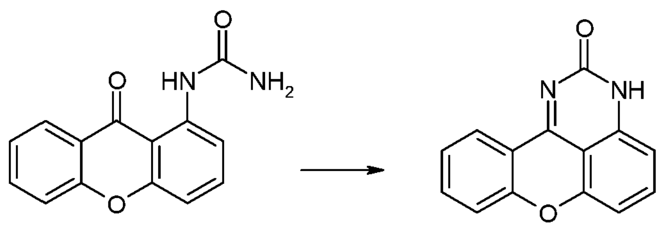

Zu einer Lösung aus 21 g (86 mmol) (9-Oxo-9H-xanthen-1-yl)-urea in 1500 ml EtOH werden 11,7 g (190 mmol) KOH zugegeben und für 40 min. zum Sieden erhitzt. Nach der Abkühlung wird das Gemisch mit $CH_2Cl_2$ versetzt, abfiltriert und mit Wasser versetzt. Nach Phasentrennung wird aus Aceton umkristallisiert. Ausbeute: 17,5 g (74 mmol) 3H-7-Oxa-1,3-diaza-benzo[de]anthracen-2-on, 90 % d. Th.; Reinheit: 93 % n. HPLC.

Analog können folgende Verbindungen hergestellt werden:

| | Edukt 1 | Produkt | Ausbeute |
|---|---|---|---|
| 1b | 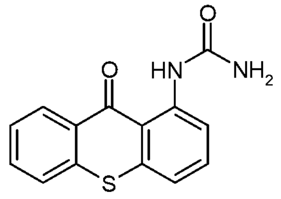 | 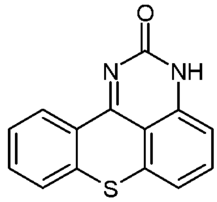 | 87% |
| 2b | 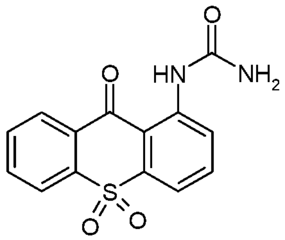 | 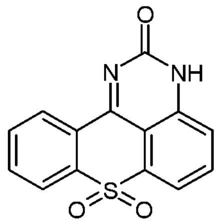 | 91% |
| 3b | 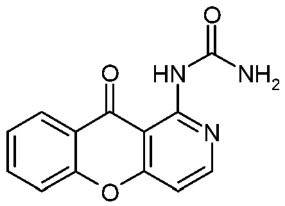 | 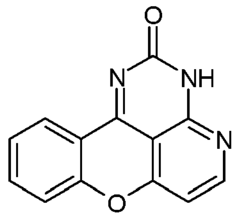 | 88% |
| 4b | 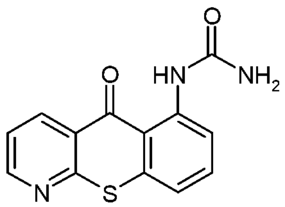 | 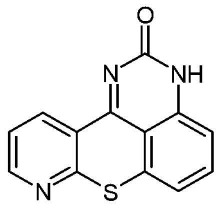 | 87% |

**c) 2-Chloro-7-oxa-1,3-diaza-benzo[de]anthracen**

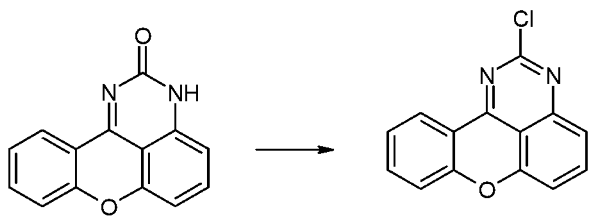

14,1 g (60 mmol) 3H-7-Oxa-1,3-diaza-benzo[de]anthracen-2-on werden in 200 ml $POCl_3$ bei 85 °C 5 h gerührt. Anschließend wird das $POCl_3$ im Vakuum entfernt, und durch Zugabe von 1N NaOH wird der pH auf 7 eingestellt. Dann wird mit $CH_2Cl_2$ extrahiert. Das Lösungsmittel wird im Vakuum abgezogen, und das Produkt wird unter Schutzgas aus Hexan umkristallisiert. Ausbeute:14,7 g (58 mmol), 97 % d. Th.; Reinheit: 93 % n. HPLC.

Analog können folgende Verbindungen hergestellt werden:

| | Edukt 1 | Produkt | Ausbeute |
|---|---|---|---|
| 1c | 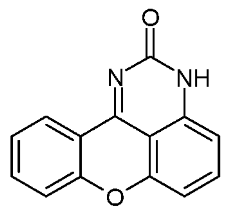 | 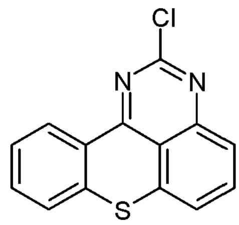 | 87% |
| 2c | 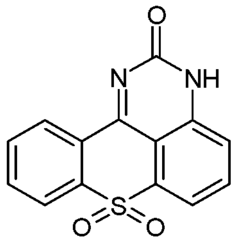 | 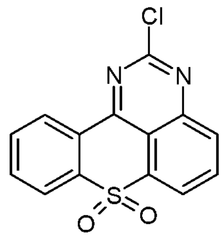 | 91% |
| 3c | 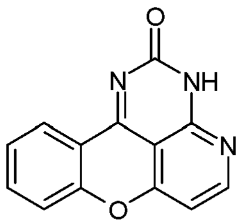 | 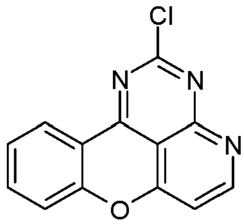 | 88% |
| 4c | 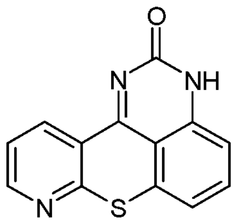 | 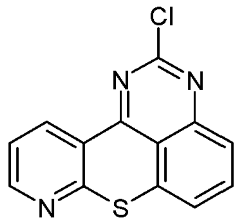 | 87% |

**d) 2-Chloro-4-(2-methylsulfanyl-phenyl)-chinazolin**

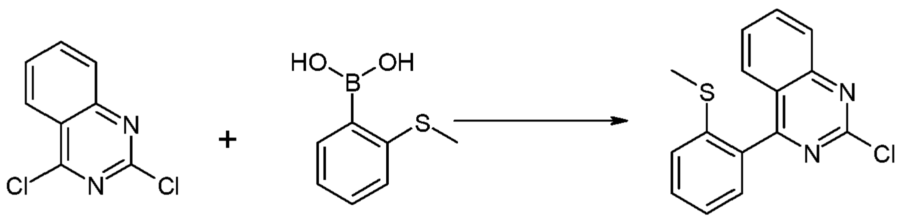

[607-68-1] [168618-42-6 ]

73 g (370 mmol) 2,4-Dichloro-chinazolin, 58 g (350 mmol) 2-Methylsulfanylphenylboronsäure und 100 g (475 mmol) $K_2CO_3$ werden in 400 ml THF und 400 ml Wasser suspendiert, das Gemisch mit $N_2$ gesättigt, 1,6 g (1,5 mmol) Tetrakis(triphenylphosphin)-palladium(0) zugegeben und für 2 h zum Sieden erhitzt. Das Gemisch wird in 1 L einer Mischung aus Wasser/MeOH/6 M HCl 1:1:1 gegossen und der beige Niederschlag abgesaugt, mit Wasser gewaschen und getrocknet. Ausbeute: 86 g (300 mmol), 82 % d. Th.; Reinheit: 95 % n. HPLC.

Analog können folgende Verbindungen hergestellt werden:

| | Edukt 1 | Edukt 2 | Produkt | Ausbeute |
|---|---|---|---|---|
| 1d | 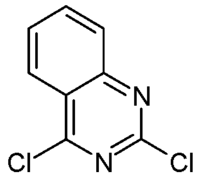 [607-68-1] | 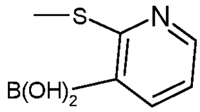 [1072944-21-8 ] | 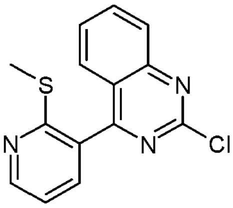 | 89% |
| 2d | 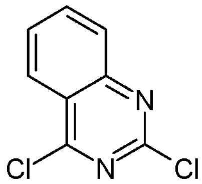 [607-68-1] | 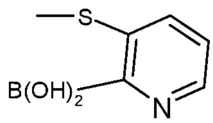 [1590418-34-0 ] | 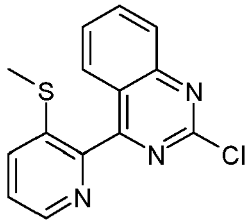 | 90% |
| 3d | 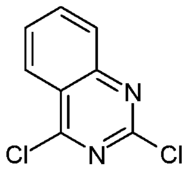 [607-68-1] | 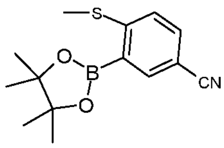 [863868-33-1 ] | 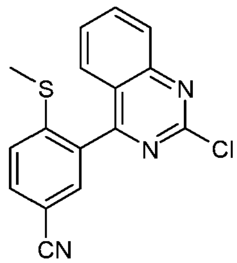 | 91% |
| 4d | 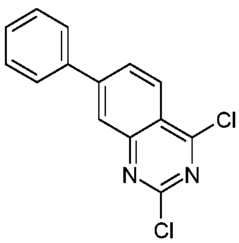 [1852465-65-6 ] | 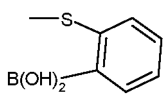 [168618-42-6 ] | 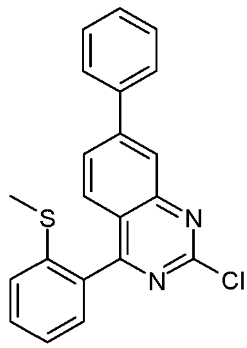 | 92% |
| 5d | 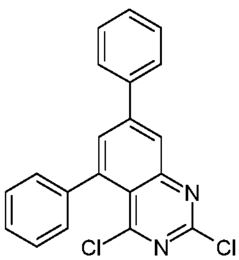 [1821394-14-2] | 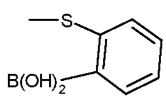 [168618-42-6 ] | 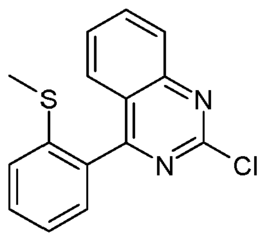 | 95% |

(fortgesetzt)

| | Edukt 1 | Edukt 2 | Produkt | Ausbeute |
|---|---|---|---|---|
| 6d | 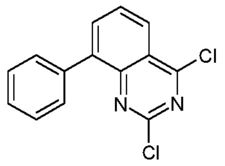 [1152237-20-1 ] | 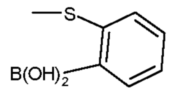 [168618-42-6 ] | 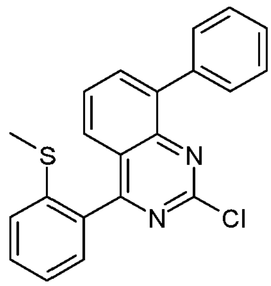 | 91% |
| 7d | 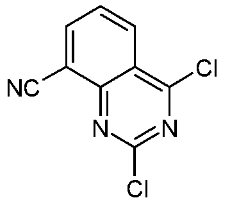 [1150617-71-2 ] | 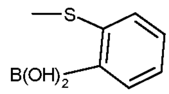 [168618-42-6 ] | 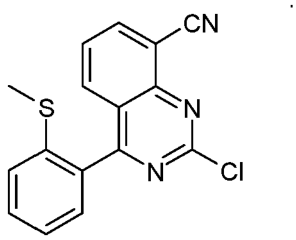 | 90% |
| 8d | 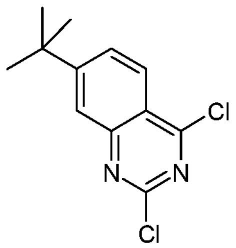 [1093985-82-0 ] | 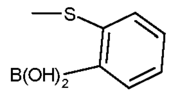 [168618-42-6 ] | 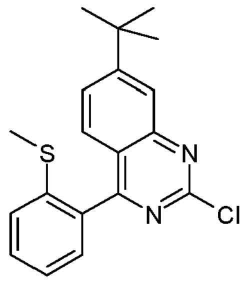 | 87% |
| 9d | 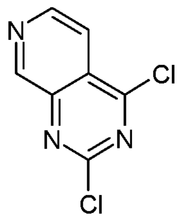 [908240-50-6 ] | 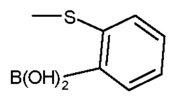 [168618-42-6 ] | 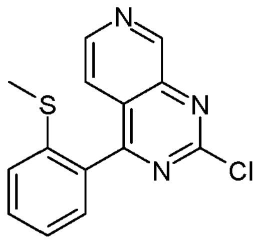 | 95% |
| 10d | 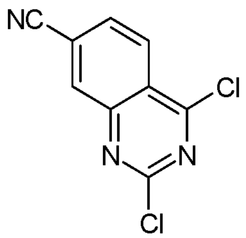 [864292-40-0 ] | 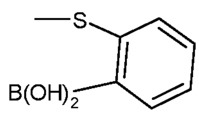 [168618-42-6 ] | 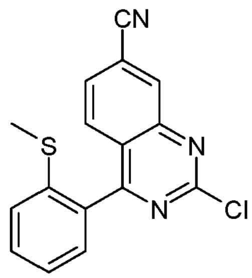 | 85% |

(fortgesetzt)

| | Edukt 1 | Edukt 2 | Produkt | Ausbeute |
|---|---|---|---|---|
| 11d | 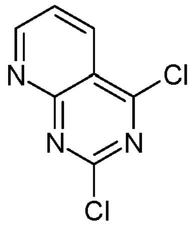 [126728-20-9 ] | 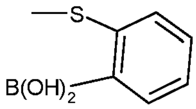 [168618-42-6 ] | 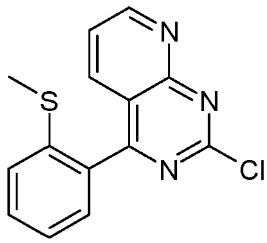 | 87% |
| 12d | 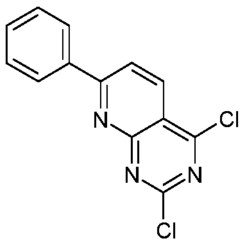 [95845-38-8 ] | 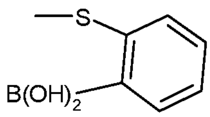 [168618-42-6 ] | 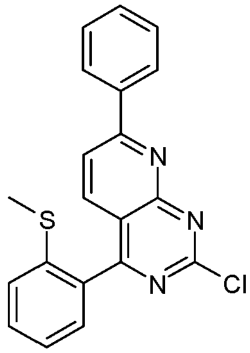 | 91% |

**e) 2-Chloro-4-(2-methansulfinyl-phenyl)-chinazolin**

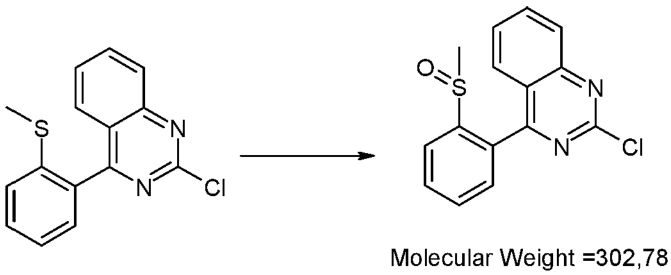

Molecular Weight =302,78

Molecular Weight =286,79

Unter Schutzgas werden 86 g (300 mmol) 2-Chloro-4-(2-methylsulfanyl-phenyl)-chinazolin in 1,1 L Eisessig und 125 mL Dichlormethan vorgelegt und auf 0 °C gekühlt. Zu dieser Lösung werden 500 mL (309 mmol) 30% $H_2O_2$-Lösung zugetropft und über Nacht gerührt. Das Gemisch wird mit $Na_2SO_3$ Lösung versetzt, die Phasen werden getrennt und das Lösungsmittel im Vakuum entfernt. Ausbeute: 86 g (285 mmol), 80 % d. Th.; Reinheit: 96 % n. HPLC.

Analog können folgende Verbindungen hergestellt werden:

| | Edukt 1 | Produkt | Ausbeute |
|---|---|---|---|
| 1e | 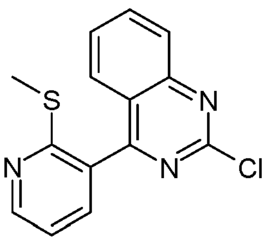 | 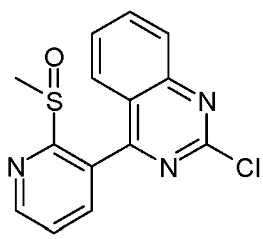 | 78% |

(fortgesetzt)

| | Edukt 1 | Produkt | Ausbeute |
|---|---|---|---|
| 2e | 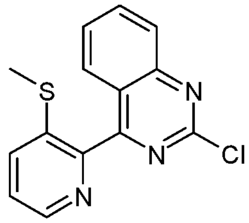 | 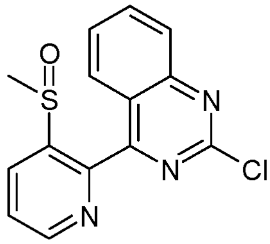 | 75% |
| 3e | 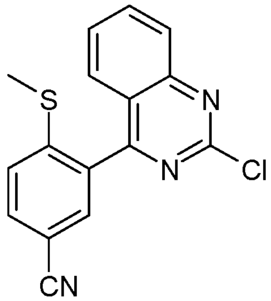 | 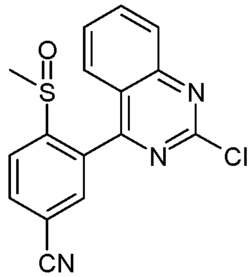 | 77% |
| 4e | 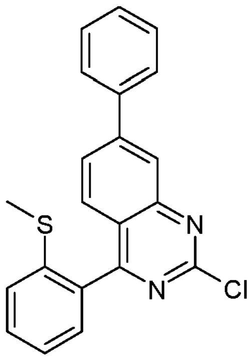 | 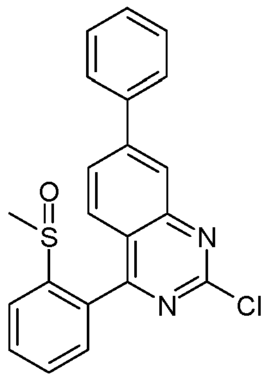 | 76% |
| 5e | 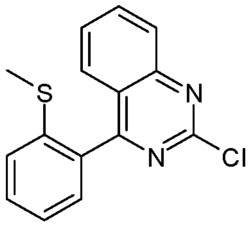 | 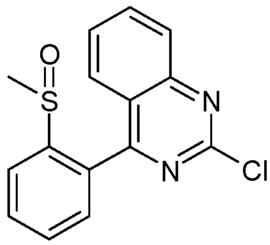 | 78% |
| 6e | 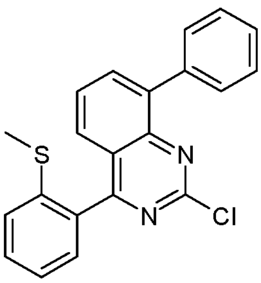 | 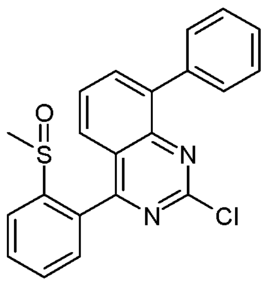 | 76% |

(fortgesetzt)

| | Edukt 1 | Produkt | Ausbeute |
|---|---|---|---|
| 7e | 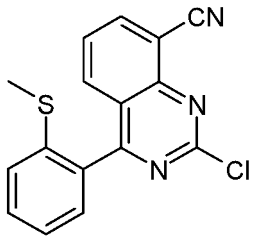 | 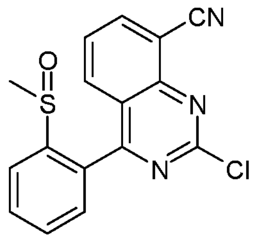 | 68% |
| 8e | 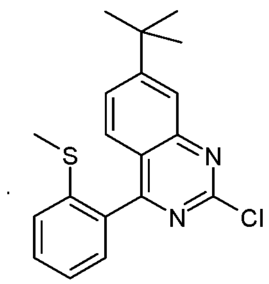 | 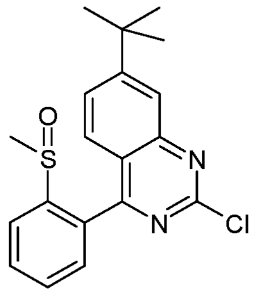 | 88% |
| 9e | 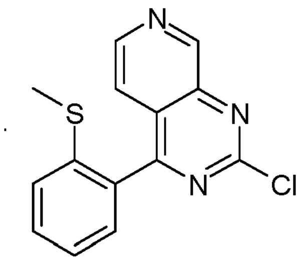 | 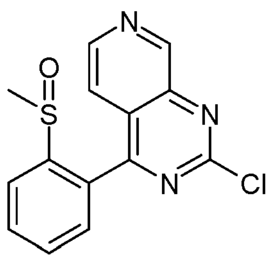 | 82% |
| 10e | 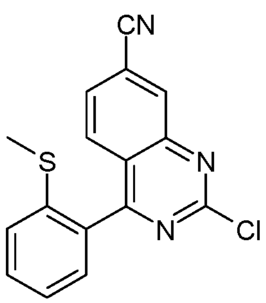 | 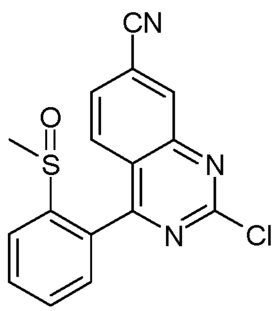 | 84% |
| 11e | 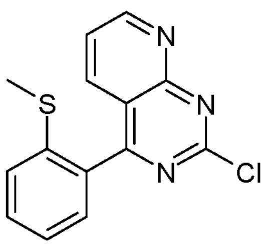 | 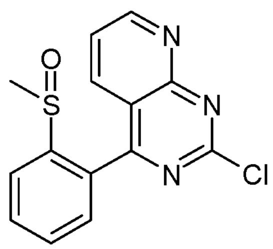 | 80% |

(fortgesetzt)

| | Edukt 1 | Produkt | Ausbeute |
|---|---|---|---|
| 12e | 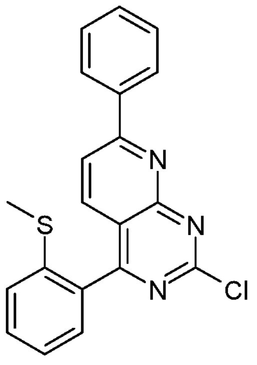 | 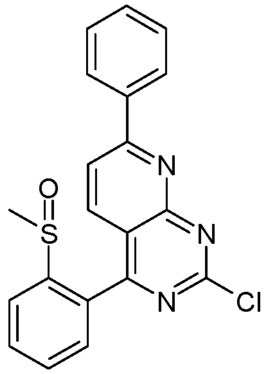 | 87% |
| 13e | 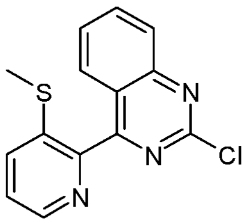 | 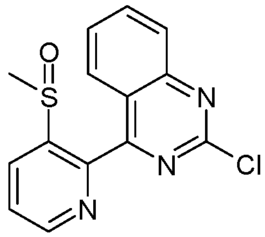 | 88% |
| 14e | 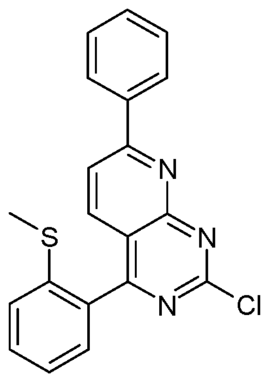 | 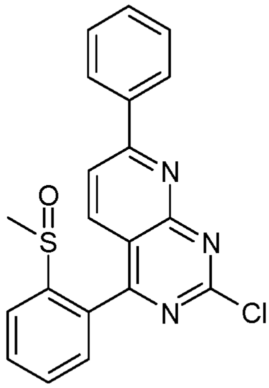 | |

**f) 2-Chloro-7-thia-1,3-diaza-benzo[de]anthracen**

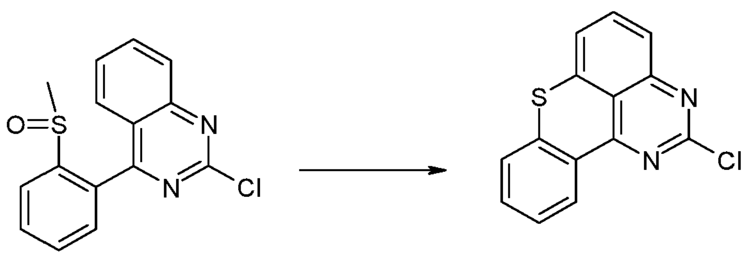

Eine Mischung aus 85 g (280 mmol) 2-Chloro-4-(2-methansulfinyl-phenyl)-chinazolin und 737 ml (8329 mmol) Trifluormethansulfonsäure wird 48 h bei 5 °C gerührt. Anschließend wird die Mischung mit 2,4 L Wasser/Pyridin 5:1 versetzt und 20 min. unter Rückfluss erhitzt. Nach Abkühlen auf Raumtemperatur werden vorsichtig 500 ml Wasser und 1000 ml Dichlormethan zugesetzt. Die organische Phase wird mit 4 x 50 mL $H_2O$ gewaschen, über $MgSO_4$ getrocknet und die Lösungsmittel im Vakuum entfernt. Das reine Produkt erhält man durch Umkristallisation aus Toluol. Ausbeute: 72g (267 mmol), 95 % d. Th.; Reinheit: 95 % n. HPLC.

Analog können folgende Verbindungen hergestellt werden:

| | **Edukt 1** | **Produkt** | **Ausbeute** |
|---|---|---|---|
| 1f | 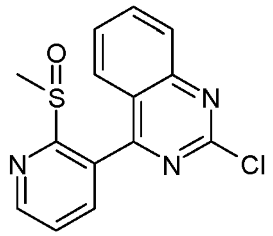 | 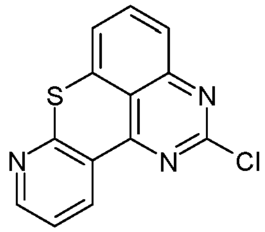 | 91% |
| 2f | 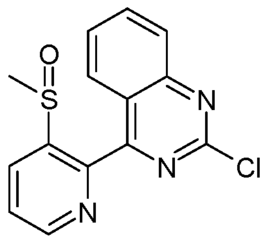 | 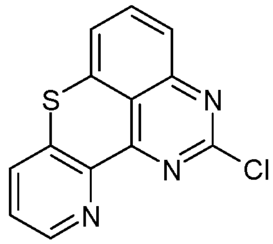 | 79% |
| 3f | 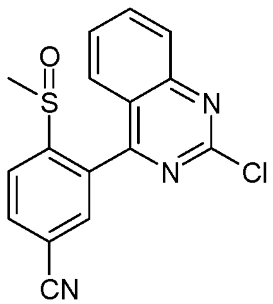 | 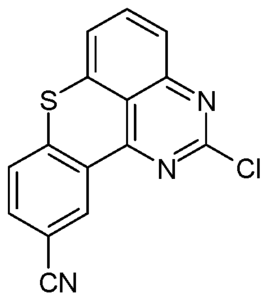 | 87% |
| 4f | 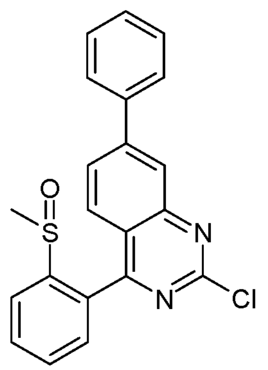 | 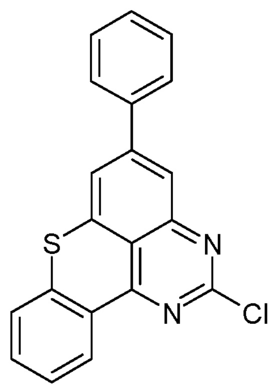 | 85% |
| 5f | 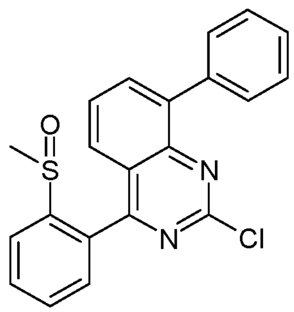 | 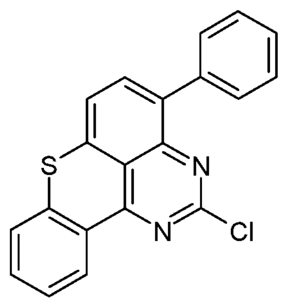 | 91% |

(fortgesetzt)

| | Edukt 1 | Produkt | Ausbeute |
|---|---|---|---|
| 6f | 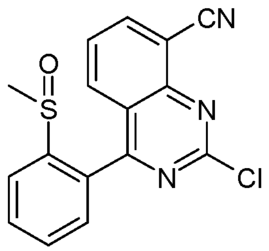 | 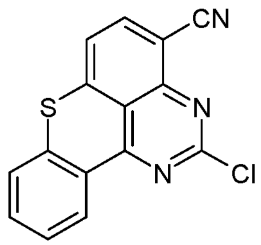 | 87% |
| 7f | 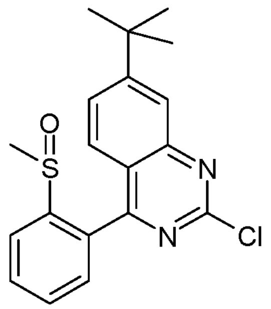 | 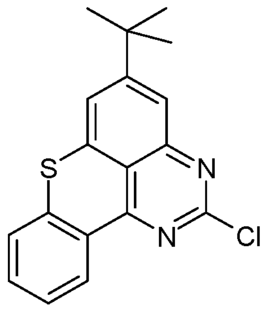 | 89% |
| 8f | 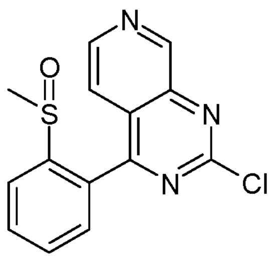 | 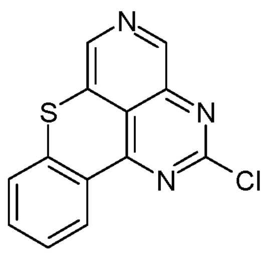 | 92% |
| 9f | 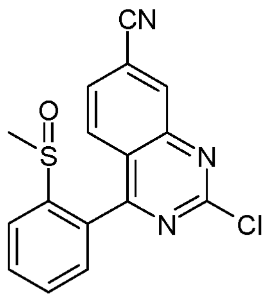 | 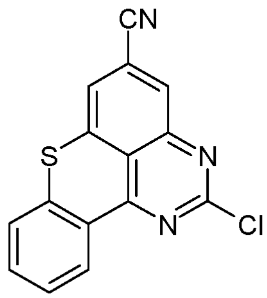 | 85% |
| 10f | 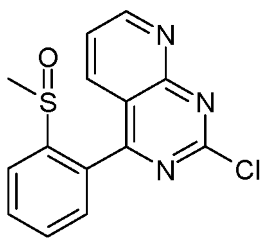 | 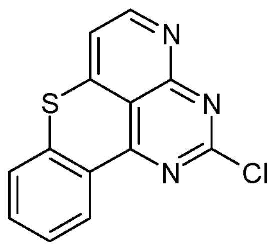 | 87% |

(fortgesetzt)

| | Edukt 1 | Produkt | Ausbeute |
|---|---|---|---|
| 11f | 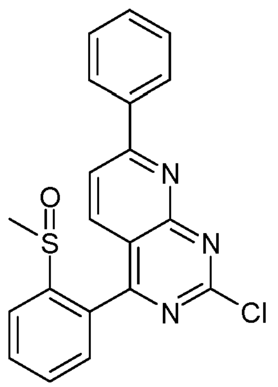 | 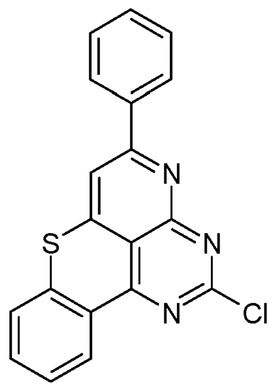 | 76% |
| 12f | 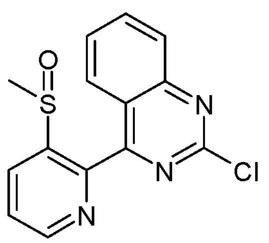 | 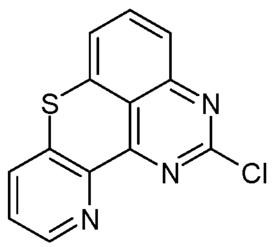 | 91% |
| 13f | 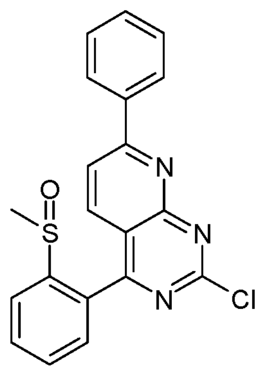 | 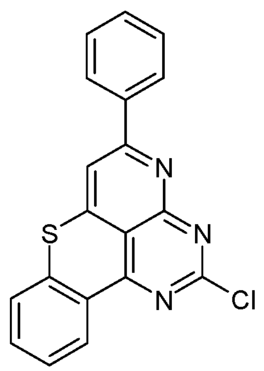 | 89% |

**g) 4-(7-Thia-1,3-diaza-benzo[de]anthracen-2-yl)-14H-13-thia-14-aza-benzo[c]indeno[2,1-a]fluoren** (nicht Gegenstand der Erfindung)

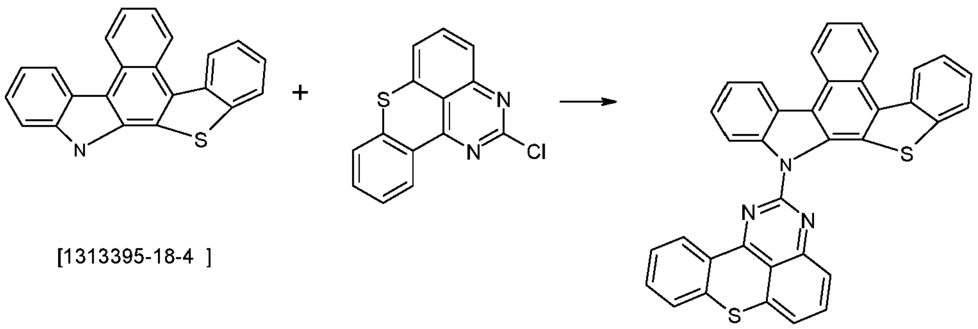

[1313395-18-4 ]

16,2 g (60 mmol) 14H-13-Thia-14-aza-benzo[c]indeno[2,1-a]fluoren werden in 300 ml Dimethylformamid unter Schutzgasatmosphäre gelöst und mit 3 g NaH (75 mmol), 60%ig in Mineralöl versetzt. Nach 1 h bei Raumtemperatur

wird eine Lösung von 16,7 g (62 mmol) 2-Chloro-7-thia-1,3-diaza-benzo[de]anthracen in 150 mL Dimethylformamid zugetropft. Das Reaktionsgemisch wird 12 h bei Raumtemperatur gerührt, dann auf Eis gegossen und dreimal mit Dichlormethan extrahiert. Die vereinigten organischen Phasen werden über $Na_2SO_4$ getrocknet und eingeengt. Der Rückstand wird mit Toluol umkristallisiert und abschließend zweimal fraktioniert sublimiert (p ca. $10^{-6}$ mbar, T = 375-390 °C). Ausbeute: 26,7 g (47 mmol), 80 % d. Th.; Reinheit: 99,9 % n. HPLC.

Analog können folgende Verbindungen hergestellt werden:

| | Edukt 1 | Edukt 2 | Produkt | Ausbeute |
|---|---|---|---|---|
| 1g | 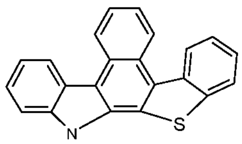 [1313395-18-4 ] | 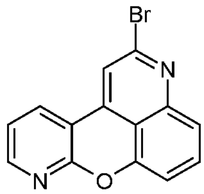 [151988-17-9 ] | 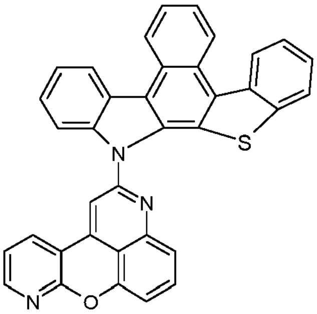 | 76% |
| 2g* | 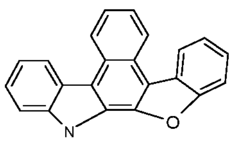 [1678511-52-8 ] | 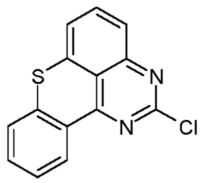 | 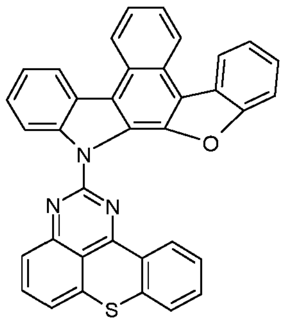 | 80% |
| 3g* | 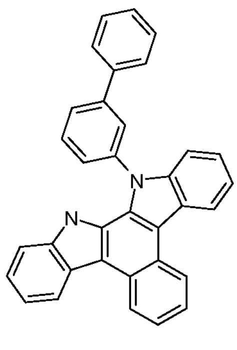 [1639394-10-7 ] | 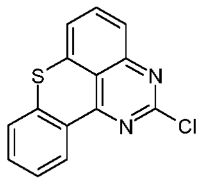 | 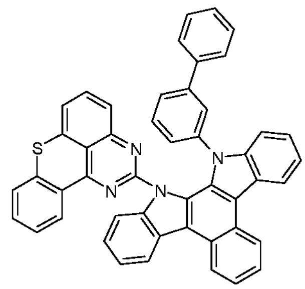 | 86% |
| 4g* | 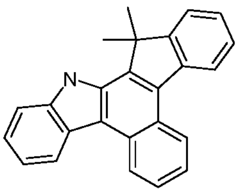 [1447709-49-0 ] | 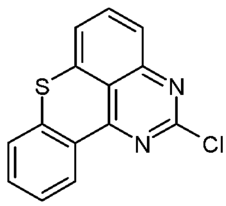 | 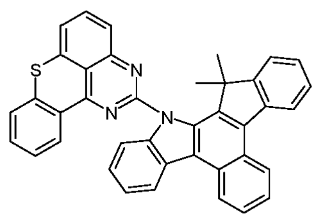 | 81% |

(fortgesetzt)

| | Edukt 1 | Edukt 2 | Produkt | Ausbeute |
|---|---|---|---|---|
| 5g* | 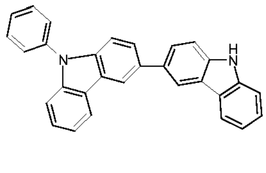 [ 1345202-03-0] | 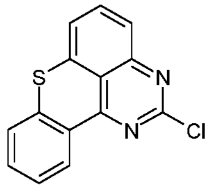 | 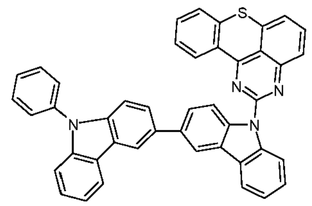 | 82% |
| 6g* | 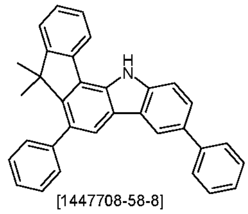 [1447708-58-8] | 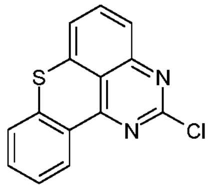 | 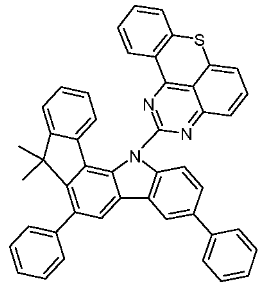 | 78% |
| 7g* | 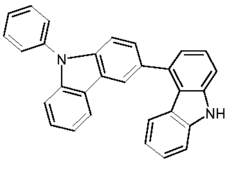 [ 1407183-66-7] | 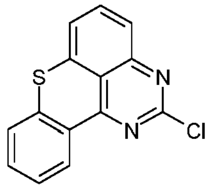 | 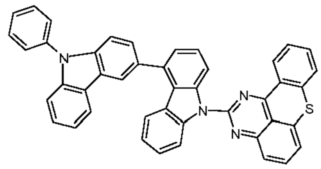 | 75% |
| 8g* | 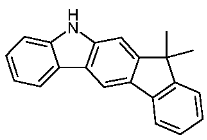 [1257220-47-5] | 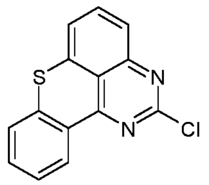 | 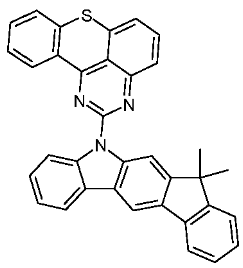 | 77% |
| 9g* | 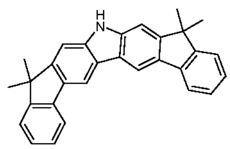 [1373281-72-1] | 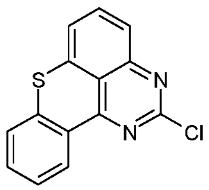 | 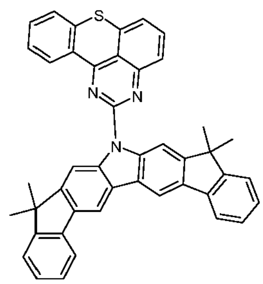 | 80% |
| 10 g* | 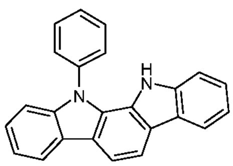 [1024598-06-8] | 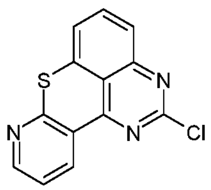 | 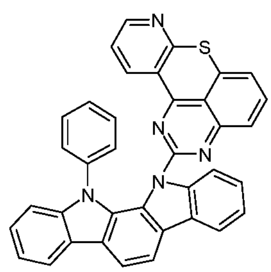 | 84% |

(fortgesetzt)

| | Edukt 1 | Edukt 2 | Produkt | Ausbeute |
|---|---|---|---|---|
| 11 g* | 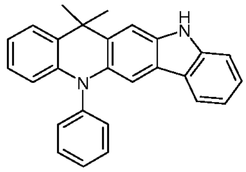 [1361126-04-6] | 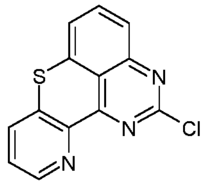 | 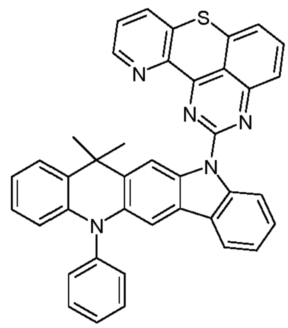 | 89% |
| 12 g* | 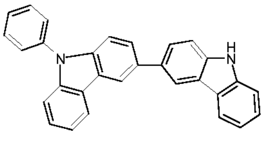 [ 1345202-03-0] | 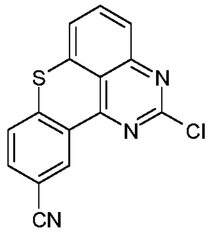 | 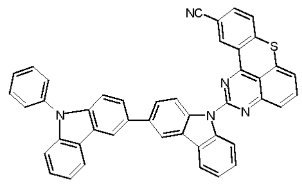 | 85% |
| 13 g* | 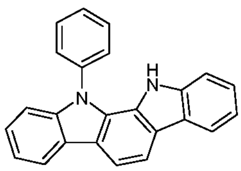 [1024598-06-8] | 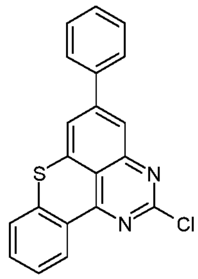 | 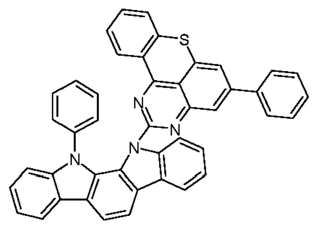 | 84% |
| 14 g* | 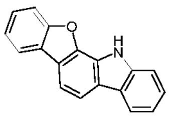 [1338919-70-2] | 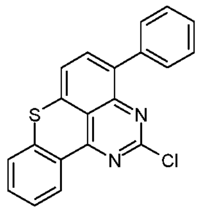 | 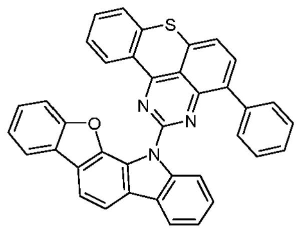 | 83% |
| 15 g* | 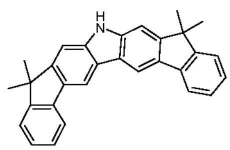 [1373281-72-1] | 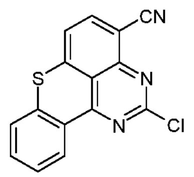 | 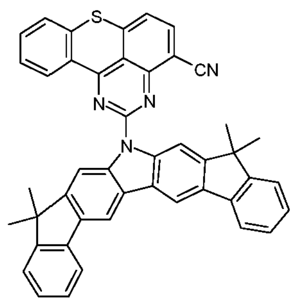 | 9^1% |

(fortgesetzt)

| | Edukt 1 | Edukt 2 | Produkt | Ausbeute |
|---|---|---|---|---|
| 16 g* | 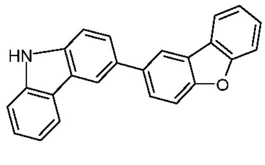 [953805-18-0] | 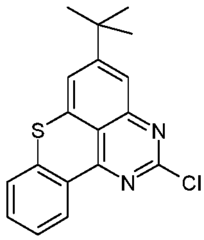 | 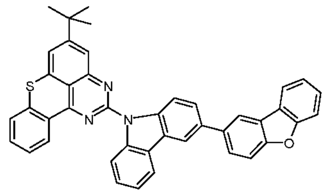 | 83% |
| 17 g* | 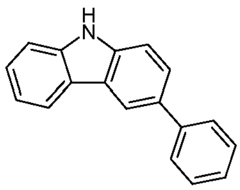 [103012-26-6 ] | 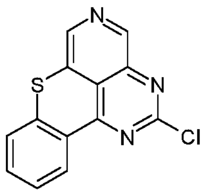 | 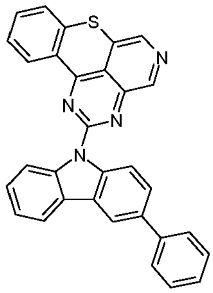 | 85% |
| 18 g* | 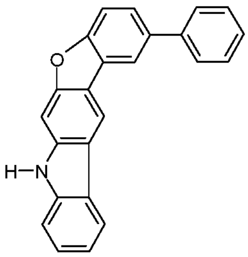 [1439927-96-4] | 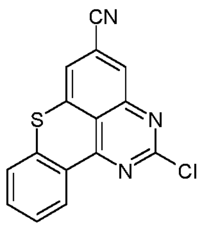 | 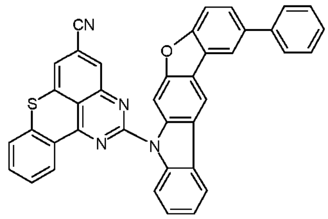 | 84% |
| 19 g* | 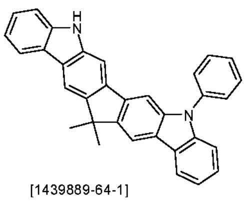 [1439889-64-1] | 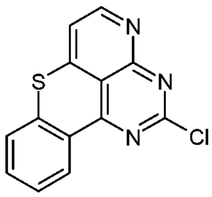 | 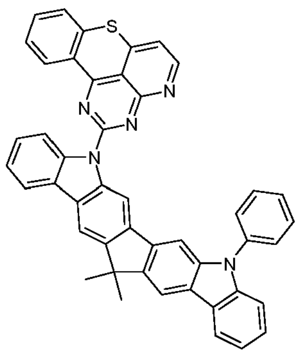 | 73% |
| 20 g* | 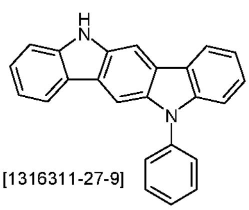 [1316311-27-9] | 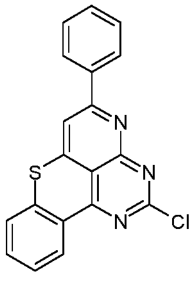 | 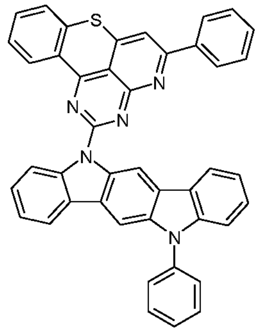 | 74% |

(fortgesetzt)

| | Edukt 1 | Edukt 2 | Produkt | Ausbeute |
|---|---|---|---|---|
| 21 g* | 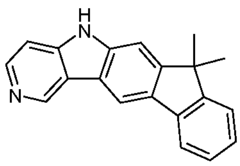 [1257248-14-8] | 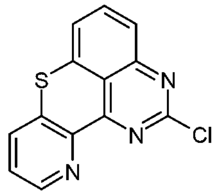 | 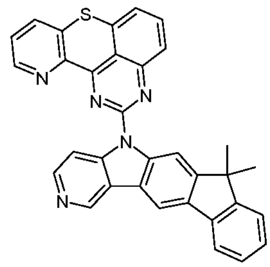 | 81% |
| 22 g* | 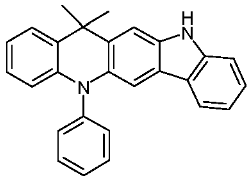 [1361126-04-6] | 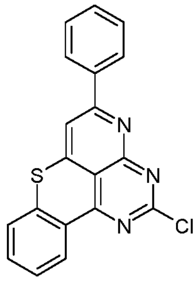 | 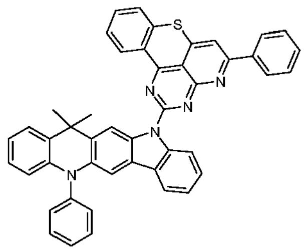 | 85% |
| 23 g | 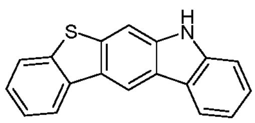 [1255309-04-6] | 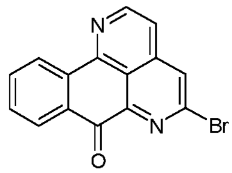 [760976-86-1] | 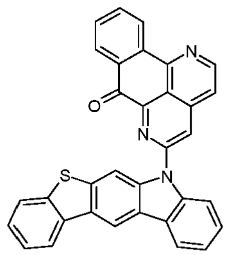 | 77% |
| 24 g | 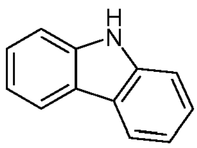 [96-74-8] | 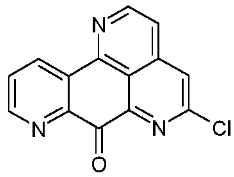 [741281-31-2 ] | 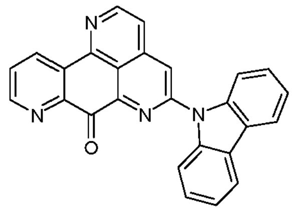 | 87% |
| 25 g* | 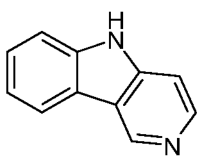 [223713-94-8] | 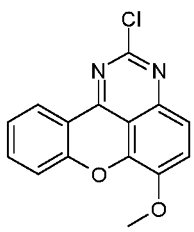 [957767-64-5] | 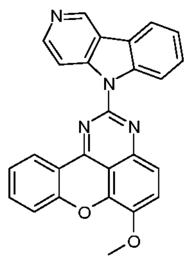 | 88% |

(fortgesetzt)

| | Edukt 1 | Edukt 2 | Produkt | Ausbeute |
|---|---|---|---|---|
| 26 g | 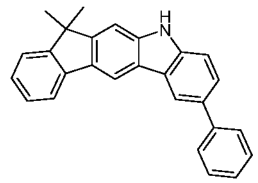 WO 2010/136109 | 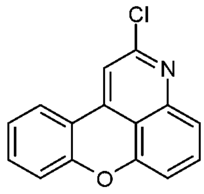 [853924-93-3 ] | 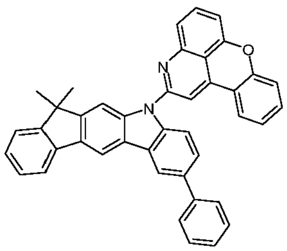 | 76% |
| 27 g* | 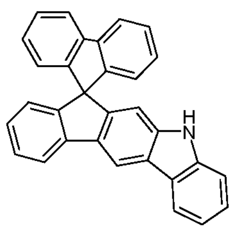 WO 2010/136109 | 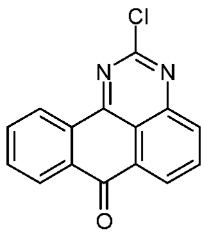 ]348616-78-4 ] | 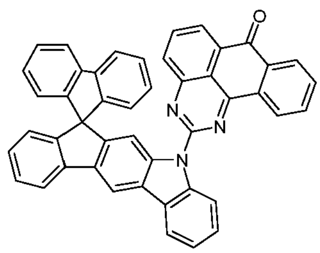 | 75% |
| 28 g | 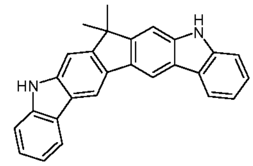 WO 2010/136109 | 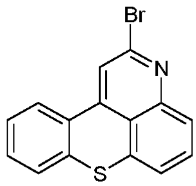 [159181-73-4 ] | 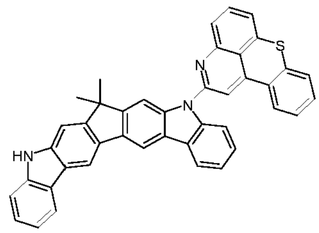 | 76% |
| 29 g | 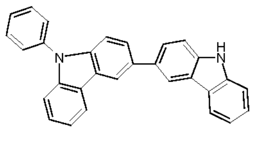 [ 1345202-03-0] | 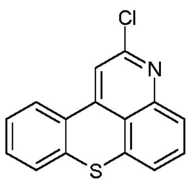 [159181-72-3 ] | 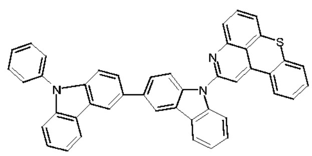 | 77% |
| 30 g | 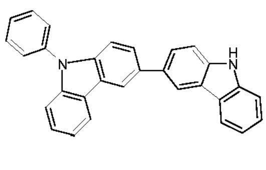 [ 1345202-03-0] | 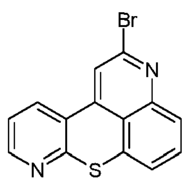 [151051-08-0 ] | 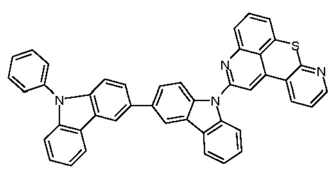 | 80% |

(fortgesetzt)

| | Edukt 1 | Edukt 2 | Produkt | Ausbeute |
|---|---|---|---|---|
| 31 g | 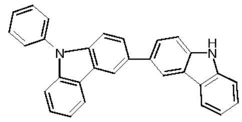 [ 1345202-03-0] | 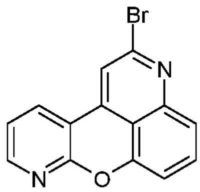 [151988-17-9 ] | 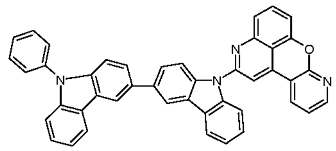 | 72% |
| 32 g* | 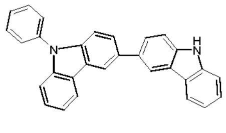 [ 1345202-03-0] | 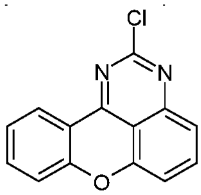 | 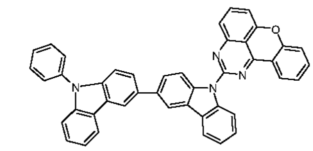 | 70% |
| 33 g | 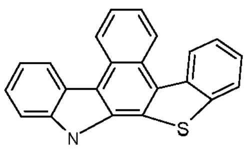 [1313395-18-4 ] | 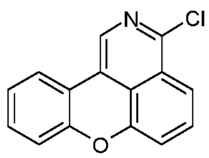 [59500-27-5] | 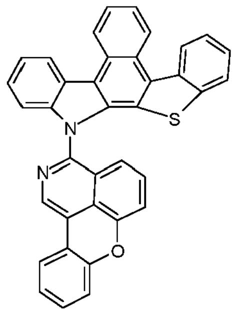 | 73% |
| 34 g* | 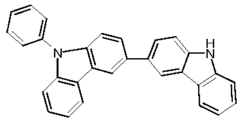 [ 1345202-03-0] | 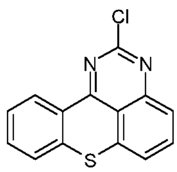 | 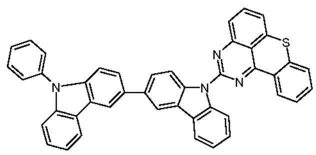 | 69% |
| 35 g | 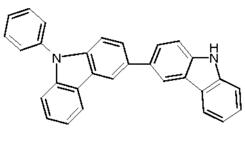 [ 1345202-03-0] | 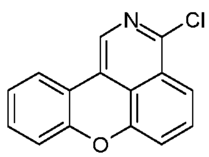 [59500-27-5] | 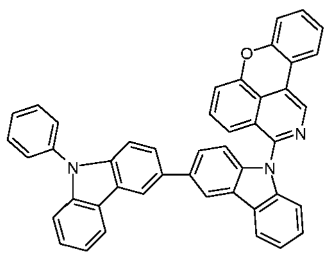 | 87% |
| 36 g | 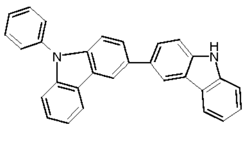 [ 1345202-03-0] | 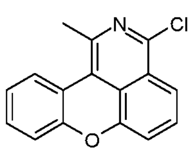 [72240-51-8 ] | 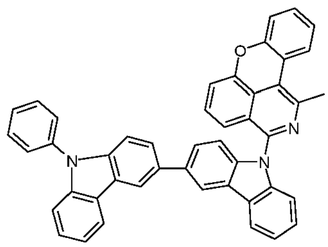 | 72% |

(fortgesetzt)

| | Edukt 1 | Edukt 2 | Produkt | Ausbeute |
|---|---|---|---|---|
| 37 g | 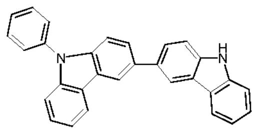 [ 1345202-03-0] | 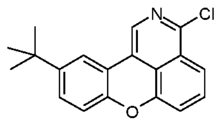 [59500-40-2 ] | 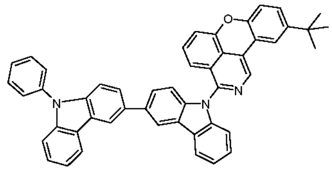 | 70% |
| 38 g | 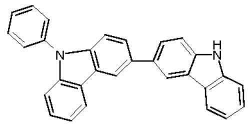 [ 1345202-03-0] | 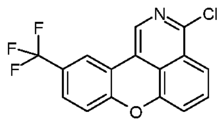 [59500-30-0] | 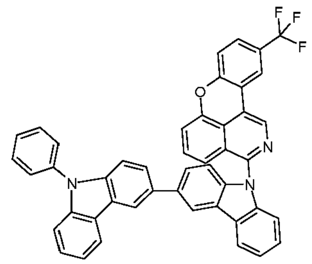 | 81% |
| 39 g | 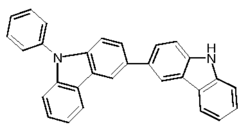 [ 1345202-03-0] | 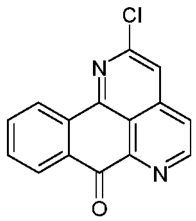 [1025849-63-1 ] | 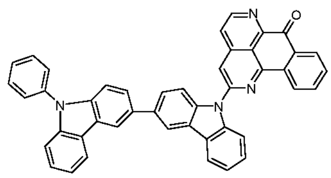 | 78% |
| 40 g* | 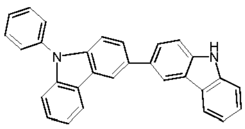 [ 1345202-03-0] | 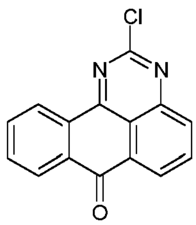 [348616-78-4 ] | 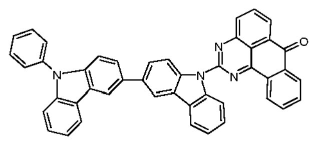 | 76% |
| 41 g* | 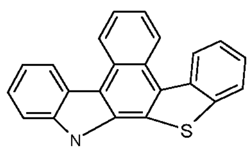 [1313395-18-4 ] | 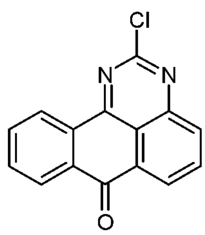 [348616-78-4 ] | 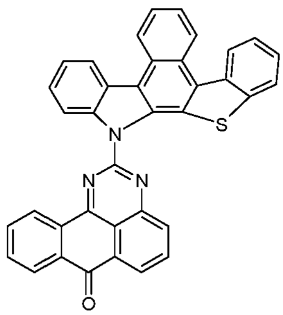 | 77% |
| 42 g* | 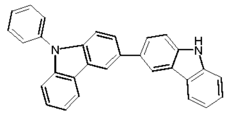 [ 1345202-03-0] | 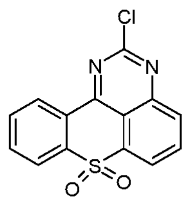 | 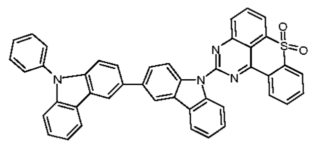 | 71% |

(fortgesetzt)

| | Edukt 1 | Edukt 2 | Produkt | Ausbeute |
|---|---|---|---|---|
| 43 g* | 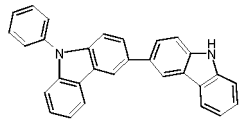 [ 1345202-03-0] | 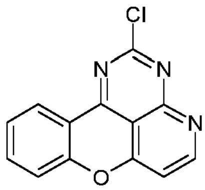 | 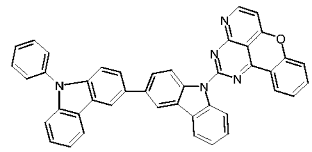 | 69% |
| 44 g* | 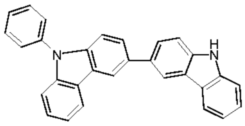 [ 1345202-03-0] | 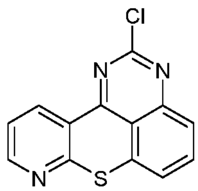 | 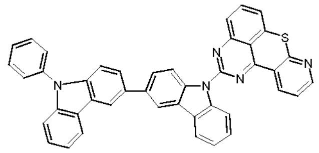 | 60% |
| 45 g* | 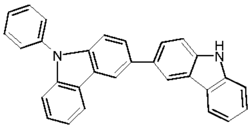 [ 1345202-03-0] | 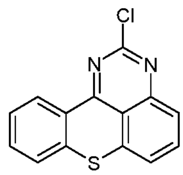 | 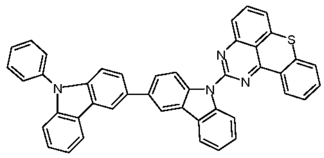 | 74% |
| 46 g* | 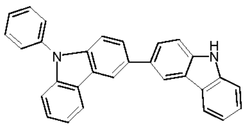 [ 1345202-03-0] | 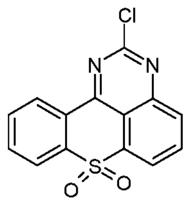 | 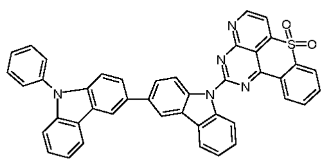 | 72% |
| 47 g* | 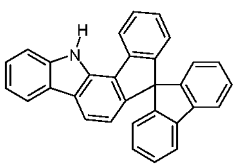 [1615703-28-0] | 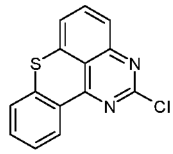 | 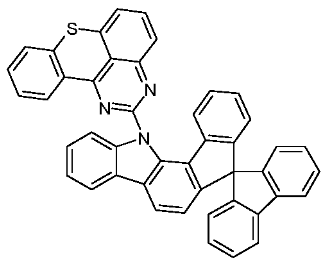 | 70% |
| 48 g* | 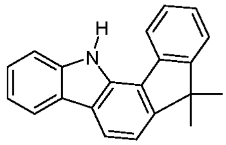 [1616231-39-0] | 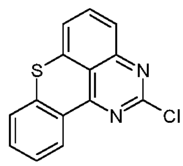 | 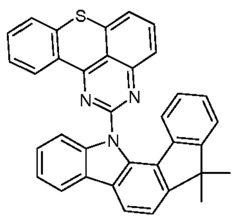 | 67% |
| * nicht Gegenstand der Erfindung | | | | |

**Herstellung der OLEDs**

In den folgenden Beispielen E1 bis E8 (siehe Tabelle 1) wird der Einsatz der erfindungsgemäßen Materialien in OLEDs vorgestellt.

**Vorbehandlung für die Beispiele E1-E8:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs

aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TER1 (50%:45%:5%) bedeutet hierbei, dass das Material IC1 in einem Volumenanteil von 50%, IC2 in einem Anteil von 45% und TER1 in einem Anteil von 5% in der Schicht vorliegt. Analog kann auch die Elektronentransportschicht aus einer Mischung von zwei Materialien bestehen.

Die OLEDs werden standardmäßig charakterisiert. Die Elektrolumineszenzspektren werden bei einer Leuchtdichte von 1000 $cd/m^2$ bestimmt und daraus die CIE 1931 x und y Farbkoordinaten berechnet.

**Verwendung von erfindungsgemäßen Mischungen in OLEDs**

Die erfindungsgemäßen Materialien können in der Emissionsschicht in phosphoreszierenden roten OLEDs eingesetzt werden. Die erfindungsgemäßen Verbindungen EG1 bis EG6 werden in den Beispielen E1 bis E8 als Matrixmaterial in der Emissionsschicht eingesetzt. Die Farbkoordinaten der Elektrolumineszenzspektren der OLEDs liegen bei CIEx = 0,67 und CIEy = 0,33. Somit eignen sich die Materialien für den Einsatz in der Emissionsschicht rot phosphoreszierender OLEDs.

Des Weiteren lassen sich die erfindungsgemäßen Materialien in der Lochblockierschicht (HBL) oder Elektronenblockierschicht (EBL) erfolgreich einsetzen. Dies ist in den Beispielen E7 und E8 gezeigt. Auch hier liegen die Farbkoordinaten des Spektrums der OLED bei CIEx = 0,67 und CIEy = 0,33.

Tabelle 1: Aufbau der OLEDs

| Bsp. | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 125nm | SpMA2 10nm | IC2:EG1:TER1 (50%:45%: 5%) 40nm | --- | ST1:LiQ (50%: 50%) 35nm | --- |
| E2* | HATCN 5nm | SpMA1 125nm | SpMA2 10nm | EG2:TER1 (95%:5%) 40nm | --- | ST1:LiQ (50%: 50%) 35nm | --- |
| E3* | HATCN 5nm | SpMA1 125nm | SpMA2 10nm | IC1:EG3:TER1 (50%:45%: 5%) 40nm | --- | ST1:LiQ (50%: 50%) 35nm | --- |
| E4* | HATCN 5nm | SpMA1 125nm | SpMA2 10nm | EG4:TER1 (95%:5%) 40nm | --- | ST1:LiQ (50%: 50%) 35nm | --- |
| E5* | HATCN 5nm | SpMA1 125nm | SpMA2 10nm | IC2:EG5:TER1 (50%:45%: 5%) 40nm | --- | ST1:LiQ (50%: 50%) 35nm | --- |
| E6* | HATCN 5nm | SpMA1 125nm | SpMA2 10nm | IC2:EG6:TER1 (50%:45%: 5%) 40nm | --- | ST1:LiQ (50%: 50%) 35nm | --- |
| E7* | HATCN 5nm | SpMA1 125nm | SpMA2 10nm | EG4:TER1 (95%:5%) 40nm | EG4 5nm | ST1:LiQ (50%: 50%) 30nm | --- |
| E8* | HATCN 5nm | SpMA1 125nm | EG4 10nm | EG4:TER1 (95%:5%) 40nm | --- | ST1:LiQ (50%: 50%) 35nm | --- |
| * nicht Gegenstand der Erfindung | | | | | | | |

Tabelle 2: Strukturformeln der Materialien für die OLEDs

| | |
|---|---|
| 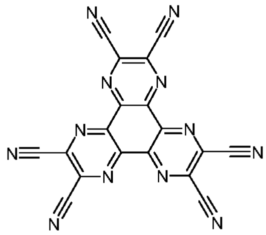 | 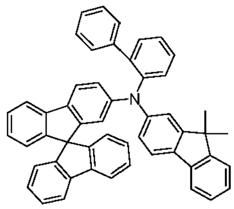 |
| HATCN | SpMA1 |
| 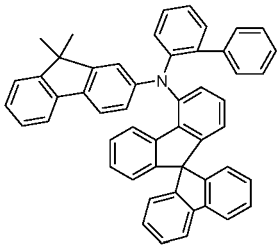 | 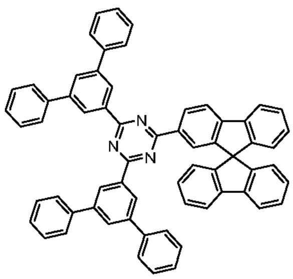 |
| SpMA2 | ST1 |
| 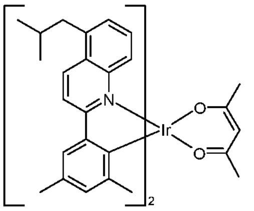 | 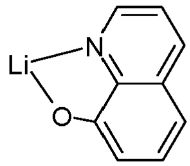 |
| TER1 | LiQ |
| 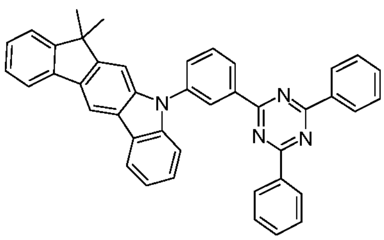 | 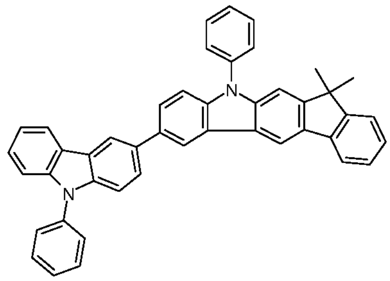 |
| IC1 | IC2 |
| 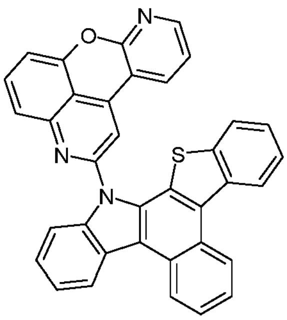 | 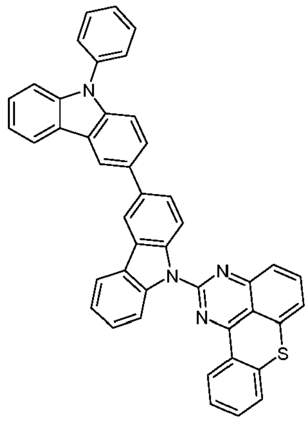 |

(fortgesetzt)

| EG1 | EG2* |
|---|---|
| 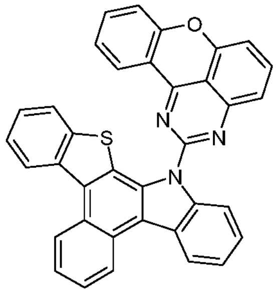 | 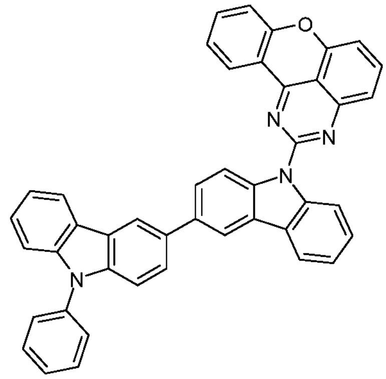 |
| EG3* | EG4* |
| 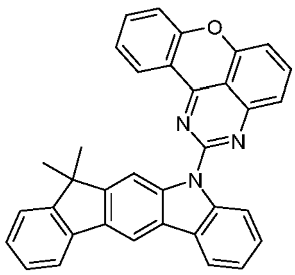 | 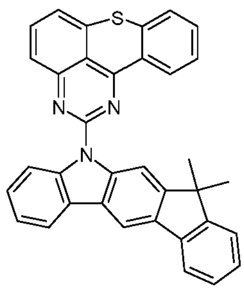 |
| EG5* | EG6* |
| * nicht Gegenstand der Erfindung | |

## Patentansprüche

**1.** Verbindung einer Formel (IIIa), (IIIb), (IIIc) oder (IIId):

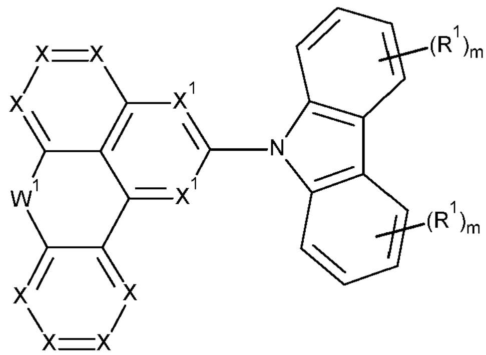

Formel (IIIa)

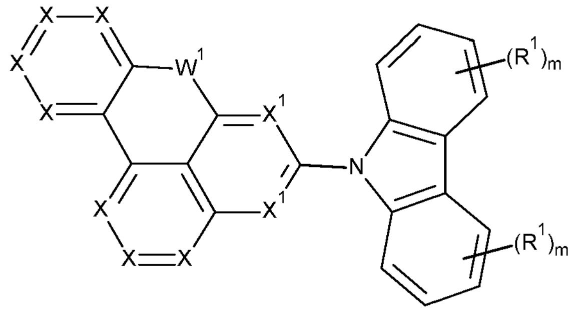

Formel (IIIb)

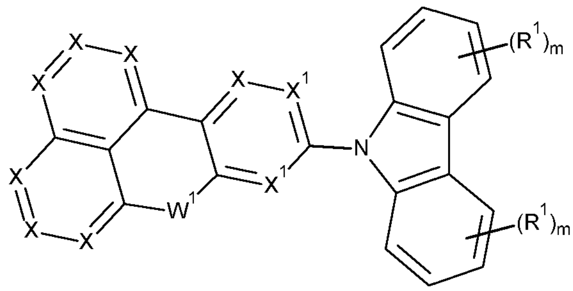

Formel (IIIc)

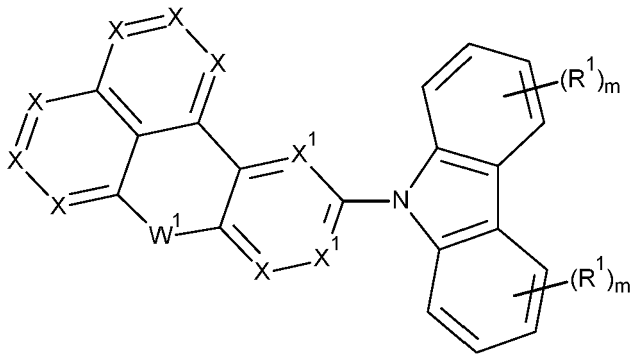

Formel (IIId)

wobei für die verwendeten Symbole gilt:

X ist bei jedem Auftreten gleich oder verschieden N oder $CR^1$;
$X^1$ ist N oder $CR^1$, wobei genau eine Gruppe $X^1$ für N steht;
$W^1$ ist O, S, SO, $SO_2$, $SiR^1_2$ oder C=O;
$R^1$ ist bei jedem Auftreten gleich oder verschieden H, D, F, Cl, Br, I, CN, $NO_2$, $N(Ar^1)_2$, $N(R^2)_2$, $OAr^1$, $OR^2$, $SAr^1$, $SR^2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenylgruppe mit 2 bis 40 C-Atomen, die jeweils mit einem oder mehreren Resten $R^2$ substituiert sein kann und wobei eine oder mehrere nicht benachbarte $CH_2$-Gruppen durch $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder eine Aralkyl- oder Heteroaralkylgruppe mit 5 bis 40 aromatischen Ringatomen, die mit einem oder mehreren

Resten $R^2$ substituiert sein kann, oder eine Kombination dieser Systeme; dabei können zwei oder mehrere Substituenten $R^1$ auch miteinander ein mono- oder polycyclisches, aliphatisches, heteroaliphatisches, aromatisches oder heteroaromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nichtaromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $OR^3$, $SR^3$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^{13})_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann und wobei eine oder mehrere nicht benachbarte $CH_2$-Gruppen durch $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr Substituenten $R^3$ miteinander ein mono- oder polycyclisches, aliphatisches Ringsystem bilden;

m ist gleich oder verschieden 0, 1, 2, 3, oder 4.

**2.** Verbindung gemäß Anspruch 1 einer Formel (IVa), (IVb), (IVc) oder (IVd),

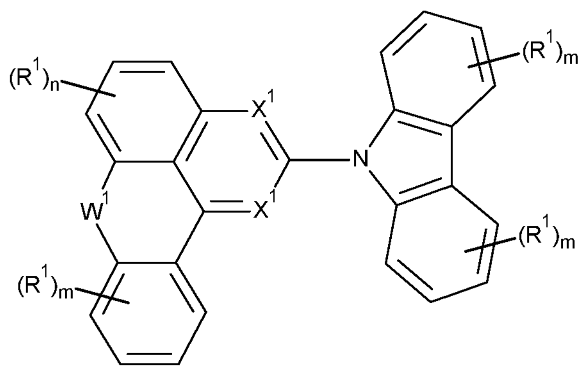

Formel (IVa)

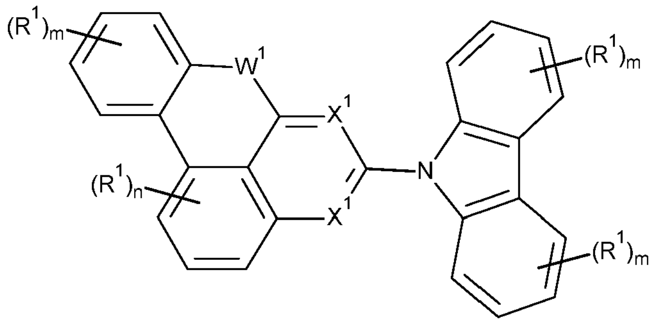

Formel (IVb)

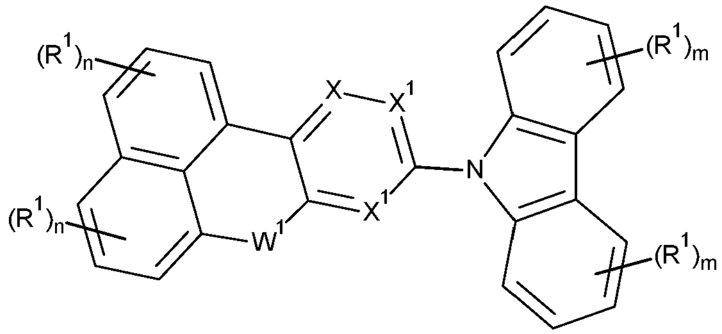

Formel (IVc)

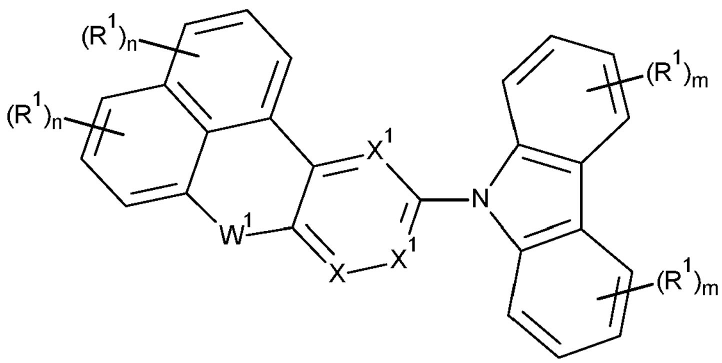

Formel (IVd)

wobei die Symbole $R^1$, $W^1$ m, X und $X^1$ die in Anspruch 1 genannte Bedeutung aufweisen, und n gleich oder verschieden 0, 1, 2, oder 3 ist.

**3.** Verbindung gemäß Anspruch 1 oder 2, **dadurch gekennzeichnet, dass** zwei benachbarte Reste $R^1$ einen Ring der Formel (DB-1), (DB-2) oder (DB-3) bilden,

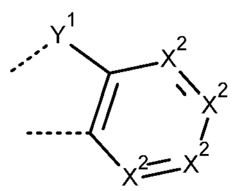

Formel (DB-1)

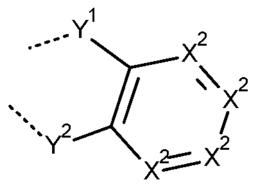

Formel (DB-2)

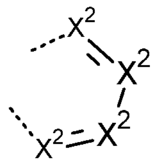

Formel (DB-3)

wobei $X^2$ gleich oder verschieden bei jedem Auftreten für N oder $CR^2$ steht, $Y^1$ und $Y^2$ bei jedem Auftreten unabhängig für O, S, $C(R^2)_2$ oder $NR^2$ stehen und die gestrichelten Linien die Bindung an die Aryl- oder Heteroarylgruppe darstellen, wobei $R^2$ die in Anspruch 1 genannte Bedeutung aufweist.

**4.** Verbindung gemäß Anspruch 3, einer Formel (VIa), (VIb), (VIc), (VId), (IXa), (IXb), (IXc), (IXd), (XIIa), (XIIb), (XIIc) oder (XIId),

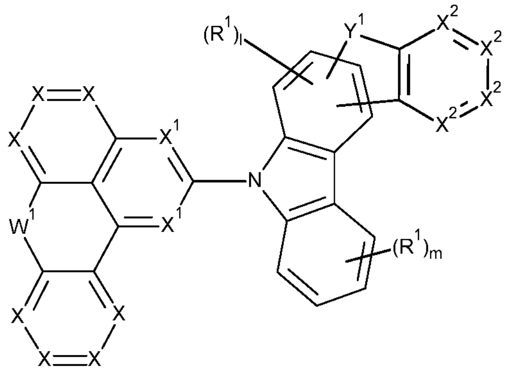

Formel (VIa)

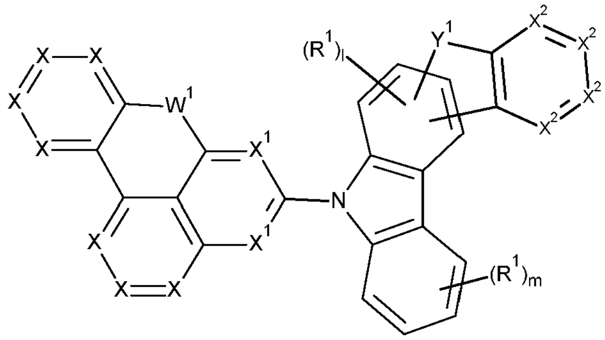

Formel (VIb)

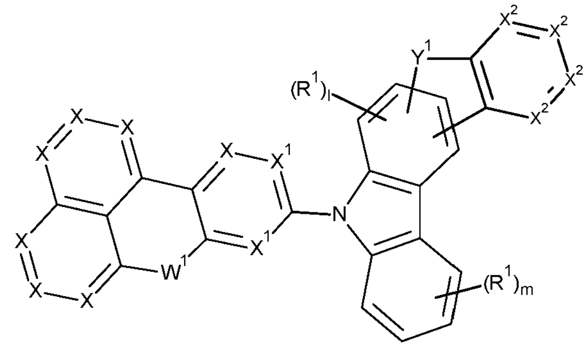

Formel (VIc)

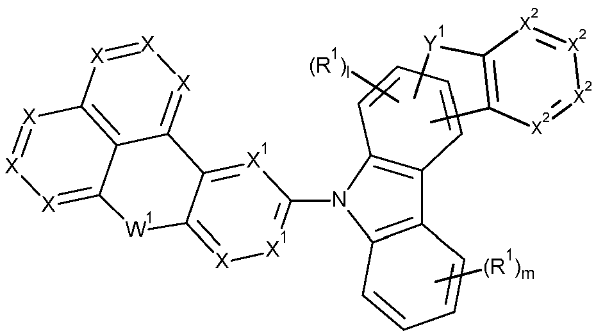

Formel (VId)

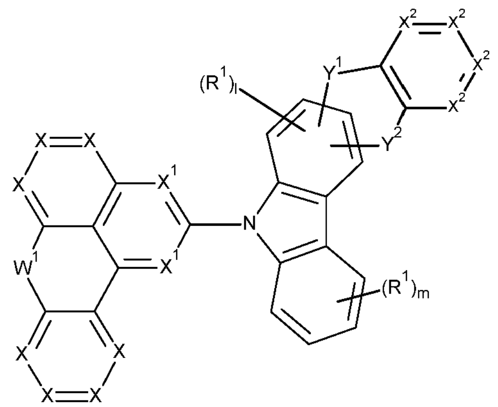

Formel (IXa)

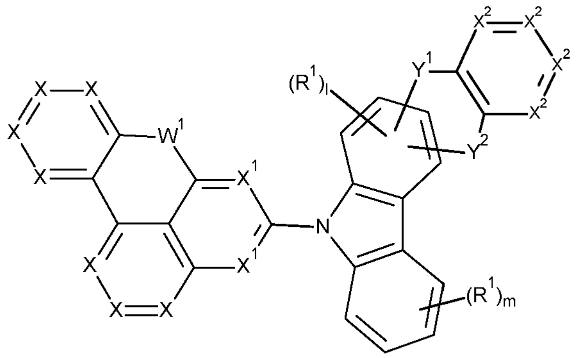

Formel (IXb)

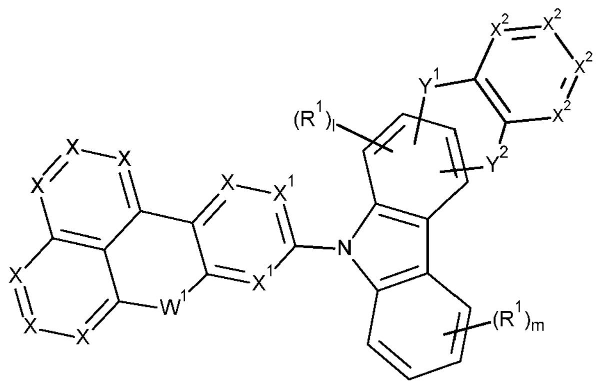

Formel (IXc)

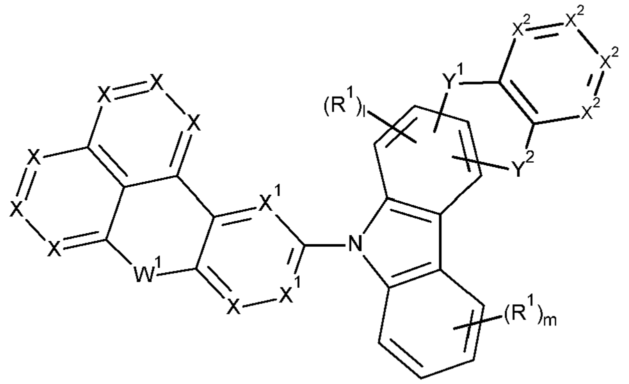

Formel (IXd)

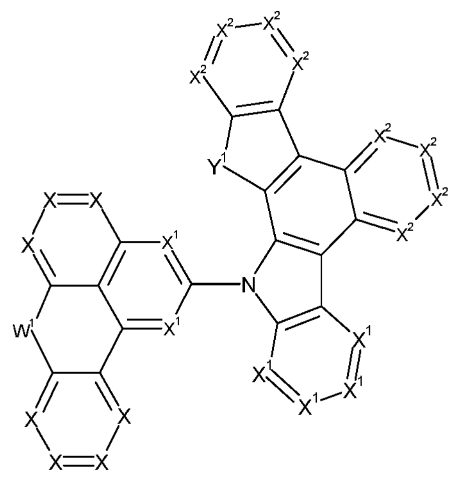

Formel (XIIa)

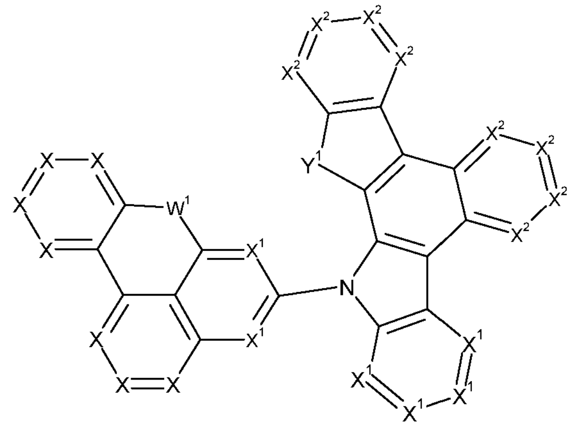

Formel (XIIb)

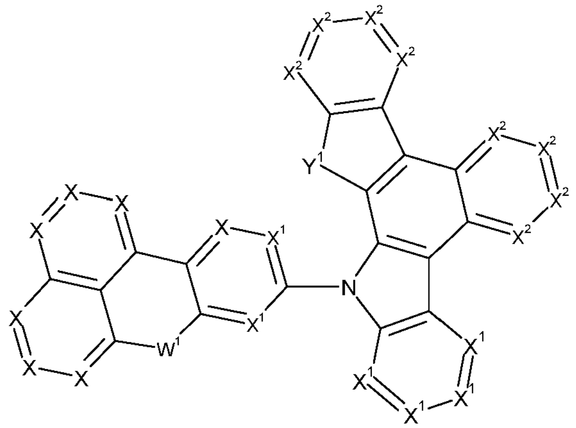

Formel (XIIc)

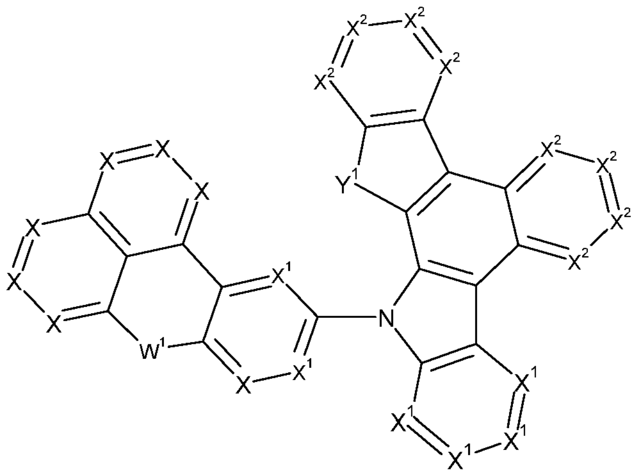

Formel (XIId)

wobei die verwendeten Symbole $W^1$, $R^1$, X, m und $X^1$ die in Anspruch 1 genannte Bedeutung aufweisen, die Symbole $Y^1$, $Y^2$ und $X^2$ die in Anspruch 3 genannte Bedeutung aufweisen, und I für 0, 1 oder 2 steht.

**5.** Verbindung gemäß Anspruch 4, einer Formel (VIIa), (VIIb), (VIIc), (VIId), (Xa), (Xb), (Xc), (Xd), (XIIia), (XIIIb), (XIIIc) oder (XIIId),

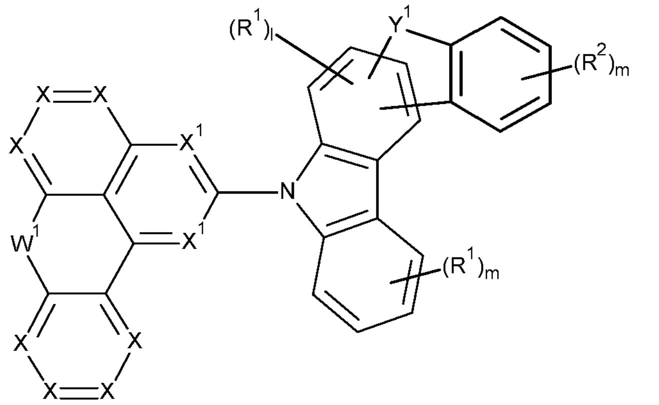

Formel (VIIa)

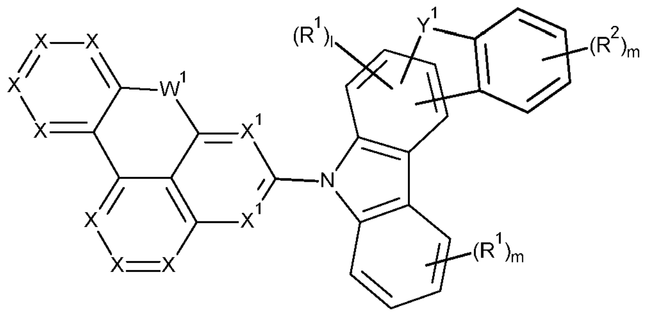

Formel (VIIb)

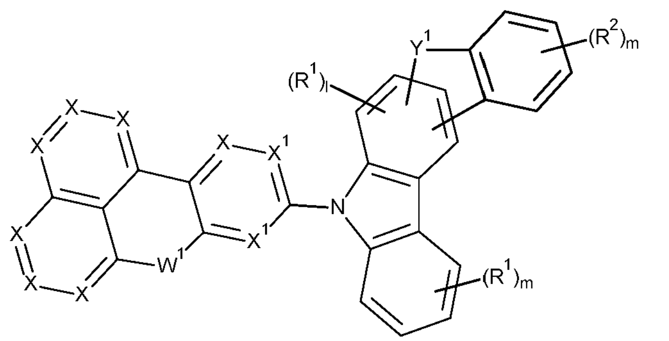

Formel (VIIc)

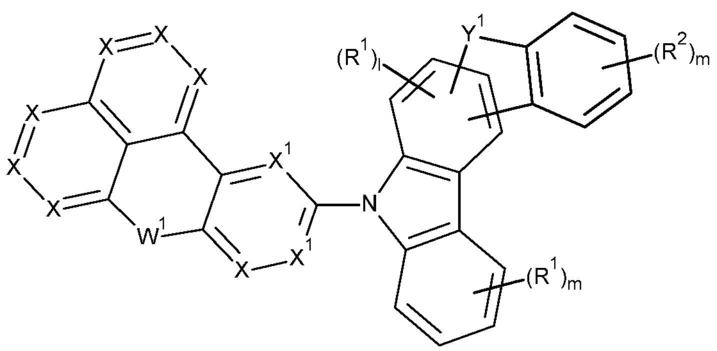

Formel (VIId)

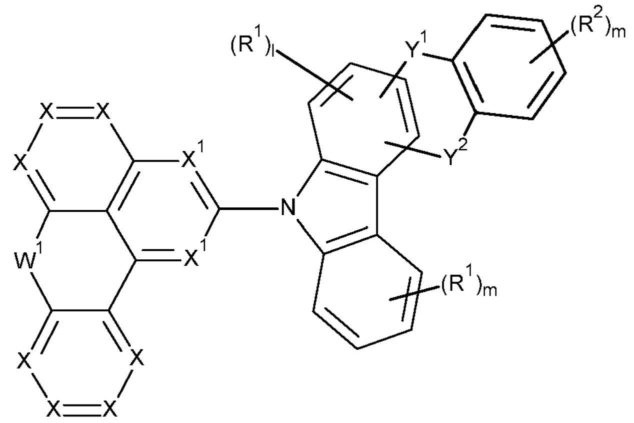

Formel (Xa)

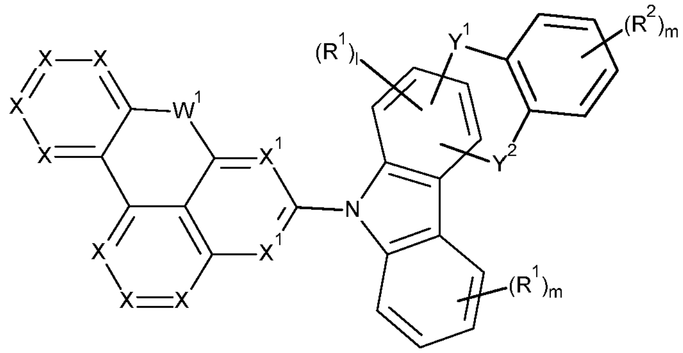

Formel (Xb)

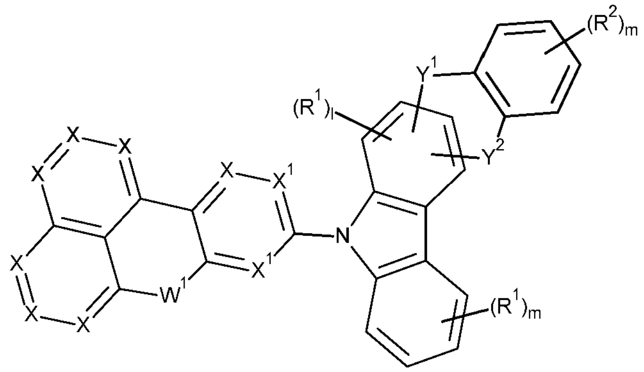

Formel (Xc)

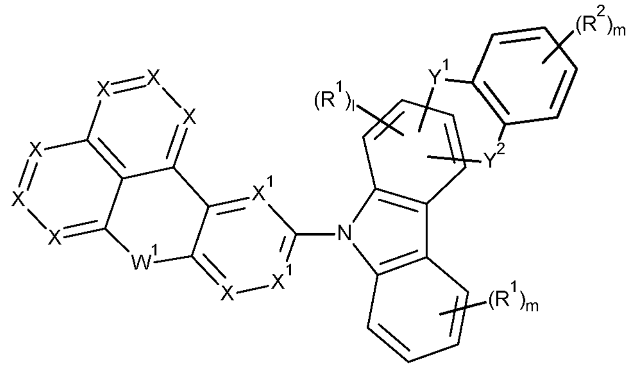

Formel (Xd)

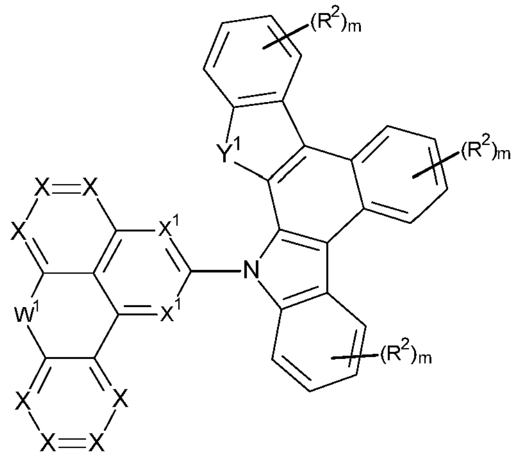

Formel (XIIIa)

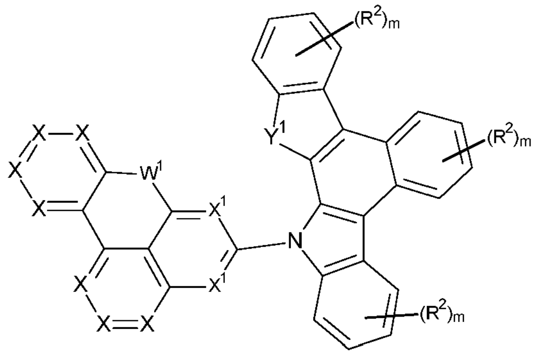

Formel (XIIIb)

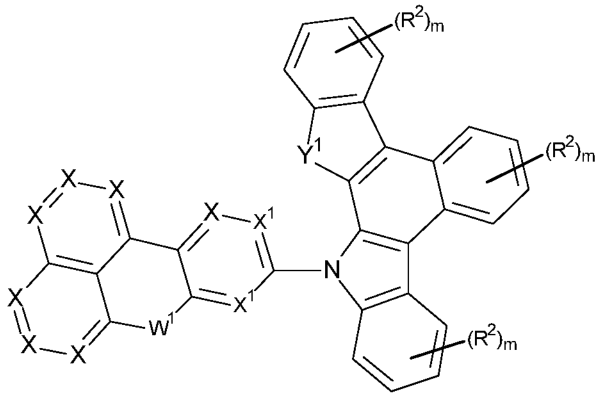

Formel (XIIIc)

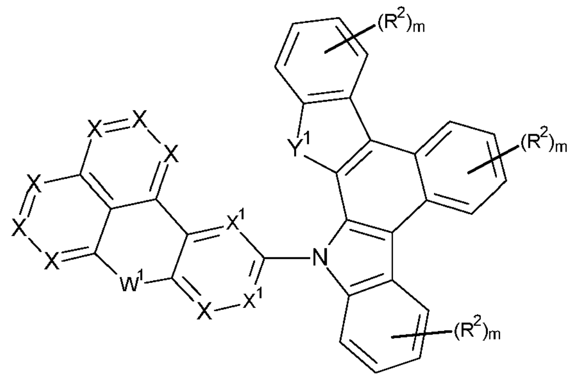

Formel (XIIId)

wobei die verwendeten Symbole $W^1$, $R^1$, $R^2$, X, m und $X^1$ die in Anspruch 1 genannte Bedeutung aufweisen, die Symbole $Y^1$ und $Y^2$ die in Anspruch 3 genannte Bedeutung aufweisen, und I für 0, 1 oder 2 steht.

**6.** Verbindung gemäß mindestens einem der Ansprüche 1 bis 5, **dadurch gekennzeichnet, dass** nicht mehr als vier, vorzugsweise nicht mehr als zwei Gruppen X für N stehen, wobei vorzugsweise höchstens 4, besonders bevorzugt höchstens 3 und speziell bevorzugt höchstens 2 der Gruppen $CR^1$, für die X steht, ungleich der Gruppe CH ist.

**7.** Oligomer, Polymer oder Dendrimer enthaltend eine oder mehrere Verbindungen nach einem der Ansprüche 1 bis 6, wobei statt eines Wasserstoffatoms oder eines Substituenten ein oder mehrere Bindungen der Verbindungen zum Polymer, Oligomer oder Dendrimer vorhanden sind.

**8.** Zusammensetzung enthaltend wenigstens eine Verbindung nach einem oder mehreren der Ansprüche 1 bis 6 oder ein Oligomer, Polymer oder Dendrimer nach Anspruch 7 und wenigstens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

**9.** Formulierung, enthaltend mindestens eine Verbindung nach einem oder mehreren der Ansprüche 1 bis 6 oder ein Oligomer, Polymer oder Dendrimer nach Anspruch 7 oder eine Zusammensetzung nach Anspruch 8 und mindestens ein Lösemittel.

**10.** Verwendung einer Verbindung nach einem oder mehreren der Ansprüche 1 bis 6, eines Oligomers, Polymers oder Dendrimers nach Anspruch 7 oder einer Zusammensetzung nach Anspruch 8 in einer elektronischen Vorrichtung, insbesondere als Hostmaterial oder Elektronentransportmaterial.

**11.** Verfahren zur Herstellung einer Verbindung nach einem oder mehreren der Ansprüche 1 bis 6 oder eines Oligomers, Polymers und/oder Dendrimers nach Anspruch 7, **dadurch gekennzeichnet, dass** in einer Kupplungsreaktion eine

Verbindung, umfassend mindestens eine stickstoffhaltige heterocyclische Gruppe, mit einer Verbindung, umfassend mindestens eine Carbazol-Gruppe, verbunden wird.

12. Elektronische Vorrichtung, enthaltend mindestens eine Verbindung nach einem oder mehreren der Ansprüche 1 bis 6, ein Oligomer, Polymer oder Dendrimer nach Anspruch 7 oder eine Zusammensetzung gemäß Anspruch 8, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

1. Compound of a formula (IIIa), (IIIb), (IIIc) or (IIId):

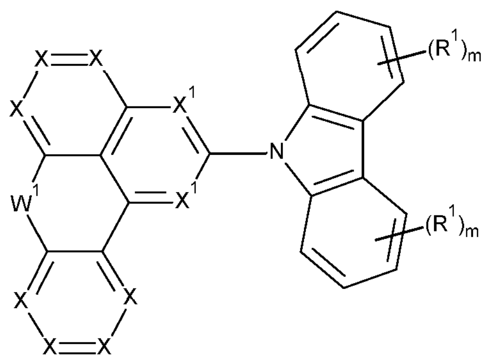

formula (IIIa)

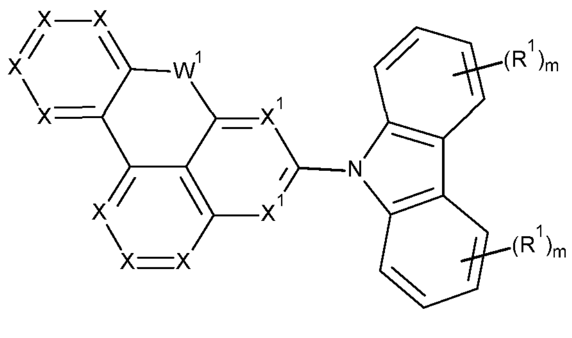

formula (IIIb)

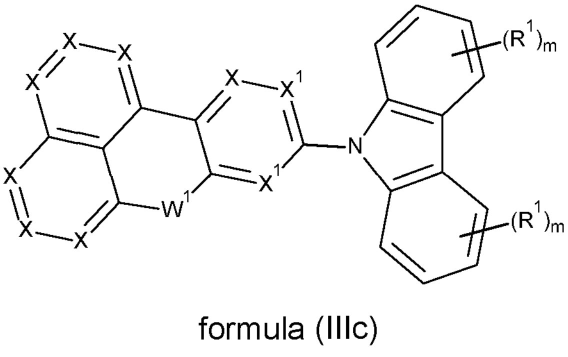

formula (IIIc)

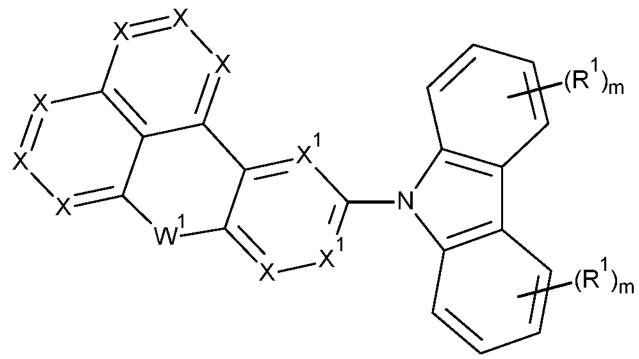

formula (IIId)

where the following applies to the symbols used:

X is on each occurrence, identically or differently, N or $CR^1$;
$X^1$ is N or $CR^1$, where precisely one group $X^1$ stands for N;
$W^1$ is O, S, SO, $SO_2$, $SiR^1_2$ or C=O;
$R^1$ is on each occurrence, identically or differently, H, D, F, Cl, Br, I, CN, $NO_2$, $N(Ar^1)_2$, $N(R^2)_2$, $OAr^1$, $OR^2$, $SAr^1$, $SR^2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, a straight-chain alkyl, alkoxy or thioalkoxy group having 1 to 40 C atoms or a branched or cyclic alkyl, alkoxy or thioalkoxy group having 3 to 40 C atoms or an alkenyl group having 2 to 40 C atoms, which may in each case be substituted by one or more radicals $R^2$ and where one or more non-adjacent $CH_2$ groups may be replaced by $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$, or a combination of these systems; two or more substituents $R^1$ may also form a mono- or polycyclic, aliphatic, heteroaliphatic, aromatic or heteroaromatic ring system with one another;
$Ar^1$ is on each occurrence, identically or differently, an aromatic or heteroaromatic ring system having 5 to 30 aromatic ring atoms, which may be substituted by one or more non-aromatic radicals $R^2$; two radicals $Ar^1$ that are bonded to the same Si atom, N atom, P atom or B atom may also be bridged to one another by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$;
$R^2$ is on each occurrence, identically or differently, H, D, F, Cl, Br, I, CN, $B(OR^3)_2$, $OR^3$, $SR^3$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $P(=O)(R^3)_2$, $OSO_2R^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$ and where one or more non-adjacent $CH_2$ groups may be replaced by $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more substituents $R^2$ may also form a mono- or polycyclic, aliphatic, heteroaliphatic, aromatic or heteroaromatic ring system with one another;
$R^3$ is selected on each occurrence, identically or differently, from the group consisting of H, D, F, CN, an aliphatic hydrocarbon radical having 1 to 20 C atoms or an aromatic or heteroaromatic ring system having 5 to 30 aromatic ring atoms, in which one or more H atoms may be replaced by D, F, Cl, Br, I or CN and which may be substituted by one or more alkyl groups, each having 1 to 4 carbon atoms; two or more substituents $R^3$ may form a mono- or polycyclic, aliphatic ring system with one another;
m is, identically or differently, 0, 1, 2, 3 or 4.

**2.** Compound according to Claim 1 of a formula (IVa), (IVb), (IVc) or (IVd),

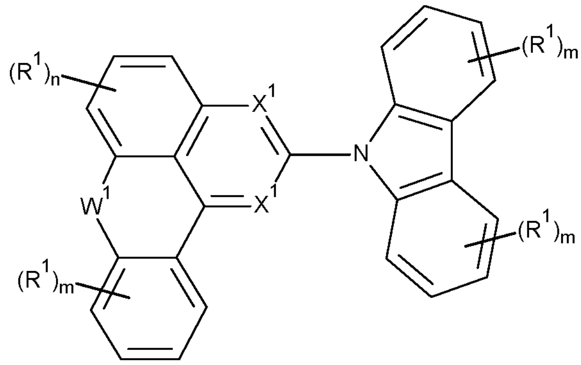

formula (IVa)

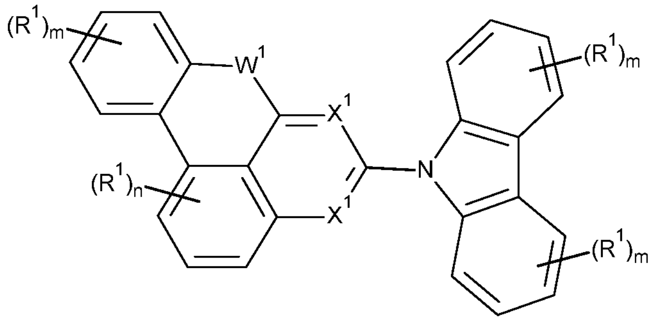

formula (IVb)

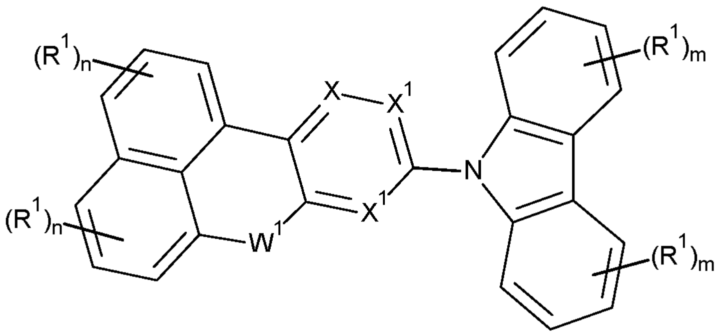

formula (IVc)

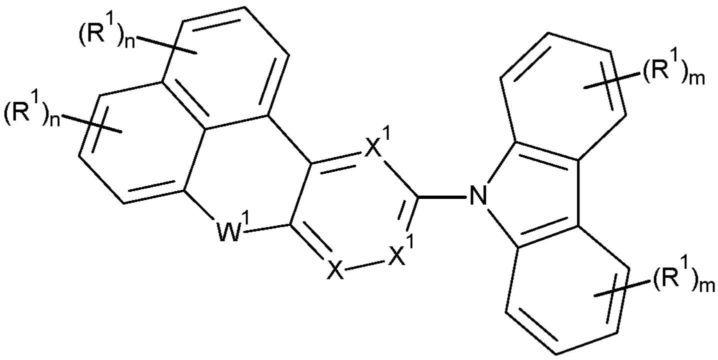

formula (IVd)

where the symbols $R^1$, $W^1$, m, X and $X^1$ have the meaning given in Claim 1, and n is, identically or differently, 0, 1, 2 or 3.

3. Compound according to Claim 1 or 2, **characterised in that** two adjacent radicals $R^1$ form a ring of the formula (DB-1), (DB-2) or (DB-3),

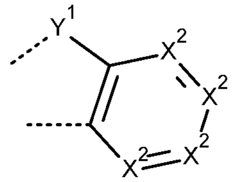

formula (DB-1)

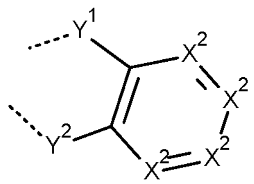

formula (DB-2)

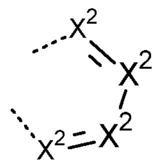

formula (DB-3)

where $X^2$ stands, identically or differently on each occurrence, for N or $CR^2$, $Y^1$ and $Y^2$ stand on each occurrence, independently, for O, S, $C(R^2)_2$ or $NR^2$ and the dashed lines represent the bond to the aryl or heteroaryl group, where $R^2$ has the meaning given in Claim 1.

4. Compound according to Claim 3, of a formula (VIa), (VIb), (VIc), (VId), (IXa), (IXb), (IXc), (IXd), (XIIa), (XIIb), (XIIc) or (XIId),

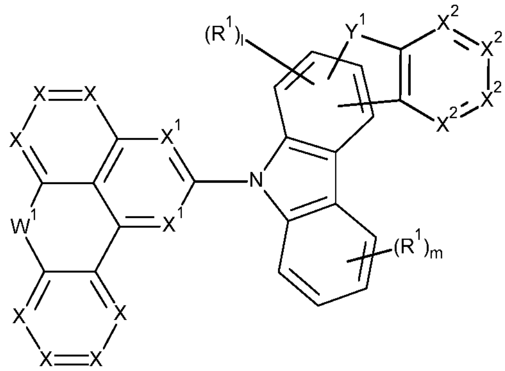

formula (VIa)

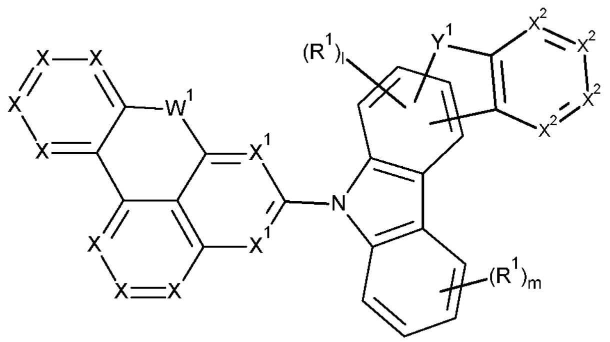

formula (VIb)

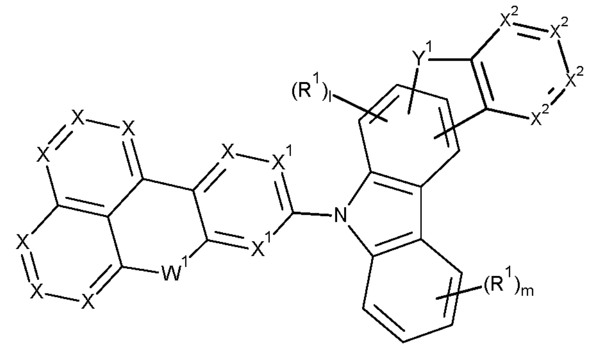

formula (VIc)

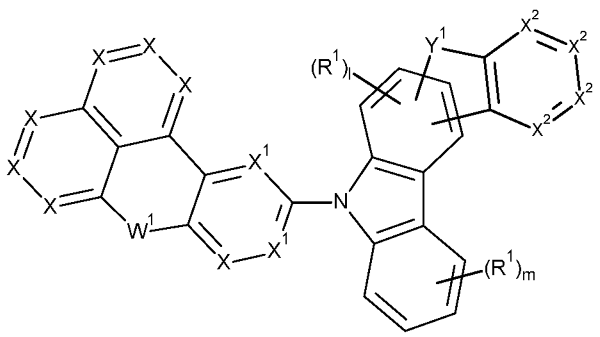

formula (VId)

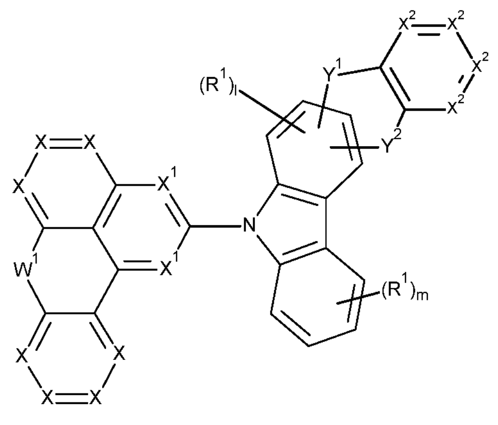

formula (IXa)

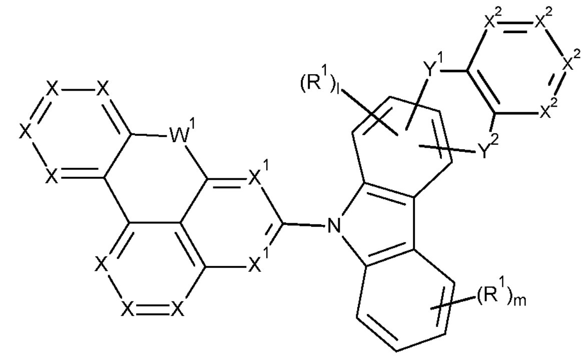

formula (IXb)

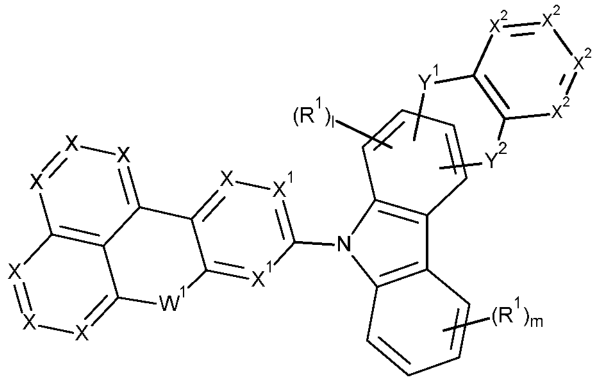

formula (IXc)

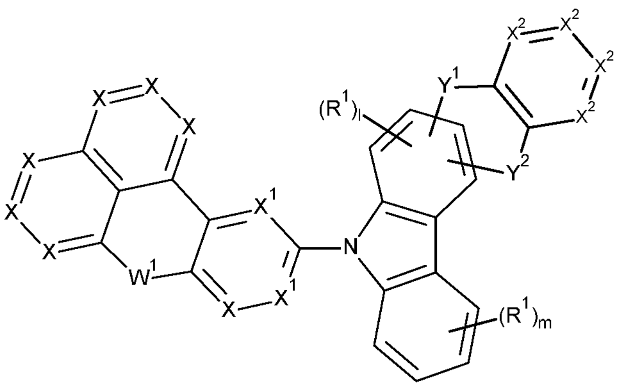

formula (IXd)

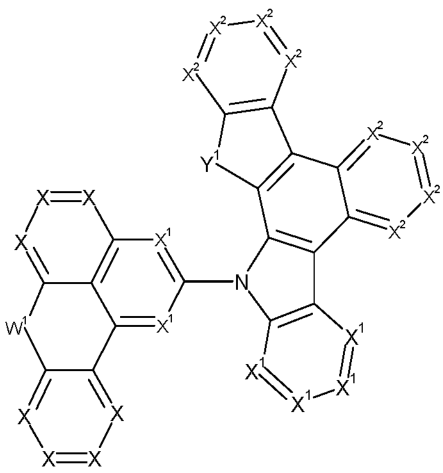

formula (XIIa)

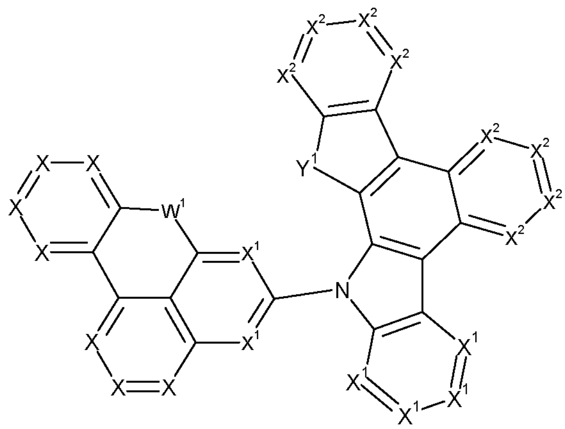

formula (XIIb)

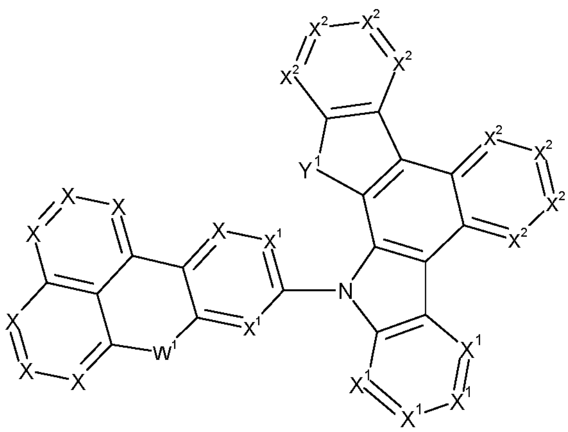

formula (XIIc)

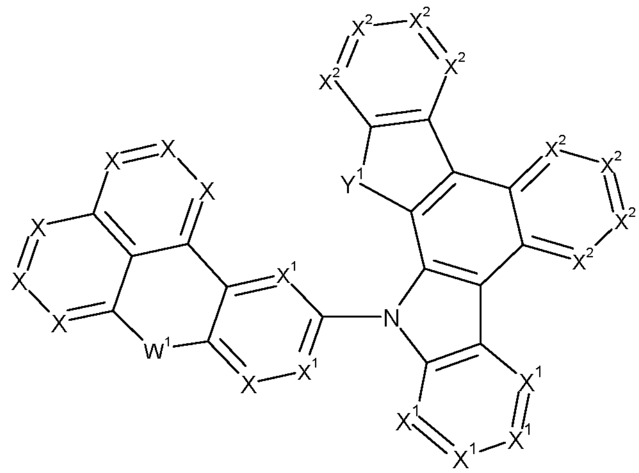

formula (XIId)

where the symbols $W^1$, $R^1$, X, m and $X^1$ used have the meaning given in Claim 1, the symbols $Y^1$, $Y^2$ and $X^2$ have the meaning given in Claim 3, and I stands for 0, 1 or 2.

**5.** Compound according to Claim 4, of a formula (VIIa), (VIIb), (Vile), (VIId), (Xa), (Xb), (Xc), (Xd), (XIIIa), (XIIIb), (XIIIc) or (XIIId),

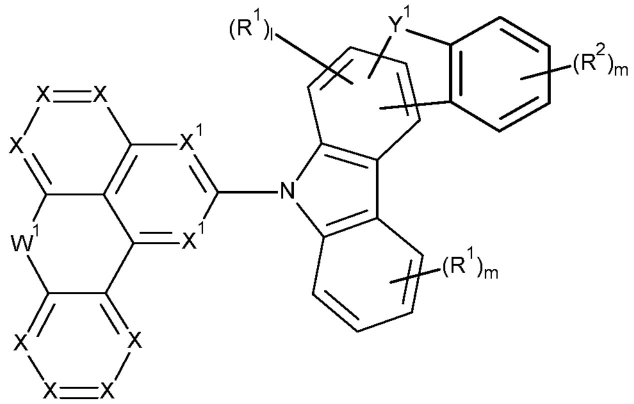

formula (VIIa)

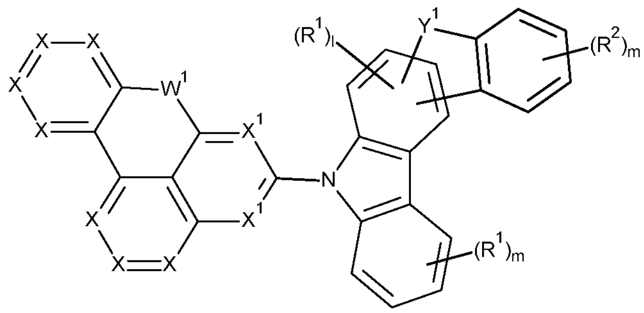

formula (VIIb)

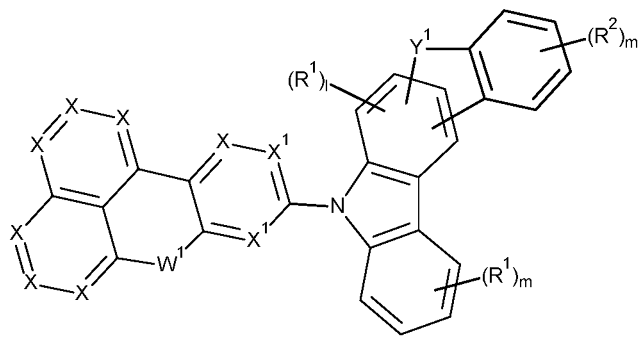

formula (VIIc)

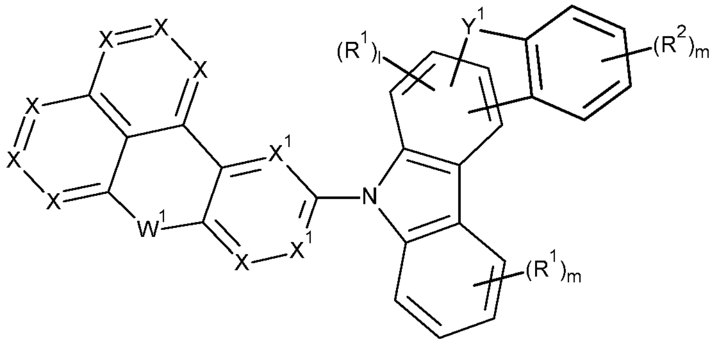

formula (VIId)

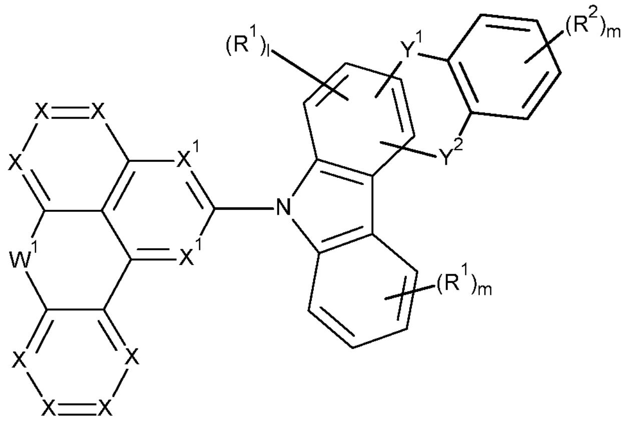

formula (Xa)

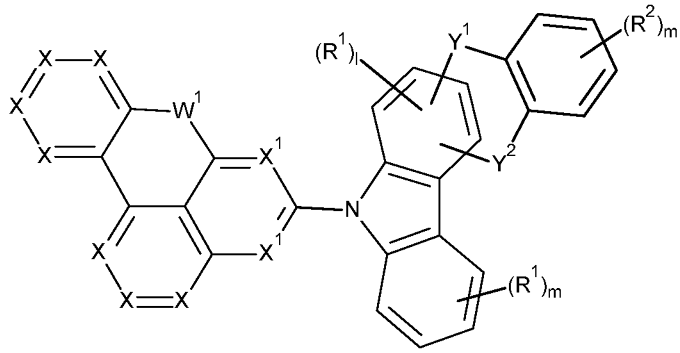

formula (Xb)

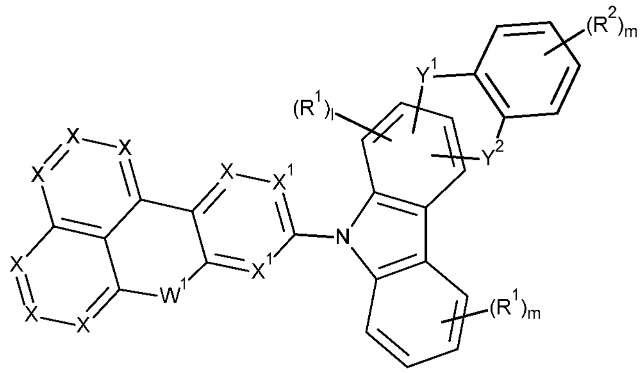

formula (Xc)

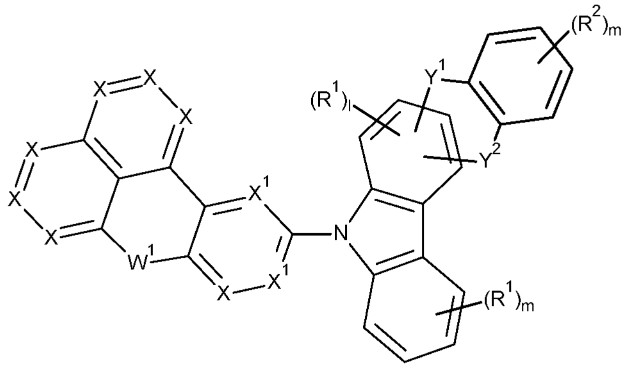

formula (Xd)

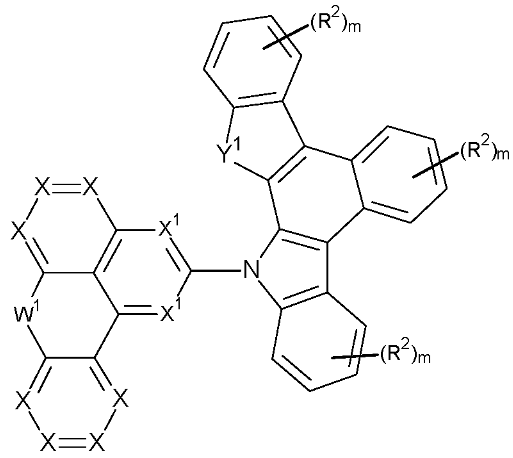

formula (XIIIa)

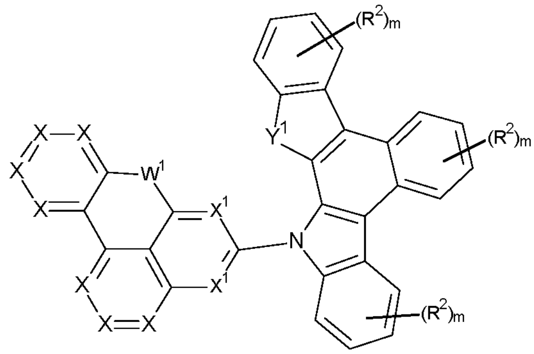

formula (XIIIb)

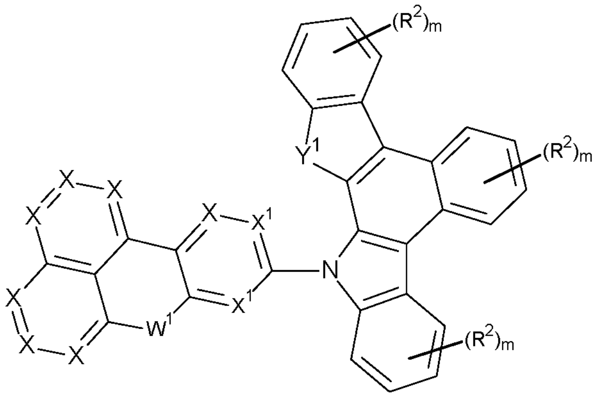

formula (XIIIc)

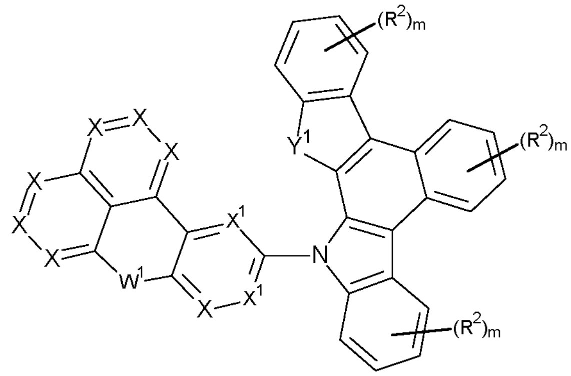

formula (XIIId)

where the symbols $W^1$, $R^1$, $R^2$, X, m and $X^1$ used have the meaning given in Claim 1, the symbols $Y^1$ and $Y^2$ have the meaning given in Claim 3, and I stands for 0, 1 or 2.

**6.** Compound according to at least one of Claims 1 to 5, **characterised in that** not more than four, preferably not more than two groups X stand for N, where preferably at most 4, particularly preferably at most 3 and especially preferably at most 2 of the groups $CR^1$ for which X stands are not equal to the group CH.

**7.** Oligomer, polymer or dendrimer containing one or more compounds according to one of Claims 1 to 6, where, instead of a hydrogen atom or a substituent, one or more bonds are present from the compounds to the polymer, oligomer or dendrimer.

**8.** Composition comprising at least one compound according to one or more of Claims 1 to 6 or an oligomer, polymer or dendrimer according to Claim 7 and at least one further compound selected from the group consisting of fluorescent emitters, phosphorescent emitters, emitters which exhibit TADF, host materials, electron-transport materials, electron-injection materials, hole-conductor materials, holeinjection materials, electron-blocking materials and hole-blocking materials.

**9.** Formulation comprising at least one compound according to one or more of Claims 1 to 6 or an oligomer, polymer or dendrimer according to Claim 7 or a composition according to Claim 8 and at least one solvent.

**10.** Use of a compound according to one or more of Claims 1 to 6, an oligomer, polymer or dendrimer according to Claim 7 or a composition according to Claim 8 in an electronic device, in particular as host material or electron-transport material.

**11.** Process for the preparation of a compound according to one or more of Claims 1 to 6 or an oligomer, polymer and/or dendrimer according to Claim 7, **characterised in that** a compound containing at least one nitrogen-containing heterocyclic group is connected to a compound containing at least one carbazole group in a coupling reaction.

**12.** Electronic device containing at least one compound according to one or more of Claims 1 to 6, an oligomer, polymer or dendrimer according to Claim 7 or a composition according to Claim 8, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

**Revendications**

**1.** Composé de formule (IIIa), (IIIb), (IIIc) ou (IIId):

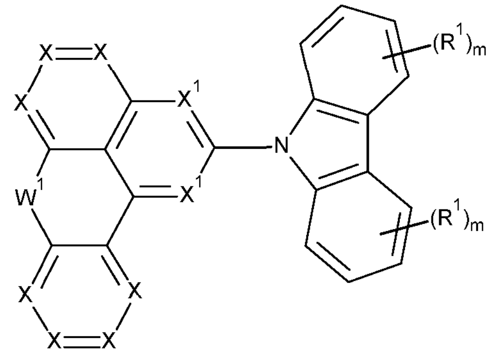

formule (IIIa)

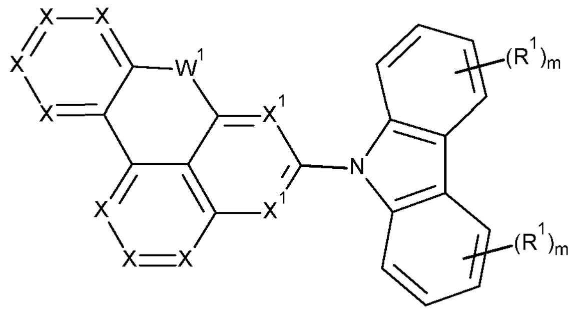

formule (IIIb)

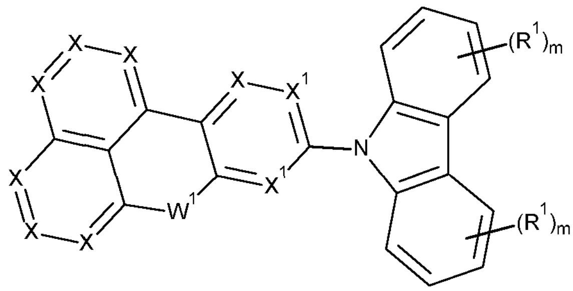

formule (IIIc)

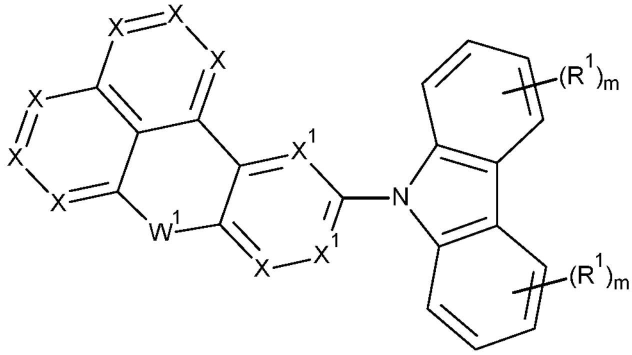

formule (IIId)

dans laquelle ce qui suit s'applique aux symboles utilisés :

X est à chaque occurrence, de manière identique ou différente, N ou $CR^1$ ;
$X^1$ est N ou $CR^1$, où précisément un groupement $X^1$ représente N ;
$W^1$ est O, S, SO, $SO_2$, $SiR^1_2$ ou C=O ;
$R^1$ est à chaque occurrence, de manière identique ou différente, H, D, F, Cl, Br, I, CN, $NO_2$, $N(Ar^1)_2$, $N(R^2)_2$, $OAr^1$, $OR^2$, $SAr^1$, $SR^2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C ou un groupement alcényle ayant de 2 à 40 atomes de C, pouvant dans chaque cas être substitué par un ou plusieurs radicaux $R^2$ et où un ou plusieurs groupements $CH_2$ non adjacents peuvent être remplacés par $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^2$, ou un groupement aryloxy ou hétéroaryloxy ayant de 5 à 40 atomes de cycle aromatique, pouvant être substitué par un ou plusieurs radicaux $R^2$, ou un groupement aralkyle ou hétéroaralkyle ayant de 5 à 40 atomes de cycle aromatique, pouvant être substitué par un ou plusieurs radicaux $R^2$, ou une combinaison de ces systèmes ; deux, ou plus, substituants $R^1$ peuvent également former un noyau mono- ou polycyclique, aliphatique, hétéroaliphatique, aromatique ou hétéroaromatique les uns avec les autres ;
$Ar^1$ est à chaque occurrence, de manière identique ou différente, un noyau aromatique ou hétéroaromatique ayant de 5 à 30 atomes de cycle aromatique, pouvant être substitué par un ou plusieurs radicaux non aromatiques $R^2$ ; deux radicaux $Ar^1$ qui sont liés au même atome de Si, atome de N, atome de P ou atome de B peuvent également être pontés l'un avec l'autre par une liaison simple ou un pont choisi parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;
$R^2$ est à chaque occurrence, de manière identique ou différente, H, D, F, Cl, Br, I, CN, $B(OR^3)_2$, $OR^3$, $SR^3$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $P(=O)(R^3)_2$, $OSO_2R^3$, $S(=O)R^3$, $S(=O)_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, pouvant dans chaque cas être substitué par un ou plusieurs radicaux $R^3$ et où un ou plusieurs groupements $CH_2$ non adjacents peuvent être remplacés par $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^3$, ou un groupement aryloxy ou hétéroaryloxy ayant de 5 à 40 atomes de cycle aromatique, pouvant être substitué par un ou plusieurs radicaux $R^3$, ou une combinaison de ces systèmes ; deux, ou plus, substituants $R^2$ peuvent également former un noyau mono- ou polycyclique, aliphatique, hétéroaliphatique, aromatique ou hétéroaromatique les uns avec les autres ;
$R^3$ est choisi à chaque occurrence, de manière identique ou différente, dans le groupe constitué par H, D, F, CN, un radical hydrocarboné aliphatique ayant de 1 à 20 atomes de C ou un noyau aromatique ou hétéroaromatique ayant de 5 à 30 atomes de cycle aromatique, où un ou plusieurs atomes de H peuvent être remplacés par D, F, Cl, Br, I ou CN et pouvant être substitué par un ou plusieurs groupements alkyle, chacun ayant de 1 à 4 atomes de carbone ; deux, ou plus, substituants $R^3$ peuvent former un noyau mono- ou polycyclique, aliphatique, les uns avec les autres ;
m vaut de manière identique ou différente, 0, 1, 2, 3 ou 4.

**2.** Composé selon la revendication 1, de formule (IVa), (IVb), (IVc) ou (IVd),

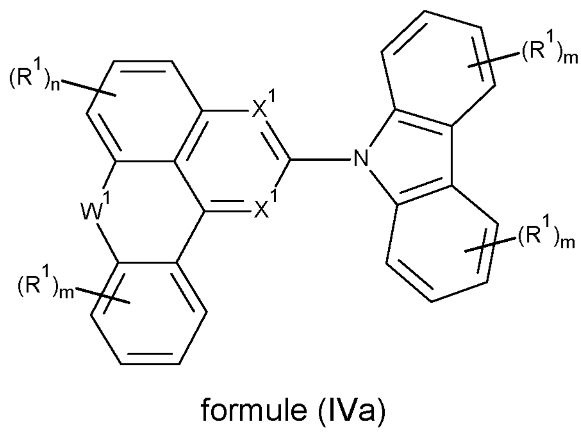

formule (IVa)

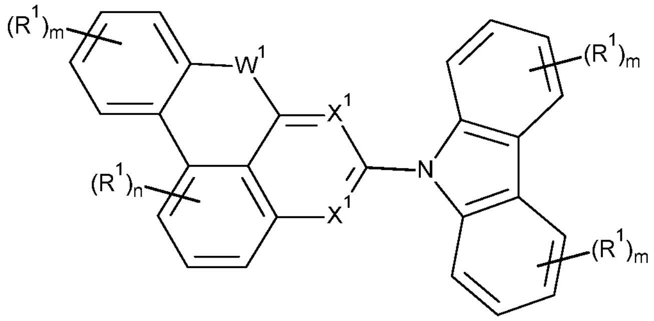

formule (IVb)

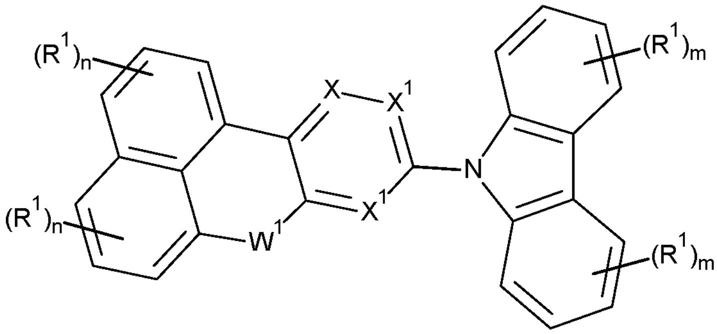

formule (IVc)

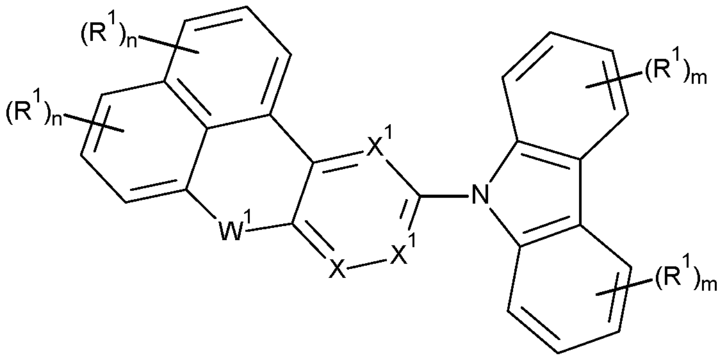

formule (IVd)

dans laquelle les symboles $R^1$, $W^1$, m, X et $X^1$ revêtent la signification donnée selon la revendication 1, et n vaut, de manière identique ou différente, 0, 1, 2 ou 3.

**3.** Composé selon la revendication 1 ou 2, **caractérisé en ce que** deux radicaux $R^1$ adjacents forment un cycle de formule (DB-1), (DB-2) ou (DB-3),

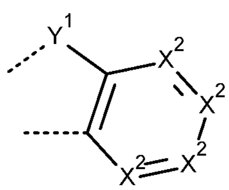

formule (DB-1)

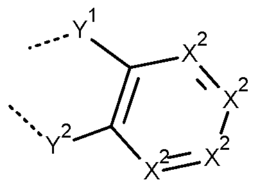

formule (DB-2)

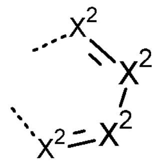

formule (DB-3)

dans laquelle $X^2$ représente, de manière identique ou différente à chaque occurrence, N ou $CR^2$, $Y^1$ et $Y^2$ représentent à chaque occurrence, indépendamment, O, S, $C(R^2)_2$ ou $NR^2$ et les lignes en pointillés représentent la liaison au groupement aryle ou hétéroaryle, où $R^2$ revêt la signification donnée selon la revendication 1.

4. Composé selon la revendication 3, de formule (VIa), (VIb), (VIc), (VId), (IXa), (IXb), (IXc), (IXd), (XIIa), (XIIb), (XIIc) ou (XIId),

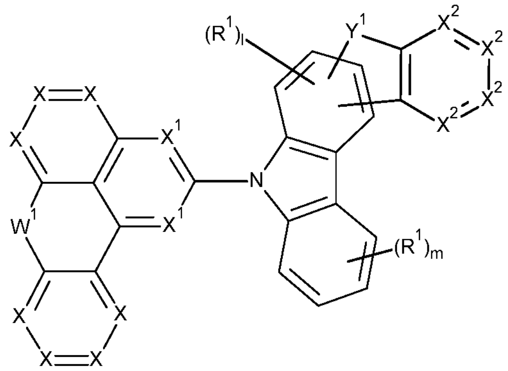

formule (VIa)

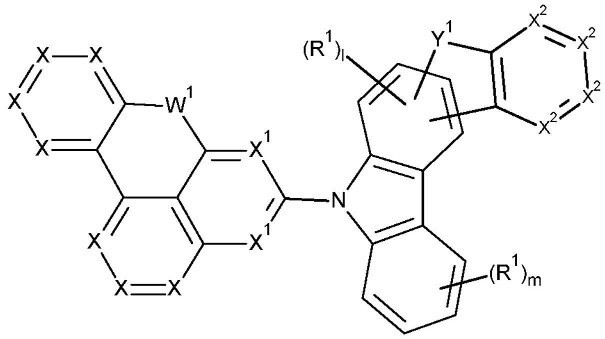

formule (VIb)

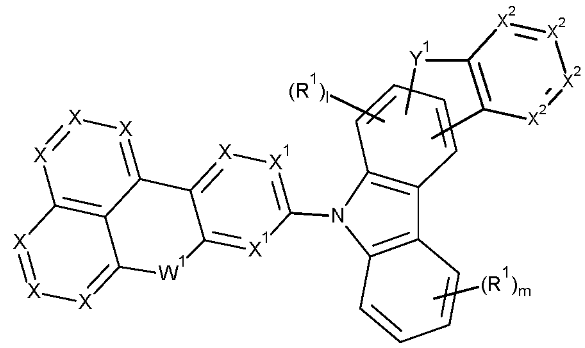

formule (VIc)

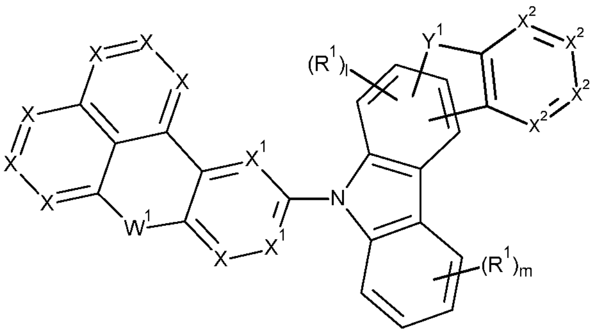

formule (VId)

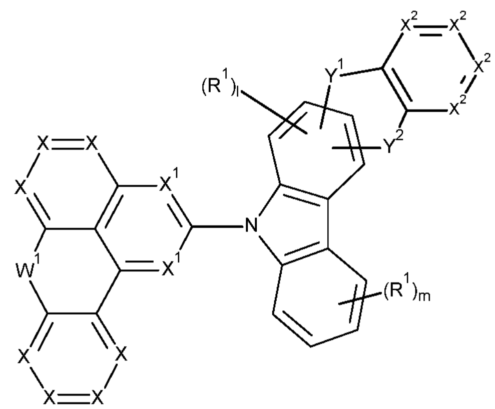

formule (IXa)

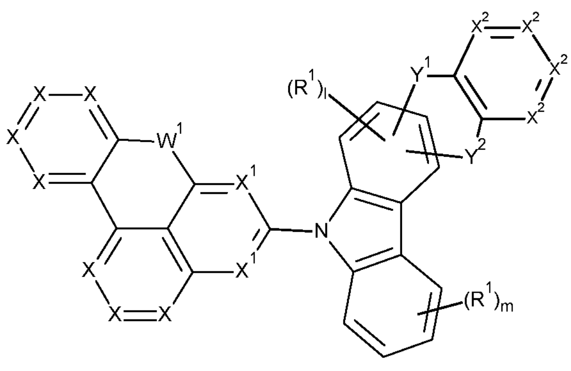

formule (IXb)

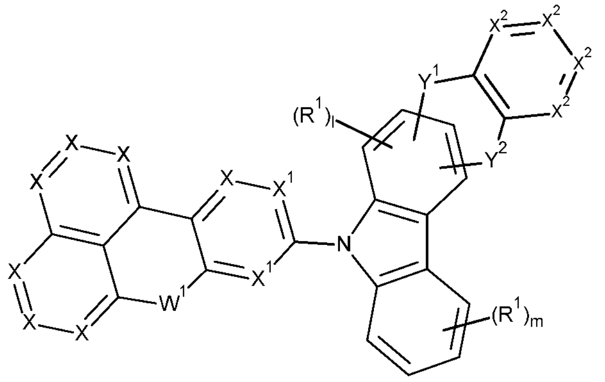

formule (IXc)

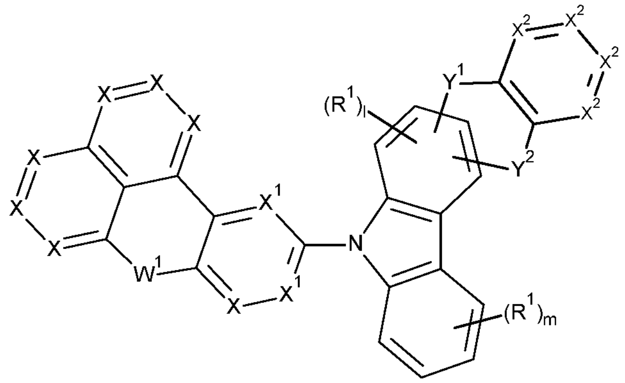

formule (IXd)

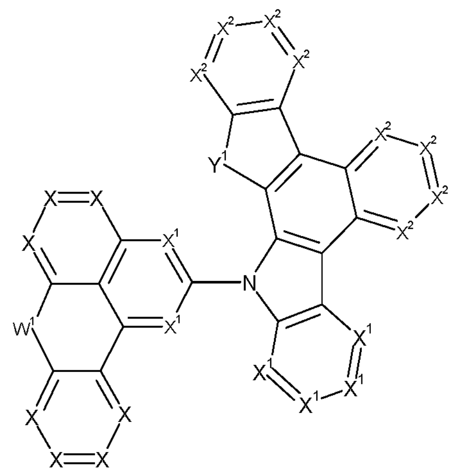

formule (XIIa)

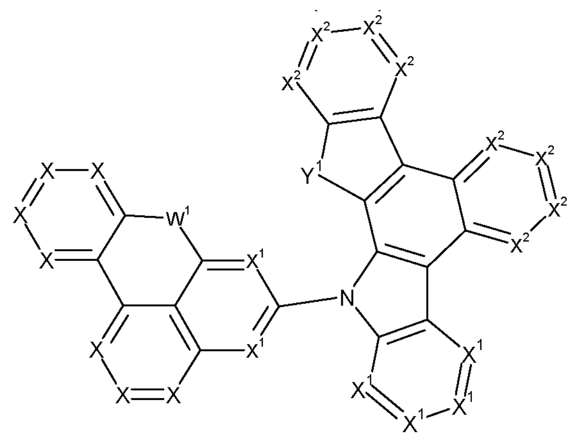

formule (XIIb)

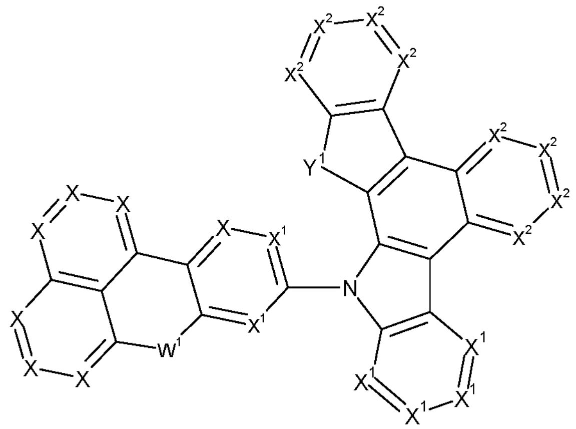

formule (XIIc)

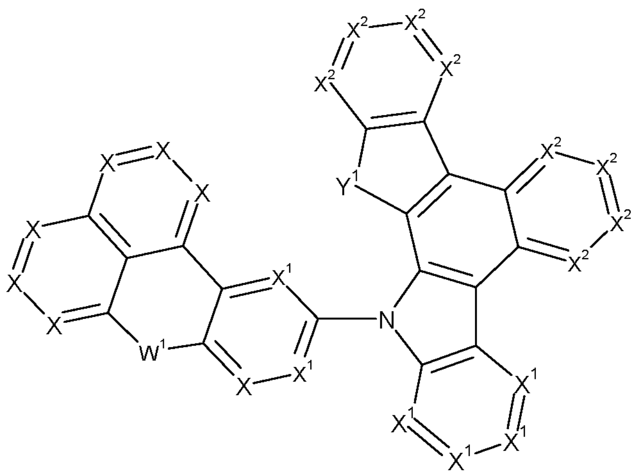

formule (XIId)

dans laquelle les symboles $W^1$, $R^1$, X, m et $X^1$ utilisés revêtent la signification donnée selon la revendication 1, les symboles $Y^1$, $Y^2$ et $X^2$ revêtent la signification donnée selon la revendication 3, et I représente 0, 1 ou 2.

**5.** Composé selon la revendication 4, de formule (VIIa), (VIIb), (Vile), (VIId), (Xa), (Xb), (Xc), (Xd), (XIIIa), (XIIIb), (XIIIc) ou (XIIId),

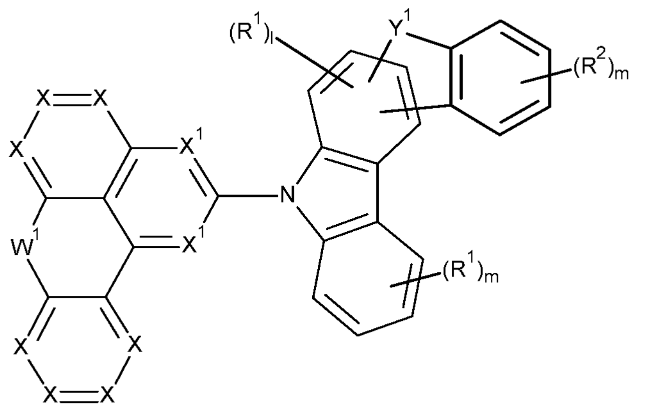

formule (VIIa)

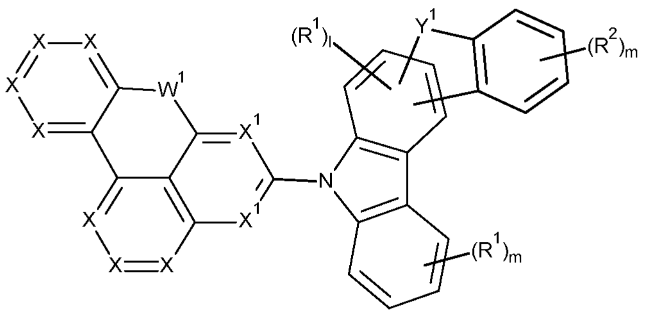

formule (VIIb)

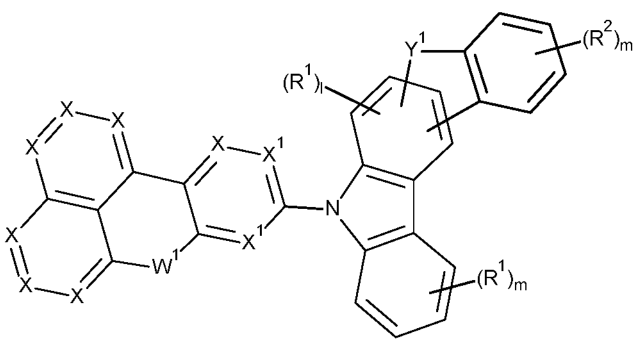

formule (VIIc)

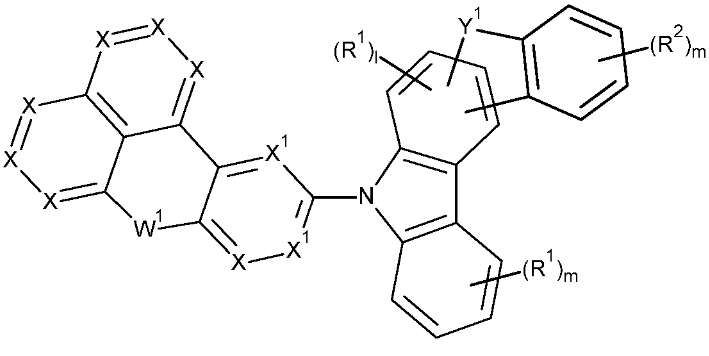

formule (VIId)

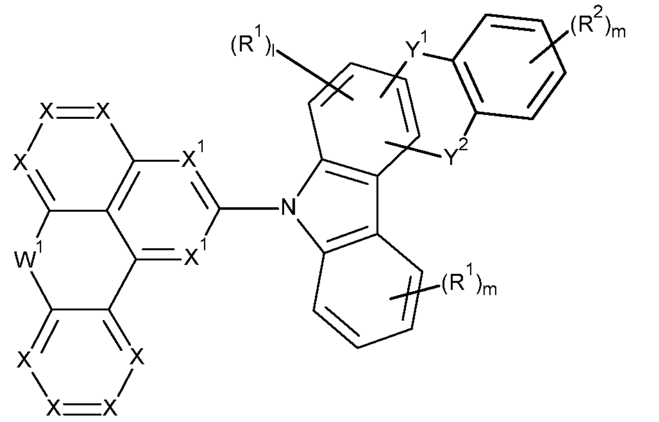

formule (Xa)

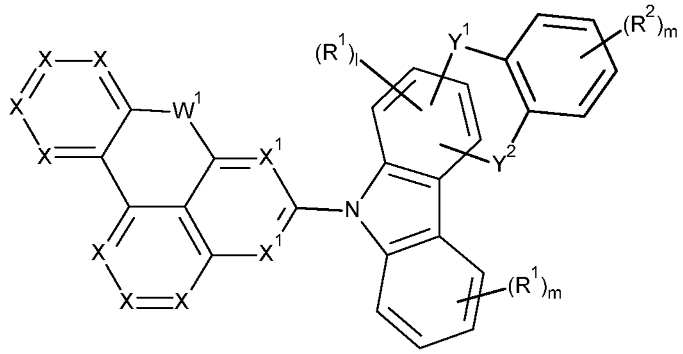

formule (Xb)

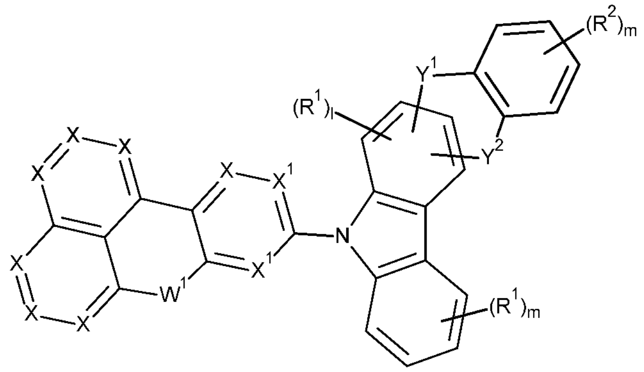

formule (Xc)

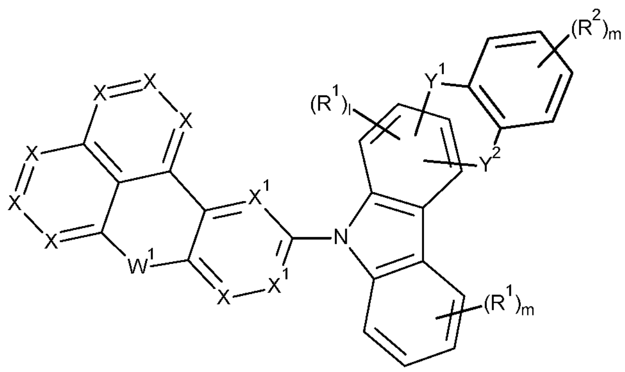

formule (Xd)

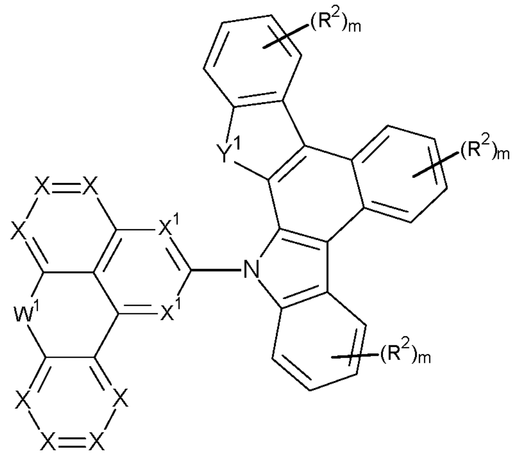

formule (XIIIa)

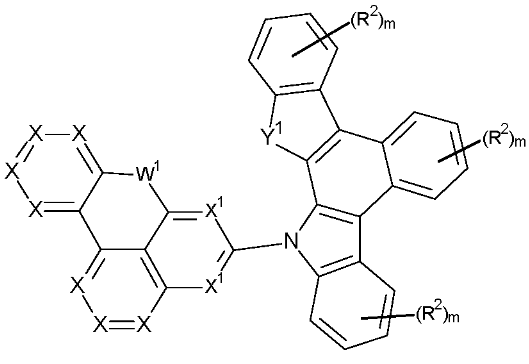

formule (XIIIb)

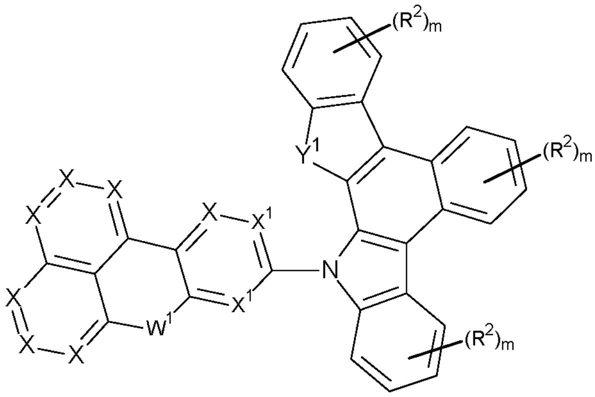

formule (XIIIc)

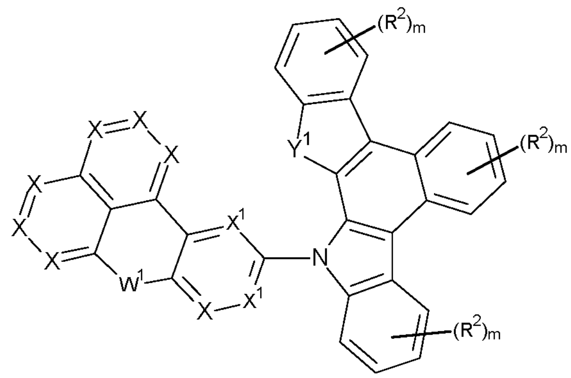

formule (XIIId)

dans laquelle les symboles $W^1$, $R^1$, $R^2$, X, m et $X^1$ utilisés revêtent la signification donnée selon la revendication 1, les symboles $Y^1$ et $Y^2$ revêtent la signification donnée selon la revendication 3, et I représente 0, 1 ou 2.

6. Composé selon au moins l'une parmi les revendications 1 à 5, **caractérisé en ce que** pas plus de quatre, préférablement pas plus de deux groupements X représentent N, où préférablement au plus 4, particulièrement préférablement au plus 3 et notamment préférablement au plus 2 parmi les groupements $CR^1$, que X représente, ne sont pas équivalents au groupement CH.

7. Oligomère, polymère ou dendrimère contenant un ou plusieurs composés selon l'une parmi les revendications 1 à 6, où, au lieu d'un atome d'hydrogène ou d'un substituant, une ou plusieurs liaisons sont présentes entre les composés et le polymère, l'oligomère ou le dendrimère.

8. Composition comprenant au moins un composé selon l'une ou plusieurs parmi les revendications 1 à 6 ou un oligomère, polymère ou dendrimère selon la revendication 7 et au moins un autre composé choisi dans le groupe constitué par les émetteurs fluorescents, les émetteurs phosphorescents, les émetteurs présentant une TADF, les matériaux hôtes, les matériaux de transport d'électrons, les matériaux d'injection d'électrons, les matériaux conducteurs de trous, les matériaux d'injection de trous, les matériaux de blocage d'électrons et les matériaux de blocage de trous.

9. Formulation comprenant au moins un composé selon l'une ou plusieurs parmi les revendications 1 à 6 ou un oligomère, polymère ou dendrimère selon la revendication 7 ou une composition selon la revendication 8 et au moins un solvant.

10. Utilisation d'un composé selon l'une ou plusieurs parmi les revendications 1 à 6, d'un oligomère, polymère ou dendrimère selon la revendication 7 ou d'une composition selon la revendication 8, dans un dispositif électronique, en particulier comme matériau hôte ou matériau de transport d'électrons.

**11.** Procédé de préparation d'un composé selon l'une ou plusieurs parmi les revendications 1 à 6 ou d'un oligomère, polymère ou dendrimère selon la revendication 7, **caractérisé en ce qu'**un composé contenant au moins un groupement hétérocyclique azoté est relié à un composé contenant au moins un groupement carbazole dans une réaction de couplage.

**12.** Dispositif électronique contenant au moins un composé selon l'une ou plusieurs parmi les revendications 1 à 6, un oligomère, polymère ou dendrimère selon la revendication 7 ou une composition selon la revendication 8, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2017109727 A
- WO 2016195441 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2016124304 A
- WO 2017032439 A
- EP 16179378
- EP 16186313
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- WO 2015192939 A
- WO 2015169412 A
- WO 2016015810 A
- WO 2016023608 A
- EP 16158460
- EP 16159829
- WO 2010108579 A
- WO 2005053051 A
- WO 2009030981 A